# FASCICULE DE BREVET EUROPEEN

(11) **EP 2 875 026 B1**
(45) Date de publication et mention de la délivrance du brevet: **21.09.2016**
(21) Numéro de dépôt: 13744752.0
(22) Date de dépôt: 16.07.2013
(51) Int. Cl.: A61K 31/155, A61K 31/437, A61K 31/444, A61K 31/5377

(54) **DERIVE D'IMIDAZOPYRIDINE UTILES DANS LE TRAITEMENT DU DIABETE**
IMIDAZOPYRIDINDERIVATE ZUR BEHANDLUNG VON DIABETES
IMIDAZOPYRIDINE DERIVATIVE USED IN THE TREATMENT OF DIABETES

(30) Priorité: 20.07.2012 FR 1257082
(43) Date de publication de la demande: 27.05.2015
(73) Titulaire: Metabrain Research, 91380 Chilly Mazarin (FR)
(72) Inventeur: LEPIFRE, Franck, F., F-91400 Saclay (FR); LENA, Gersande, R., F-69840 Julienas (FR); AUTIER, Valérie, F-91190 Gif Sur Yvette (FR); KERGOAT, Micheline, R., F-91440 Bures Sur Yvette (FR); CHARON, Christine, G., F-91940 Gometz Le Chatel (FR); RAYNAL, Sophie, N., F-75019 Paris (FR); AUDET, Annick,M., F-91630 Leudeville (FR)
(74) Mandataire: Mendelsohn, Isabelle M. N.
(86) Numéro de dépôt international: PCT/FR2013/051703
(87) Numéro de publication internationale: WO 2014/013182

(56) Documents cités:
- EP-A1- 2 072 516
- EP-A1- 2 168 965

## Description

La présente invention concerne des dérivés d'imidazopyridine utiles dans le traitement de pathologies associées au syndrome métabolique, en particulier le traitement ou la prévention du diabète.

Le diabète sucré représente un groupe très hétérogène de maladies ayant toutes en commun un certain nombre de caractéristiques : une hyperglycémie, des anomalies fonctionnelles et quantitatives des cellules bêta pancréatiques, une résistance tissulaire à l'insuline et un risque accru de développer à long terme des complications, en particulier cardiovasculaires.

Le diabète de type 2 est devenu un problème majeur de santé publique. Sa prévalence est en forte augmentation dans les pays les plus industrialisés mais encore plus dans les pays en pleine expansion économique. On peut parler aujourd'hui d'épidémie pour cette maladie qui entraîne d'importantes complications pouvant devenir très invalidantes ou même létales du fait entre autres d'insuffisance rénale, d'infarctus du myocarde, ou d'accidents vasculaires cérébraux.

Quelques chiffres sur le diabète (données OMS) :
- Plus de 220 millions de personnes sont diabétiques dans le monde.
- Le diabète multiplie par 3 les risques d'accident vasculaire
- Le diabète est la première cause de cécité et d'insuffisance rénale dans le monde occidental.
- Selon les estimations, le diabète a tué 1,1 million de personnes en 2005.
- Selon les projections de l'OMS, le nombre de décès par diabète va doubler entre 2005 et 2030.

En France, les soins et traitements des diabétiques pèsent très lourds sur le budget de l'Assurance Maladie. Compte-tenu des chiffres alarmants du nombre de patients diabétiques dans le monde d'ici 2030, de nombreuses compagnies pharmaceutiques et biotechnologiques investissent de façon intense en R&D dans le domaine du métabolisme et plus particulièrement dans celui du diabète de type 2 afin de mettre sur le marché de nouvelles alternatives médicamenteuses.

A l'heure actuelle, aucun traitement du diabète de type 2 n'est capable de rétablir un équilibre glycémique normal sur les 24 heures et n'est dénué d'effets secondaires. Aucun ne prend en compte la pathologie complète de la maladie et ne vise qu'à corriger l'un ou l'autre défaut. Les antidiabétiques les plus récemment mis sur le marché n'ont pas montré d'amélioration du contrôle glycémique supérieure à celle observée avec les traitements préexistants et ont entraîné des effets secondaires indésirables, ce qui laisse la place pour de nouveaux traitements potentiels.

Il existe donc un besoin de nouvelles molécules utiles dans le traitement ou la prévention du diabète, de ses complications et/ou des pathologies associées, avantageusement du diabète de type 2.

Les inventeurs ont découvert de façon surprenante que certains dérivés d'imidazopyridine présentaient une activité inhibitrice de la production hépatique de glucose et activatrice de la sécrétion d'insuline en réponse au glucose et en particulier pouvaient être utilisés comme produits à usage pharmaceutique chez les patients qui en ont besoin, notamment pour la prévention et/ou le traitement du diabète et de ses complications et/ou des pathologies associées (obésité, hypertension, etc...), avantageusement le diabète de type 2.

EP 2168965 décrit les dérivés d' imidazolopyridine, pour le traitement des maladies liées au récepteur mélanocortine-4.

### Description détaillée de l'invention

La présente invention concerne donc des dérivés d'imidazopyridine de formule générale I suivante : 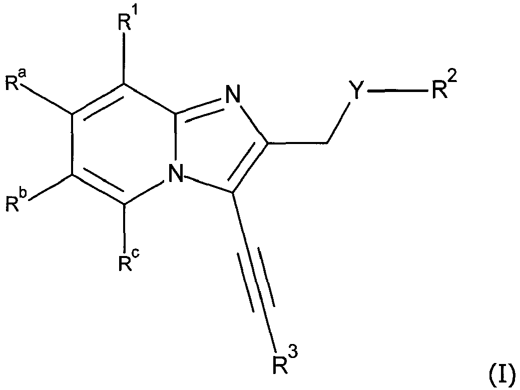 dans laquelle :
**Y** représente un atome d'oxygène ou de soufre, le groupe SO, SO₂ ou-NR¹⁹, dans lequel **R¹⁹** représente un atome d'hydrogène ou un groupe alkyle en C₁-C₆, avantageusement méthyle (Me); avantageusement **Y** représente un atome d'oxygène, de soufre ou un groupe -NR¹⁹, en particulier un atome d'oxygène ou un groupe -NR¹⁹.
**R¹ R^{a}, R^{b}** et **R^{C}** représentent indépendamment l'un de l'autre un atome d'hydrogène ; un atome d'halogène, avantageusement Cl ; un groupe alkyle en C₁-C₆, avantageusement méthyle, éventuellement substitué par un groupe -OH ; un groupe -OH ; un groupe -O(alkyle en C₁-C₆) tel que - OMe, un groupe -O(alkyle en C₁-C₆)O(alkyle en C₁-C₆) ; un groupe -CN ; un groupe -NR⁴R⁵ dans lequel **R⁴** et **R⁵** représentent indépendamment l'un de l'autre un atome d'hydrogène ou un groupe alkyle en C₁-C₆ ; ou un groupe -(alkyle en C₁-C₆)NR⁶R⁷ dans lequel **R⁶** et **R⁷** représentent indépendamment l'un de l'autre un atome d'hydrogène ou un groupe alkyle en C₁-C₆ ;
**R²** représente
   - un atome d'hydrogène ;
   - un groupe alkyle en C₁-C₆ substitué par un groupe -OH, en particulier-(CH₂)₂OH ;
   - un groupe -(alkyle en C₁-C₆)COOR⁸, avantageusement -CH₂COOR⁸, dans lequel **R⁸** représente un atome d'hydrogène ou un groupe alkyle en C₁-C₆, en particulier méthyle (Me), éthyle (Et) ou isopropyle (iPr), le groupe alkyle pouvant être substitué par un groupe -NH₂ ou -OH (avantageusement, le groupe alkyle n'est pas substitué), des exemples de groupes -(alkyle en C₁-C₆)COOR⁸ sont -CH₂COOH, -CH₂COOMe,-CH₂COOEt, -CH₂CH(NH₂)COOEt et -CH₂COOiPr;
   - un groupe -(alkyle en C₁-C₆)CONHR⁹, avantageusement -CH₂CONHR⁹, dans lequel **R⁹** représente un groupe -OH ; un groupe alkyle en C₁-C₆, avantageusement éthyle ; un groupe -O(alkyle en C₁-C₆), avantageusement -OEt ; un groupe aryle, avantageusement phényle ; un groupe hétéroaryle, avantageusement pyridyle ; un groupe - (C=NH)NHCOO(alkyle en C₁-C₆), avantageusement -(C=NH)NHCOOt-butyle ; un groupe -(C=NH)NH₂; ou un groupe -(alkyle en C₁-C₆)NR¹⁰R¹¹, avantageusement -(CH₂)₂NR¹⁰R¹¹, dans lequel **R¹⁰** et **R¹¹** représentent indépendamment l'un de l'autre un groupe alkyle en C₁-C₆, avantageusement méthyle ; des exemples de groupes -(alkyle en C₁-C₆)CONHR⁹ sont -CH₂CONHOH, -CH₂CONHOEt et -CH₂CONH(CH₂)₂NMe₂;
   - un groupe -(alkyle en C₁-C₆)COmorpholine, avantageusement-CH₂COmorpholine;
   - un groupe -C(=O)R¹² dans lequel **R¹²** représente un groupe -O(alkyle en C₁-C₆), avantageusement -Oisobutyle (OiBu) ; un groupe alkyle en C₁-C₆, avantageusement éthyle ou isopropyle, éventuellement substitué par un groupe -OH, tel que -(CH₂)₂OH ; un groupe morpholine ; un groupe NH-aryle, avantageusement -NHPhényle, dans lequel le groupe aryle est éventuellement substitué par un groupe -COOH ou -COO(alkyle en C₁-C₆), avantageusement -COOMe ; ou un groupe -NR¹³R¹⁴ dans lequel **R¹³** et **R¹⁴** représentent indépendamment l'un de l'autre un groupe alkyle en C₁-C₆, avantageusement éthyle ; des exemples de groupes -C(=O)R¹² sont-C(=O)OiBu, -C(=O)(CH₂)₂OH, -C(=O)NEt₂, -C(=O)Morpholine, -C(=O)iPr,-C(=O)NH-3-HO₂C-Ph et -C(=O)NH-3-MeO₂C-Ph ;
   - un groupe (alkyle en C₁-C₆)aryle, avantageusement benzyle, dans lequel le groupe aryle est éventuellement substitué par un groupe -CN, -COOH ou -COO(alkyle en C₁-C₆) ; un exemple de groupe (alkyle en C₁-C₆)aryle substitué est -CH₂-3-NC-Ph ;
   - un groupe (alkyle en C₁-C₆)hétéroaryle, avantageusement (alkyle en C₁-C₆)thiophène, en particulier un groupe méthylthiophène ;
   - un groupe aryle, avantageusement phényle, dans lequel le groupe aryle est éventuellement substitué par un ou plusieurs groupes choisi parmi-COOH, -COO(alkyle en C₁-C₆), avantageusement -COOMe, -alkyle en C₁-C₆ substitué par un groupe -OH, avantageusement -CH₂OH, -CN, -CONHOH, - NHSO₂(alkyl en C₁-C₆), avantageusement -NHSO₂Me ou -CONH-(alkyle en C₁-C₆)NR¹⁵R¹⁶, avantageusement -CONH-(éthyle)NR¹⁵R¹⁶, dans lequel **R¹⁵** et **R¹⁶** représentent indépendamment l'un de l'autre un groupe alkyle en C₁-C₆, avantageusement méthyle ; des exemples de groupes aryle éventuellement substitué sont -Ph ; -2-MeO₂C-Ph, -2-HO₂C-Ph, -3-MeO₂C-Ph, -3-HO₂C-Ph -4-MeO₂C-Ph, -4-HO₂C-Ph ; -2-HO₂HC-Ph, -3-MeSO₂HN-Ph, -3-NC-Ph, -2-HONHOC-Ph, et -2-Me₂N(CH₂)₂HNOC-Ph ;
   - un groupe hétéroaryle, avantageusement pyridyle ;
   - un groupe hétérocyclique, avantageusement le tétrahydrofurane, en particulier comportant un ou deux hétéroatomes, avantageusement choisi parmi N, S et O, le groupe hétérocyclique pouvant éventuellement comporter une insaturation, tel que le thiazoline ;
   - un groupe lactone de 3 à 6 membres, en particulier la tétrahydrofuranone, éventuellement substitué par un ou plusieurs groupes alkyles en C₁-C₆, en particulier par deux groupes tels que deux groupes méthyles ;
   - ou un groupe -SO₂(alkyle en C₁-C₆), avantageusement -SO₂(isopropyle) ;
**R³** représente un groupe aryle, avantageusement phényle, ou hétéroaryle, avantageusement pyridyle ou thiényle, dans lequel le groupe aryle est éventuellement substitué par un ou plusieurs groupes choisi parmi -alkyle en C₁-C₆, avantageusement méthyle, dans lequel le groupe alkyle est éventuellement substitué par un ou plusieurs atomes d'halogène avantageusement F, tel que par exemple -CF₃, ou par un groupe -CN, tel que par exemple -CH₂CN; un atome d'halogène avantageusement F, - O(alkyle en C₁-C₆), avantageusement -OMe, dans lequel le groupe alkyle est éventuellement substitué par un ou plusieurs atomes d'halogène avantageusement F, tel que par exemple -OCF₃ ou -OCH₂F; -CN ; -OH ;-NO₂ ;-COOH ; -NR¹⁷R¹⁸ dans lequel **R¹⁷** et **R¹⁸** représentent indépendamment l'un de l'autre un groupe alkyle en C₁-C₆, avantageusement méthyle ; ou -NHCO-(alkyle en C₁-C₆), avantageusement -NHCOMe ; des exemples de groupes aryle éventuellement substitué sont -Ph, -2-F₃C-Ph, -3-F₃C-Ph, -4-F₃C-Ph, -2-F-Ph, -3-F-Ph, -4-F-Ph, -3,4-(F)₂-Ph, -3-F-4-F₃CO-Ph, -3-F₃CO-Ph, -4-F₃CO-Ph, -3-F₂HCO-Ph, -3-NC-Ph, -3-HO-Ph, -2-MeO-Ph, -3-MeO-Ph, -4-MeO-Ph, -3,4-(MeO)₂-Ph, -4-Me-Ph, -4-Me₂N-Ph, -4-O₂N-Ph, -3-HO₂C-Ph, -3-MeCOHN-Ph et -4-NCH₂C-Ph ;
ou un énantiomère, diastéréoisomère, hydrate, solvate, tautomère, mélange racémique ou sel pharmaceutiquement acceptable de celui-ci.

Dans un mode de réalisation particulier de la présente invention Y représente un atome d'oxygène ou le groupe -NH ou -NMe, avantageusement un atome d'oxygène.

Dans un autre mode de réalisation particulier de la présente invention, **R¹ R^{a}, R^{b}** et **R^{C}** représentent indépendamment l'un de l'autre un atome d'hydrogène, un atome d'halogène, en particulier Cl ou un groupe-O(alkyle en C₁-C₆) tel que OMe, en particulier un atome d'hydrogène ou un atome d'halogène. Avantageusement trois parmi **R¹ R^{a}, R^{b}** et **R^{c}** représentent un atome d'hydrogène et le quatrième d'entre eux représente un atome d'hydrogène, un atome d'halogène, en particulier Cl ou un groupe -O(alkyle en C₁-C₆) tel que OMe, avantageusement un atome d'hydrogène ou un atome d'halogène. De façon particulièrement avantageuse, **R¹ R^{a}, R^{b}** et **R^{c}** représentent un atome d'hydrogène.

Dans encore un autre mode de réalisation particulier de la présente invention, **R²** représente
- un groupe (alkyle en C₁-C₆)COOR⁸, avantageusement -H₂COOR⁸, dans lequel **R⁸** représente un atome d'hydrogène ou un groupe alkyle en C₁-C₆ en particulier méthyle (Me), éthyle (Et) ou isopropyle (iPr), des exemples de groupes -(alkyle en C₁-C₆)COOR⁸ sont -CH₂COOH, -CH₂COOMe,-CH₂COOEt et -CH₂COOiPr ;
- un groupe (alkyle en C₁-C₆)CONHR⁹, avantageusement -CH₂CONHR⁹, dans lequel **R⁹** représente un groupe -OH ; un groupe -O(alkyle en C₁-C₆), avantageusement -OEt, ou un groupe -(alkyle en C₁-C₆)NR¹⁰R¹¹ dans lequel **R¹⁰** et **R¹¹** représentent indépendamment l'un de l'autre un groupe alkyle en C₁-C₆, avantageusement méthyle ; des exemples de groupes - (alkyle en C₁-C₆)CONHR⁹ sont -CH₂CONHOH, -CH₂CONHOEt et-CH₂CONH(CH₂)₂NMe₂;
- un groupe aryle, avantageusement phényle, dans lequel le groupe aryle est éventuellement substitué par un ou plusieurs groupes choisi parmi-COOH, -COO(alkyle en C₁-C₆), avantageusement -COOMe, -CONHOH, ou -CONH-(alkyle en C₁-C₆)NR¹⁵R¹⁶, avantageusement -CONH-(éthyle)NR¹⁵R¹⁶, dans lequel **R¹⁵** et **R¹⁶** représentent indépendamment l'un de l'autre un groupe alkyle en C₁-C₆, avantageusement méthyle ; des exemples de groupes aryle éventuellement substitué sont -Ph ; -2-MeO₂C-Ph, -2-HO₂C-Ph, -3-MeO₂C-Ph, -3-HO₂C-Ph, -4-MeO₂C-Ph, -4-HO₂C-Ph ;-2-HONHOC-Ph, et -2-Me₂N(CH₂)₂HNOC-Ph ; ou
- un groupe -CONH aryle, avantageusement -CONHphényle, éventuellement substitué par un groupe -COOH ou -COO(alkyle en C₁-C₆) avantageusement -COOMe.

De façon avantageuse **R²** représente un groupe -(alkyle en C₁-C₆)COOR⁸ dans lequel **R⁸** représente un atome d'hydrogène ou un groupe alkyle en C₁-C₆ en particulier méthyle (Me), éthyle (Et) ou isopropyle (iPr), ou un groupe aryle, avantageusement phényle dans lequel le groupe aryle est éventuellement substitué par un ou plusieurs groupes, en particulier un groupe, choisi parmi -COOH, -COO(alkyle en C₁-C₆), avantageusement-COOMe. De façon particulièrement avantageuse **R²** représente un groupe -(alkyle en C₁-C₆)COOH, avantageusement -CH₂COOH.

Dans encore un autre mode de réalisation particulier de la présente invention **R³** représente un groupe aryle, avantageusement phényle, éventuellement substitué par un ou plusieurs groupes choisi parmi -alkyle en C₁-C₆, avantageusement méthyle, dans lequel le groupe alkyle est éventuellement substitué par un ou plusieurs atomes d'halogène avantageusement F, tel que par exemple -CF₃; un atome d'halogène avantageusement F, - O(alkyle en C₁-C₆), avantageusement -OMe, dans lequel le groupe alkyle est éventuellement substitué par un ou plusieurs atomes d'halogène avantageusement F, tel que par exemple -OCF₃ ou-OCH₂F ; -CN ; ou -NO₂. ; des exemples de groupes aryle éventuellement substitué sont -Ph, -2-F₃C-Ph, -3-F₃C-Ph, -4-F₃C-Ph, -2-F-Ph, -3-F-Ph, -4-F-Ph, -3,4-(F)₂-Ph, -3-F-4-F₃CO-Ph, -3-F₃CO-Ph, -4-F₃CO-Ph, -3-F₂HCO-Ph,-3-NC-Ph, -2-MeO-Ph, -3-MeO-Ph, -4-MeO-Ph, -3,4-(MeO)₂-Ph, -4-Me-Ph et -4-O₂N-Ph ;
De façon avantageuse **R³** représente un groupe phényle substitué par un ou plusieurs groupes choisi parmi -alkyle en C₁-C₆, avantageusement méthyle, substitué par un ou plusieurs atomes d'halogène, avantageusement F, tel que par exemple -CF₃; un atome d'halogène avantageusement F, - O(alkyle en C₁-C₆)), avantageusement -OMe, dans lequel le groupe alkyle est substitué par un ou plusieurs atomes d'halogène, avantageusement F, tel que par exemple -OCF₃ ; ou -CN.

Dans le cadre de la présente invention on entend par « groupe aryle », un cycle aromatique ayant 5 à 8 atomes de carbones ou plusieurs cycles aromatiques fusionnés ayant 5 à 14 atomes de carbones. En particulier, les groupes aryles peuvent être des groupes monocycliques ou bicycliques, de préférence phényle ou naphthyle. Avantageusement il s'agit d'un groupe phényle (Ph).

Dans le cadre de la présente invention on entend par « groupe hétéroaryle », tout groupe aromatique hydrocarboné de 3 à 9 atomes contenant un ou plusieurs hétéroatomes, tels que par exemple des atomes de soufre, azote ou oxygène. L'hétéroaryle selon la présente invention peut être constitué par un ou plusieurs cycles fusionnés. Des exemples de groupes hétéroaryle sont les groupes furyle, isoxazyle, pyridyle, thiazolyle, pyrimidyle, benzimidazole, benzoxazole, thiényle, thiophènyle, benzothiazole. Avantageusement le groupe hétéroaryle est choisi parmi les groupes furyle, pyridyle, thiazolyle, thiophènyle et thiényle. De façon avantageuse il s'agit du groupe pyridyle, thiényle ou thiophènyle.

Dans le cadre de la présente invention on entend par « groupe hétérocyclique », tout groupe hydrocarboné cyclique saturé de 3 à 9 atomes contenant un ou plusieurs hétéroatomes, tels que par exemple des atomes de soufre, azote ou oxygène. Le groupe hétérocyclique selon la présente invention peut être constitué par un ou plusieurs cycles fusionnés. Des exemples de groupes hétérocycliques sont les groupes tétrahydrofurane, pyrrolidyl, pipéridyl, thiolane, oxirane, oxane, thiane, thiazolidine, morpholine. Avantageusement le groupe hétérocyclique est choisi parmi les groupes tétrahydrofurane, thiazolidine et morpholine. Dans le cadre de la présente invention on entend par «atome d'halogène» tout atome d'halogène, avantageusement choisi parmi CI, Br, I ou F, en particulier choisi parmi F, Cl ou Br, en particulier Cl ou F.

Dans le cadre de la présente invention on entend par « groupe alkyle en C₁-C₆ », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle, éthyle, isobutyle, ou iso-propyle, en particulier d'un groupe méthyle ou éthyle, plus particulièrement d'un groupe méthyle.

Dans le cadre de la présente invention on entend par «groupe (alkyle en C₁-C₆)aryle », tout groupe aryle tel que défini ci-dessus, lié par l'intermédiaire d'un groupe alkyle en C₁-C₆ tel que défini ci-dessus. En particulier un exemple de groupe (alkyle en C₁-C₆)aryle est un groupe benzyle.

Dans le cadre de la présente invention on entend par «groupe (alkyle en C₁-C₆)hétéroaryle », tout groupe hétéroaryle tel que défini ci-dessus, lié par l'intermédiaire d'un groupe alkyle en C₁-C₆ tel que défini ci-dessus. En particulier un exemple de groupe (alkyle en C₁-C₆)hétéroaryle est un groupe méthylthiophène.

Dans le cadre de la présente invention on entend par «pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine. Dans le cadre de la présente invention on entend par « sels pharmaceutiquement acceptables d'un composé » des sels qui sont pharmaceutiquement acceptables, comme défini ici, et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Dans le cadre de la présente invention on entend par « solvate d'un composé », tout composé obtenu par addition d'une molécule de solvant inerte sur le composé selon l'invention, le solvate se formant en raison de leur force d'attraction mutuelle. Les solvates sont par exemple des alcoolates du composé. Un hydrate est un solvate dans lequel le solvant inerte utilisé est l'eau. Il peut être mono, di ou trihydraté
Dans le cadre de la présente invention on entend par « tautomère » tout isomère de constitution des composés selon la présente invention qui sont interconvertibles par la réaction chimique réversible appelée tautomérisation. Dans la plupart des cas, la réaction se produit par migration d'un atome d'hydrogène accompagnée d'un changement de localisation d'une double liaison. Dans une solution d'un composé capable de tautomérisation, un équilibre entre les 2 tautomères se crée. Le rapport entre tautomères est alors fonction du solvant, de la température et du pH. La tautomérie est donc la transformation d'un groupement fonctionnel en un autre, le plus souvent par déplacement concomitant d'un atome d'hydrogène et d'une liaison n (liaison double ou triple). Des tautomères courants sont par exemple les paires aldéhydes / cétones - alcools ou plus précisément énol; amides - acides imidiques; lactames - lactimes ; imines - énamines ; énamines - énamines. En particulier il peut inclure une tautomérie cycle - chaîne qui a lieu lorsque le mouvement du proton est accompagné par la transformation d'une structure ouverte à un cycle.

Dans un mode de réalisation particulièrement intéressant de la présente invention, les dérivés d'imidazopyridine sont choisis parmi les composés de formule 1 à 138 tels qu'indiqués dans le tableau 1 ci-après.

Dans un autre mode de réalisation encore plus intéressant les dérivés d'imidazopyridine sont choisis parmi les 84 composés numérotés 1-3, 5, 7, 10-12, 14, 17, 18, 20, 23, 25-27, 29, 30, 32, 35, 38-40, 42, 44, 45, 46, 47, 48, 51, 54, 55, 57-59, 68, 72-79, 81, 83, 87-90, 92, 93, 95, 96, 101-126, 129-131 et 135 indiqués dans le tableau 1 ci-après.

Encore plus avantageusement il s'agit des composés 3, 7, 14, 25, 26, 29, 44, 51, 54, 58, 59, 72, 76-79, 81, 87-90, 92, 93, 101, 102, 104, 110-112, 114-120 et 135 indiqués dans le tableau 1 ci-après.

La présente invention concerne en outre une composition pharmaceutique comprenant un dérivé d'imidazopyridine selon la présente invention et un excipient pharmaceutiquement acceptable.

Ces compositions peuvent être formulées pour l'administration aux mammifères, y compris l'homme. La posologie varie selon le traitement et selon l'affection en cause. Ces compositions pharmaceutiques sont adaptées pour une administration par n'importe quelle voie appropriée, par exemple par voie orale (incluant buccale et sublinguale), par voie rectale, nasale, topique (incluant transdermique), vaginale, intraoculaires ou parentérale (incluant sous-cutanée, intramusculaire ou intraveineuse). Avantageusement les compositions pharmaceutiques sont adaptées pour une administration par voie orale. Ces formulations peuvent être préparées en utilisant tous les procédés connus par l'homme du métier en combinant les ingrédients actifs avec les excipients pharmaceutiquement acceptables appropriés.

Les formes unitaires d'administrations appropriées par voie orale comprennent les comprimés, les gélules, les poudres, les granules et les solutions ou suspensions orales dans des liquides aqueux ou non aqueux, les mousses comestibles ou alimentaires, ou les émulsions liquides eau-dans-huile ou huile-dans-eau. Lorsque l'on prépare une composition solide sous forme de comprimé, on mélange l'ingrédient actif principal, avantageusement sous forme de poudre, avec un excipient pharmaceutique approprié tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif, avantageusement sous forme de poudre, avec un diluant et en versant le mélange obtenu dans des gélules molles ou dures, en particulier des gélules de gélatine. Des lubrifiants tels que par exemple le talc, le stéarate de magnésium, le stéarate de calcium ou le polyéthylène glycol sous forme solide peuvent être ajoutés dans la composition avant sa mise en gélule. Un désintégrant ou un solubilisant tels que par exemple le carbonate de calcium ou le carbonate de sodium peuvent également être ajoutés afin d'améliorer la disponibilité du médicament après la prise de la gélule.

En outre on peut ajouter si nécessaire dans le mélange, des liants, des lubrifiants et des désintégrant appropriés de même que des colorants. Les liants appropriés peuvent être par exemple l'amidon, la gélatine, les sucres naturels tels que par exemple le glucose ou le bêta-lactose, des agents sucrants fabriqués à partir de maïs, du caoutchouc synthétique ou naturel tel que par exemple l'acacia ou l'alginate de sodium, de la carboxyméthylcellulose, du polyéthylène glycol, des cires et similaires. Les lubrifiants utilisables dans ces formes de dosage incluent l'oléate de sodium, le stéarate de sodium, le stéarate de magnésium, le benzoate de sodium, l'acétate de sodium, le chlorure de sodium et similaires. Les désintégrant incluent l'amidon, la méthylcellulose, l'agar, la bentonite, la gomme de xanthane et similaires. Les comprimés sont formulés par exemple par préparation d'un mélange de poudre, granulation ou pressage à sec du mélange, addition d'un lubrifiant et d'un désintégrant et pressage du mélange pour donner les comprimés. Un mélange de poudre est préparé par mélange du principe actif additionné de façon appropriée avec un diluant ou une base et optionnellement avec un liant tel que par exemple la carboxyméthylcellulose, l'alginate, la gélatine ou la polyvinylpyrrolidone, un retardant de dissolution tel que par exemple la paraffine, un accélérant d'absorption tel que par exemple un sel quaternaire et/ou un absorbant tel que par exemple la bentonite, le kaolin ou le phosphate dicalcique. Les mélanges de poudres peuvent être granulés par mouillage avec un liant tel que par exemple un sirop, une pâte d'amidon, du mucilage d'acadia ou des solutions de cellulose ou de matériaux polymères et pressage à travers un tamis. Les granules peuvent être lubrifiés par addition d'acide stéarique, de sel de stéarate, de talc ou d'une huile minérale de façon à éviter qu'ils collent aux moules permettant la fabrication des comprimés. Le mélange lubrifié est alors pressé pour donner les comprimés. Une couche protectrice opaque ou transparente consistant en une couche shellac, une couche de sucre ou de matériaux polymères est éventuellement présente. Des colorants peuvent être ajoutés à ces enrobages façon à les différencier des autres comprimés. Une préparation sous forme de sirop ou d'élixir peut contenir l'ingrédient actif conjointement avec un édulcorant, un antiseptique, ainsi qu'un agent donnant du goût et un colorant approprié. En général les préparations de sirop sont obtenues par dissolution du composé dans une solution aqueuse avec un agent donnant du goût approprié alors que les élixirs sont préparés en utilisant un véhicule alcoolique non toxique.

Les poudres ou les granules dispersibles dans l'eau peuvent contenir l'ingrédient actif en mélange avec des agents de dispersion ou des agents mouillants, ou des agents de mise en suspension, tels que par exemple des alcools isostéaryle éthoxylés et des éthers de sorbitol polyoxyéthylène, et de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les compositions pharmaceutiques adaptées pour une administration par voie topique peuvent être formulées sous forme de crème, d'onguent, de suspension, de lotion, de poudre, de solution, de pâte, de gel, de spray, d'aérosols ou d'huiles.

Des compositions pharmaceutiques adaptées à l'administration par voie nasale dans lequel l'excipient support est à l'état solide comprennent des poudres ayant des tailles de particules par exemple dans la gamme de 20 à 500 microns, administrées par inhalation à partir d'un récipient contenant la poudre disposé à proximité du nez.

Les formulations pharmaceutiques adaptées à l'administration par voie vaginale peuvent assez être administrées sous forme de tampon, crème, gel, pâtes, mousse ou spray.

Dans un mode de réalisation avantageux, la composition pharmaceutique selon la présente invention comprend en outre un autre agent actif, ayant avantageusement un effet complémentaire ou synergique En particulier cet agent actif est un autre antidiabétique, avantageusement choisi parmi l'insuline, les sulfonylurées, les glinides, les biguanides, les thiazolidinediones, les agonistes GLP-1R, les inhibiteurs de DPP-IV, les inhibiteurs SGLT-2, de façon avantageuse choisie parmi l'insuline, le glibenclamide, le gliclazide, le glipizide, le glimepiride, le repaglinide, le nateglinide, la metformine, la troglitazone, la rosiglitazone, la pioglitazone, l'exenatide, le liraglutide, la sitagliptine, la vildagliptine, la saxagliptine, l'alogliptine, la dapagliflozine. Plus particulièrement il s'agit de la metformine.

Ce second agent actif peut être administré dans la même composition pharmaceutique que le dérivé d'imidazopyridine de la présente invention. Il peut être aussi administré séparément, soit au même moment soit de façon étalée dans le temps. Avantageusement ce second agent actif est administré par voie orale.

La présente invention concerne de plus un dérivé d'imidazopyridine selon la présente invention pour utilisation à titre de médicament.

La présente invention concerne également l'utilisation d'un dérivé d'imidazopyridine selon la présente invention pour la préparation d'un médicament.

Selon la présente invention, les composés de formule (I) présentent une activité antihyperglycémiante. Ils peuvent réduire l'hyperglycémie, plus particulièrement l'hyperglycémie du diabète de type 2. Notamment, les composés de l'invention présentent une activité antihyperglycémique et sont donc utiles dans le traitement et/ou la prévention du diabète, de ses complications et/ou des pathologies associées, tel que par exemple les pathologies associées au syndrome métabolique, avantageusement le diabète de type 2 ou l'hyperglycémie. Ces médicaments sont particulièrement actifs chez les personnes âgées. On entend par « personnes âgées » des personnes, homme ou femmes, ayant 65 ans ou plus.

Le terme « résistance à l'insuline » tel qu'utilisé dans le cadre de la présente invention, se réfère à une condition ou une quantité normale d'insuline incapable de produire une réponse physiologique ou moléculaire normale.

La présente invention concerne donc un dérivé d'imidazopyridine selon l'invention pour utilisation à titre de médicament destiné au traitement et/ou à la prévention du diabète, de ses complications et/ou des pathologies associées, avantageusement du diabète de type 2 et de l'hyperglycémie.

Les inventeurs ont découvert que les dérivés selon la présente invention permettaient de stimuler la sécrétion d'insuline par les cellules INS1 et d'inhiber la production hépatique de glucose au niveau d'hépatocytes isolés de rat.

Avantageusement le diabète est choisi parmi les diabètes précoces, tardifs, pédiatriques, des personnes âgées et gestationnels, en particulier des personnes âgées. De façon avantageuse les défauts du diabète et les complications et/ou pathologies associées au diabète sont choisies parmi l'hyperglycémie, les anomalies fonctionnelles et quantitatives des cellules pancréatiques endocrines, la résistance à l'insuline, la neuropathie diabétique, la néphropathie diabétique, la rétinopathie diabétique, l'inflammation, l'obésité, l'hypertension, les problèmes cardiovasculaires, microvasculaires, neurologiques et de cicatrisation des plaies. Avantageusement il s'agit de l'hyperglycémie, des anomalies fonctionnelles et quantitatives des cellules pancréatiques endocrines, de la résistance à l'insuline et de l'inflammation.

De façon avantageuse le patient traité présente des facteurs de risques associés au diabète, c'est-à-dire un taux d'affection directement ou indirectement associée avec l'apparition du diabète. En particulier cela comprend l'historique familial, le diabète gestationnel, l'excès de poids, l'obésité, l'insuffisance d'exercice physique, l'hypertension, un haut niveau de triglycérides, l'inflammation et l'hyperlipidémie.

La présente invention concerne de plus l'utilisation d'un dérivé d'imidazopyridine selon l'invention pour la fabrication d'un médicament destiné au traitement et/ou à la prévention du diabète, de ses complications et/ou des pathologies associées, avantageusement du diabète de type 2 et de l'hyperglycémie.

Elle concerne enfin une méthode de traitement et/ou de prévention et/ou de traitement prophylactique et/ou pour retarder l'apparition du diabète, de ses complications et/ou des pathologies associées, avantageusement du diabète de type 2 et de l'hyperglycémie, comprenant l'administration d'une quantité efficace d'un dérivé d'imidazopyridine selon l'invention à un patient qui en a besoin.

La quantité efficace sera adaptée selon la nature et la sévérité de la pathologie à traiter, la voie d'administration et aussi le poids et l'âge du patient. En générale l'unité de doses variera entre 0,5 mg et 2000 mg par jour, en une prise ou plusieurs, avantageusement entre 1 et 1000 mg.

Les dérivés d'imidazopyridine selon l'invention sont fabriqués par des méthodes bien connues de l'homme du métier est en partie par des méthodes telles que décrites ci-après.

L'invention sera mieux comprise à la lecture de la description et des exemples qui suivent qui sont donnés à titre indicatif non limitatif.

### Description des synthèses et schémas généraux

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les procédures qui suivent.

La synthèse des molécules de formule générale (I) est proche, parfois identique, de ce qui a été décrit dans les documents [1] à [8] sans que cette liste de références puisse être considérée comme exhaustive.

Les différents groupements R¹ à R³ et Y des schémas 1 à 4 font référence aux définitions précédemment données ; « GP » signifie groupement protecteur ; « Hal » fait référence à un atome d'halogène.
**Schéma 1 :** Les dérivés de formule I peuvent être préparés selon un procédé connu partant de la 2-aminopyridine qui réagit en une étape avec la 1,3-dichloropropan-2-one pour former l'hétérocycle **1.2.** Le chlore des dérivés de type **1.2** est particulièrement adapté pour être impliqué dans différentes réactions de *O*-, S- ou *N*-Alkylation réalisées en milieu basique avec des alcoolates, phénates et amines secondaires. Une fois la chaîne introduite, une réaction d'halogénation (de préférence une réaction d'iodation) sélective permet d'obtenir les dérivés de type **1.4** qui sont engagés dans des réactions de type Sonogashira pour obtenir les dérivés de type **1.5.** 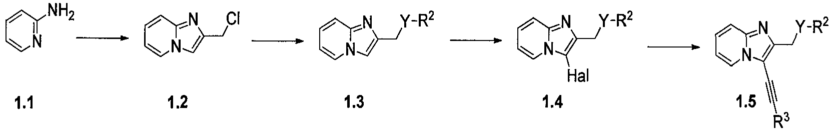
**Schéma 2 :** Dans le cas particulier de Y = NH, il est avantageux d'utiliser une autre approche pour la synthèse des dérivés de formule I. 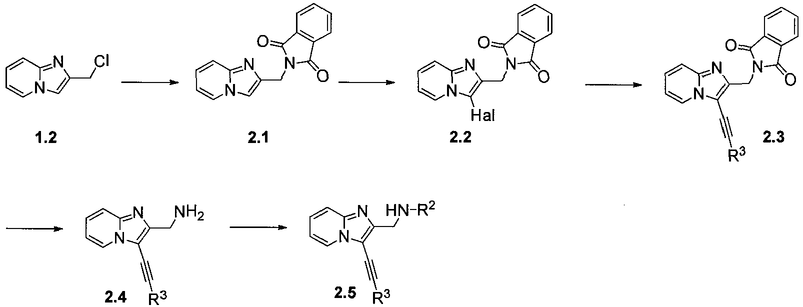 Partant du chloré **1.2,** une synthèse d'amine par la méthode de Gabriel est réalisée. Le dérivé phtalimide **2.1** est halogéné (de préférence iodé) pour être ensuite engagé dans des réactions de type Sonogashira afin d'obtenir les dérivés de type **2.3,** l'amine primaire est libérée en conditions basiques. Les dérivés de type **2.4** peuvent ensuite être impliqués avantageusement dans des réactions d'aminations réductrices, d'acylations ou encore mis en présence d'isocyanates afin d'obtenir des amines secondaires, des amides, des carbamates ou encore des urées de type **2.5.**
**Schéma 3 :** Une méthode alternative à la synthèse des dérivés de formule I pour lesquels Y = O a été évaluée partant du imidazo[1,2-a]pyridin-2-ylméthanol **2.1** qui peut être protégé par un groupement protecteur adapté (par exemple le groupement triisopropylsilyle) afin d'être engagé dans une réaction d'halogénation (de préférence une réaction d'iodation), suivie d'une réaction de type Sonogashira. Le groupement protecteur est clivé suite à la mise en oeuvre des conditions adaptées (fluorure de tétrabutylammonium dans le cas d'une protection par un groupement triisopropylsilyle), l'alcool primaire est ensuite déprotoné par une base et engagé dans des réactions d'alkylations, voir d'acylation, pour obtenir des dérivés de type **3.6.** 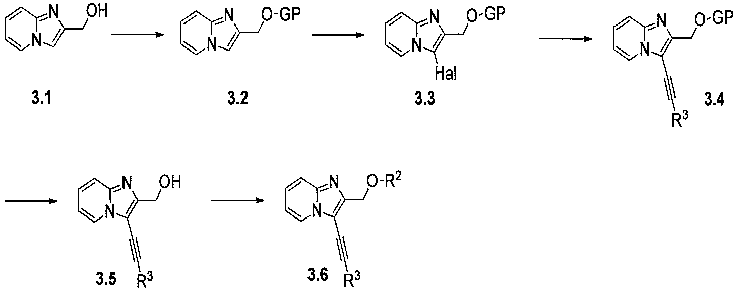
**Schéma** 4 : Une deuxième méthode alternative à la synthèse des dérivés de formule I pour lesquels Y = O a été évaluée. Cette approche fait appel à une stratégie de départ différente pour la construction de l'hétérocycle puisque la 2-aminopyridine est mise en présence de bromo pyruvate d'éthyle pour former le dérivé ester **4.1** qui est ensuite engagé dans une réaction d'halogénation (de préférence une réaction d'iodation), suivie d'une réaction de type Sonogashira. La fonction ester est réduite en alcool par des méthodes classiques (réaction avec un hydrure d'alumimiun) afin d'engager les dérivés de type **3.5** dans des réactions d'alkylation, voir d'acylation.

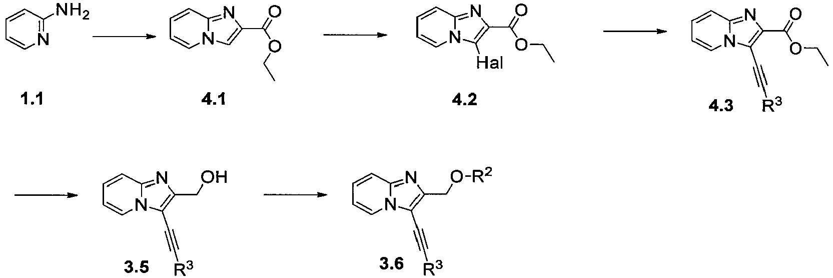

### EXEMPLES:

### Matériel et méthode

Les spectres de résonance magnétique nucléaire (**RMN**) du proton **¹H** sont effectués sur un appareil Bruker Avance DPX300 (300,16 MHz). Les déplacements chimiques (δ) sont mesurés en partie par million (**ppm**). Les spectres sont calibrés sur le déplacement chimique du solvant deutéré utilisé. Les constantes de coupage (**J**) sont exprimées en Hertz (**Hz**) et la multiplicité est représentée de la manière suivante, singulet (s), doublet (d), doublet de doublet (dd), triplet (t), triplet de doublet (td), quadruplet (q), multiplet (m). Les spectres de masse (**SM**) sont réalisés par un spectromètre Agilent Technologies MSD, type G1946A, les échantillons sont ionisés par une source « Atmospheric pressure chemical ionization » (**APCI**).

### Abréviations

- AIBN: azoisobutyronitrile
- dba: dibenzylidèneacétone
- DIAD: diisopropylazadicarboxylate
- DME: 1,2-diméthoxyéthane
- EDC: *N*-(3-diméthylaminopropyl)-*N'*-éthylcarbodiimide
- HOBt: 1-hydroxybenzotriazole
- LAH: hydrure d'aluminium et de lithium
- RED-AI: Dihydrobis(2-méthoxyéthoxy) aluminate de sodium
- TIPS: triisopropylsilyle
- CDCl3: chloroforme deutéré
- DMSO: diméthylsulfoxyde deutéré
- PyBOP: (benzotriazol-1-yl-oxytripyrrolidinophosphonium hexafluorophosphate)
- DMPU: 1,3-Diméthyl-3,4,5,6-tetrahydro-2(1H)-pyrimidinone
- DMF: diméthylformamide
- Boc: tert-butoxycarbonyle
- mmol: milli mole(s)
- µM: micromolaire
- ml: milli litre(s)
- g: gramme(s)
- M: mol / litre
- N: normal(e)
- nm: nano mètre(s)
- min: minute(s)
- h: heure(s)
- j: jour(s)
- t.a.: température ambiante
- UV: ultra violet
- ctrl: contrôle
- HGP: Production Hépatique de Glucose

La liste des exemples ci-dessous sert à illustrer le propos de cette invention et non à en limiter le champ d'application.

**Tableau 1 : Liste des molécules dont la synthése est exemplifiée**

| **N°** | **Structure chimique** | **Nom chimique** |
|---|---|---|
| **1** | 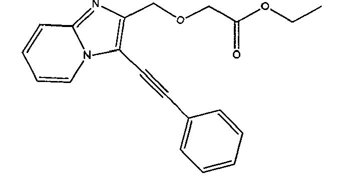 | 2-((3-(phényléthynyl)imidazo[1,2-a]pyridin-2-yl)méthoxy)acétate d'éthyle |
| **2** | 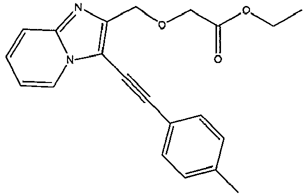 | 2-((3-(p-tolyléthynyl)imidazo[1,2-a]pyridin-2-yl)méthoxy)acétate d'éthyle |
| **3** | 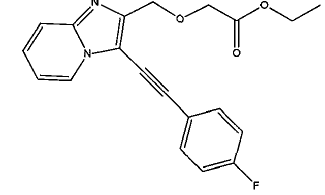 | 2-((3-((4-fluorophényl)éthynyl)imidazo[1,2-a)pyridin-2-yl)méthoxy)acétate d'éthyle |
| **4** | 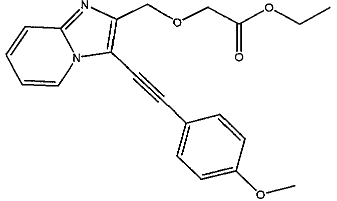 | 2-((3-((4-méthoxyphényl)éthynyl)imidazo[1,2-a)pyridin-2-yl)méthoxy)acétate d'éthyle |
| **5** | 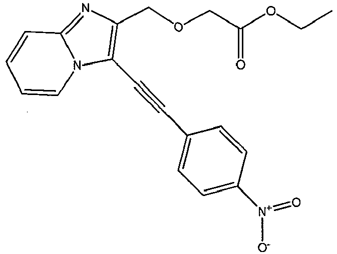 | 2-((3-((4-nitrophényl)éthynyl)imidazo[1,2-a]pyridin-2-yl)méthoxy)acétate d'éthyle |
| **6** | 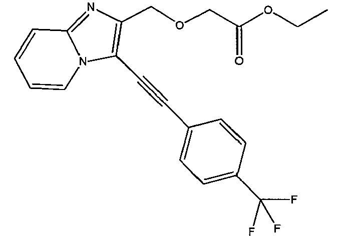 | 2-((3-((4-(trifluorométhyl)phényl)éthynyl)imid azo[1,2-a]pyridin-2-yl)méthoxy)acétate d'éthyle |
| **7** | 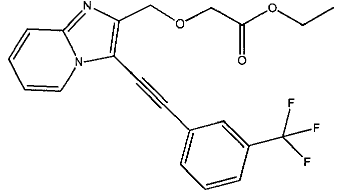 | 2-((3-((3-(trifluorométhyl)phényl)éthynyl)imid azo[1,2-a]pyridin-2-yl)méthoxy)acétate d'éthyle |
| **8** | 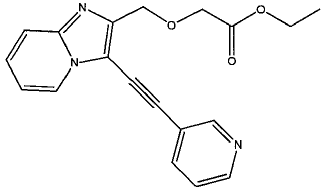 | 2-((3-(pyridin-3-yléthynyl)imidazo[1,2-a]pyridin-2-yl)méthoxy)acétate d'éthyle |
| **9** | 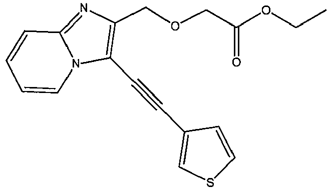 | 2-((3-(thiophén-3-yléthynyl)imidazo[1,2-a]pyridin-2-yl)méthoxy)acétate d'éthyle |
| **10** | 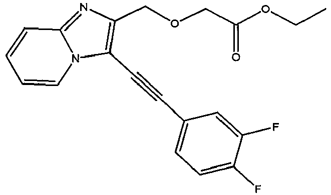 | 2-((3-((3,4-difluorophényl)éthynyl)imidazo[1,2-a]pyridin-2-yl)méthoxy)acétate d'éthyle |
| **11** | 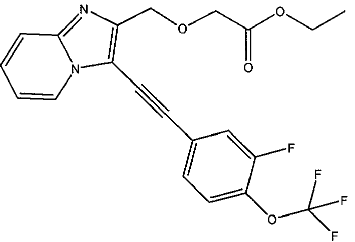 | 2-((3-((3-fluoro-4-(trifluorométhoxy)phényl)éthynyl)imi dazo[1,2-a]pyridin-2-yl)méthoxy)acétate d'éthyle |
| **12** | 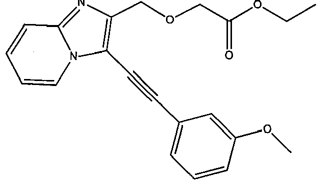 | 2-((3-((3-méthoxyphényl)éthynyl)imidazo[1,2-a]pyridin-2-yl)méthoxy)acétate d'éthyle |
| **13** | 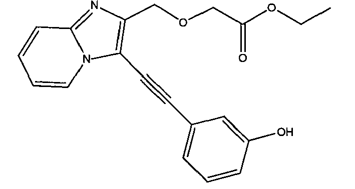 | 2-((3-((3-hydroxyphényl)éthynyl)imidazo[1,2-a]pyridin-2-yl)méthoxy)acétate d'éthyle |
| **14** | 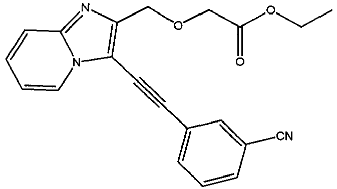 | 2-((3-((3-cyanophényl)éthynyl)imidazo[1,2-a]pyridin-2-yl)méthoxy)acétate d'éthyle |
| **15** | 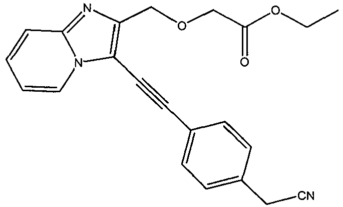 | 2-((3-((4-(cyanométhyl)phényl)éthynyl)imidaz o[1,2-a]pyridin-2-yl)méthoxy)acétate d'éthyle |
| **16** | 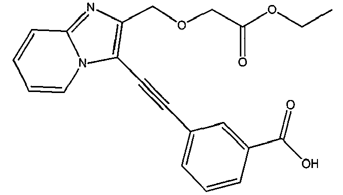 | 3-((2-((2-éthoxy-2-oxoéthoxy)méthyl)imidazo[1,2-a]pyridin-3-yl)éthynyl) benzoïque |
| **17** | 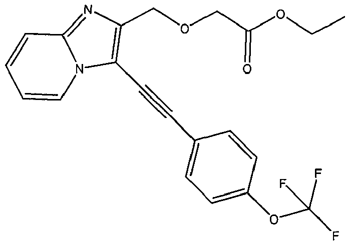 | 2-((3-((4-(trifluorométhoxy)phényl)éthynyl)imi dazo[1,2-a]pyridin-2-yl)méthoxy)acétate d'éthyle |
| **18** | 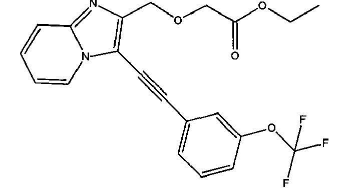 | 2-((3-((3-(trifluorométhoxy)phényl)éthynyl)imi dazo[1,2-a]pyridin-2-yl)méthoxy)acétate d'éthyle |
| **19** | 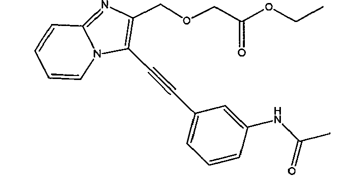 | 2-((3-((3-acétamidophényl)éthynyl)imidazo[1, 2-a]pyridin-2-yl)méthoxy)acétate d'éthyle |
| **20** | 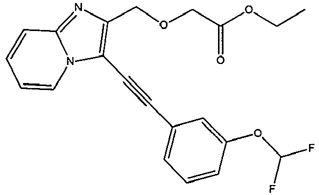 | 2-((3-((3-(difluorométhoxy)phényl)éthynyl)imi dazo[1,2-a]pyridin-2-yl)méthoxy)acétate d'éthyle |
| **21** | 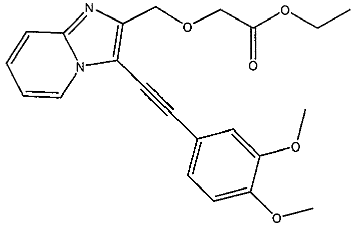 | 2-((3-((3,4-diméthoxyphényl)éthynyl)imidazo[1, 2-a]pyridin-2-yl)méthoxy)acétate d'éthyle |
| **22** | 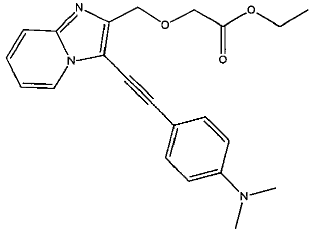 | 2-((3-((4-(diméthylamino)phényl)éthynyl)imid azo[1,2-a]pyridin-2-yl)méthoxy)acétate d'éthyle |
| **23** | 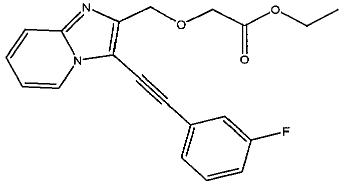 | 2-((3-((3-fluorophényl)éthynyl)imidazo[1,2-a]pyridin-2-yl)méthoxy)acétate d'éthyle |
| **24** | 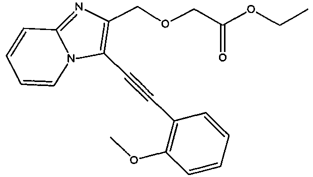 | 2-((3-((2-méthoxyphényl)éthynyl)imidazo[1,2-a]pyridin-2-yl)méthoxy)acétate d'éthyle |
| **25** | 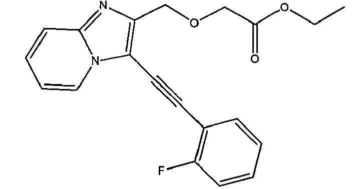 | 2-((3-((2-fluorophényl)éthynyl)imidazo[1,2-a]pyridin-2-yl)méthoxy)acétate d'éthyle |
| **26** | 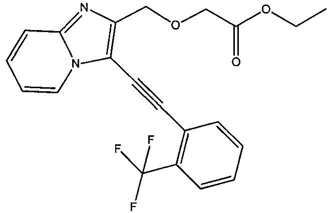 | 2-((3-((2-(trifluorométhyl)phényl)éthynyl)imid azo[1,2-a]pyridin-2-yl)méthoxy)acétate d'éthyle |
| **27** | 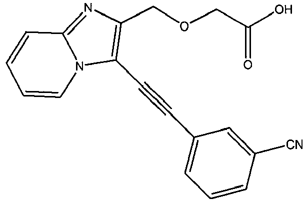 | Acide 2-((3-((3-cyanophényl)éthynyl)imidazo[1,2-a]pyridin-2-yl)méthoxy) acétique |
| **28** | 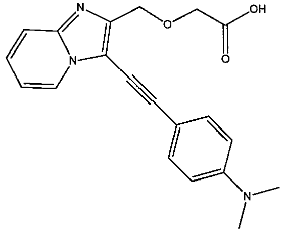 | Acide 2-((3-((4-(diméthylamino)phényl)éthynyl)imid azo[1,2-a]pyridin-2-yl)méthoxy) acétique |
| **29** | 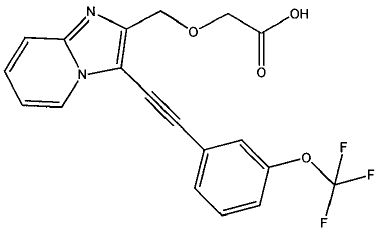 | Acide 2-((3-((3-(trifluorométhoxy)phényl)éthynyl)imi dazo[1,2-a]pyridin-2-yl)méthoxy) acétique |
| **30** | 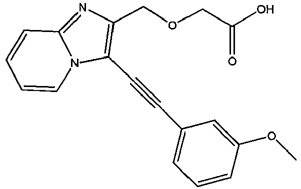 | Acide 2-((3-((3-méthoxyphényl)éthynyl)imidazo[1,2-a]pyridin-2-yl)méthoxy) acétique |
| **31** | 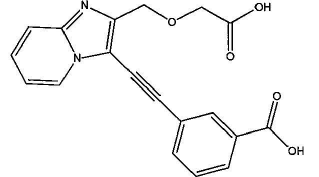 | Acide 3-((2-((carboxyméthoxy)méthyl)imidazo[1, ,2-a]pyridin-3-yl)éthynyl) benzoïque |
| **32** | 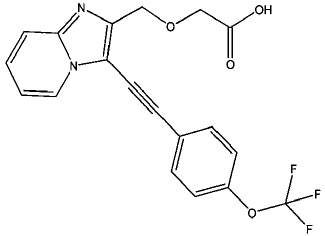 | Acide 2-((3-((4-(trifluorométhoxy)phényl)éthynyl)imi dazo[1,2-a]pyridin-2-yl)méthoxy) acétique |
| **33** | 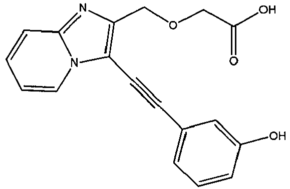 | Acide 2-((3-((3-hydroxyphényl)éthynyl)imidazo[1,2-a]pyridin-2-yl)méthoxy) acétique |
| **34** | 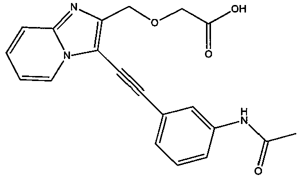 | Acide 2-((3-((3-acétamidophényl)éthynyl)imidazo[1, 2-a]pyridin-2-yl)méthoxy) acétique |
| **35** | 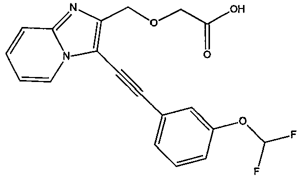 | Acide 2-((3-((3-(difluorométhoxy)phényl)éthynyl)imi dazo[1,2-a]pyridin-2-yl)méthoxy) acétique |
| **36** | 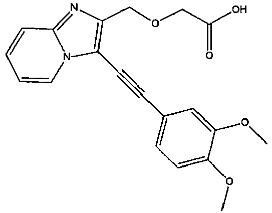 | Acide 2-((3-((3,4-diméthoxyphényl)éthynyl)imidazo[1, 2-a]pyridin-2-yl)méthoxy) acétique |
| **37** | 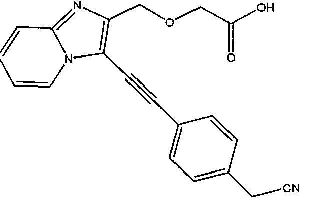 | Acide 2-((3-((4-(cyanométhyl)phényl)éthynyl)imidaz o[1,2-a]pyridin-2-yl)méthoxy) acétique |
| **38** | 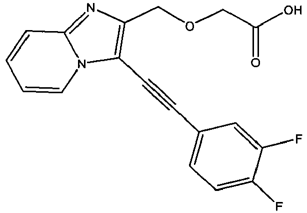 | Acide 2-((3-((3,4-difluorophényl)éthynyl)imidazo[1,2-a]pyridin-2-yl)méthoxy) acétique |
| **39** | 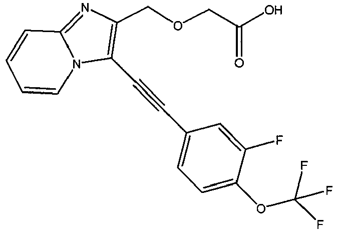 | Acide 2-((3-((3-fluoro-4-(trifluorométhoxy)phényl)éthynyl)imi dazo[1,2-a]pyridin-2-yl)méthoxy) acétique |
| **40** | 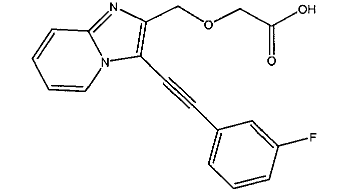 | Acide 2-((3-((3-fluorophényl)éthynyl)imidazo[1,2-a]pyridin-2-yl)méthoxy) acétique |
| **41** | 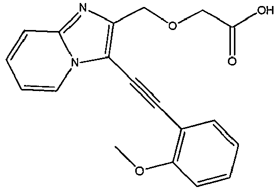 | Acide 2-((3-((2-méthoxyphényl)éthynyl)imidazo[1,2-a]pyridin-2-yl)méthoxy) acétique |
| **42** | 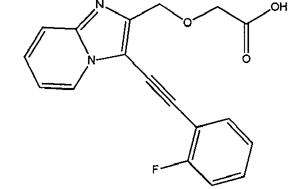 | Acide 2-((3-((2-fluorophényl)éthynyl)imidazo[1,2-a]pyridin-2-yl)méthoxy) acétique |
| **43** | 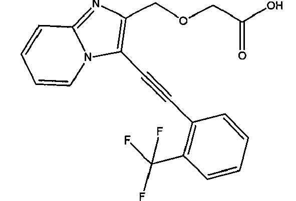 | Acide 2-((3-((2-(trifluorométhyl)phényl)éthynyl)imid azo[1,2-a]pyridin-2-yl)méthoxy) acétique |
| **44** | 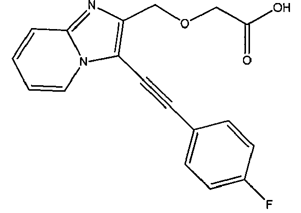 | Acide 2-((3-((4-fluorophényl)éthynyl)imidazo[1,2-a]pyridin-2-yl)méthoxy) acétique |
| **45** | 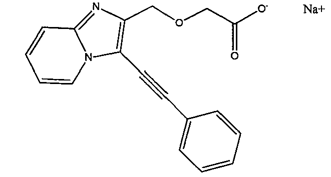 | 2-((3-(phényléthynyl)imidazo[1,2-a]pyridin-2-yl)méthoxy)acétate de sodium |
| **46** | 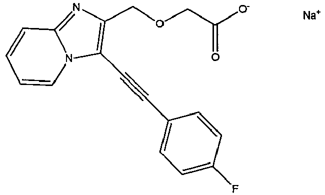 | 2-((3-((4-fluorophényl)éthynyl)imidazo[1,2-a]pyridin-2-yl)méthoxy) acétate de sodium |
| **47** | 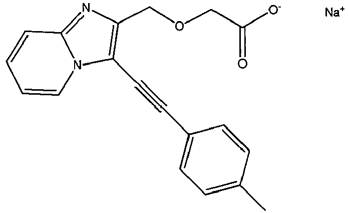 | 2-((3-(p-tolyléthynyl)imidazo[1,2-a]pyridin-2-yl)méthoxy) acétate de sodium |
| **48** | 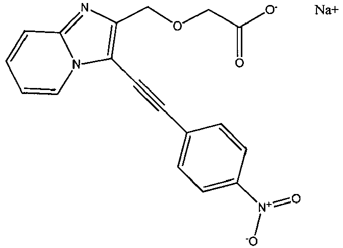 | 2-((3-((4-nitrophényl)éthynyl)imidazo[1,2-a]pyridin-2-yl)méthoxy) acétate de sodium |
| **49** | 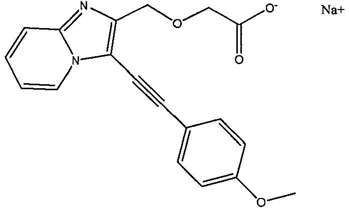 | 2-((3-((4-méthoxyphényl)éthynyl)imidazo[1,2-a]pyridin-2-yl)méthoxy) acétate de sodium |
| **50** | 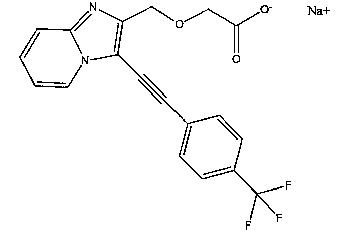 | 2-((3-((4-(trifluorométhyl)phényl)éthynyl)imid azo[1,2-a]pyridin-2-yl)méthoxy) acétate de sodium |
| **51** | 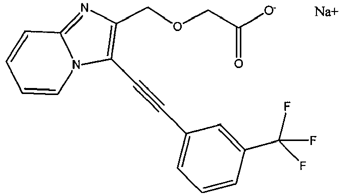 | 2-((3-((3-(trifluorométhyl)phényl)éthynyl)imid azo[1,2-a]pyridin-2-yl)méthoxy) acétate de sodium |
| **52** | 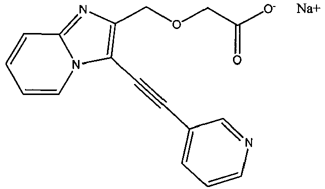 | 2-((3-(pyridin-3-yléthynyl)imidazo[1,2-a]pyridin-2-yl)méthoxy) acétate de sodium |
| **53** | 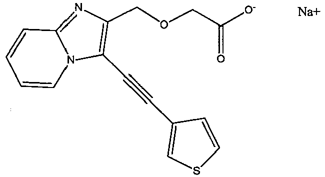 | 2-((3-(thiophén-3-yléthynyl)imidazo[1,2-a]pyridin-2-yl)méthoxy) acétate de sodium |
| **54** | 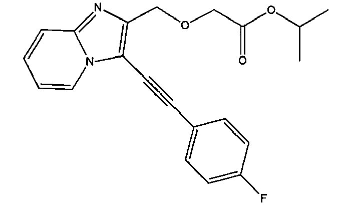 | 2-((3-((4-fluorophényl)éthynyl)imidazo[1,2-a]pyridin-2-yl)méthoxy) acétate d' isopropyle |
| **55** | 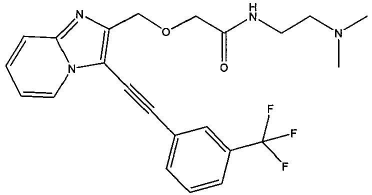 | N-(2-(diméthylamino)éthyl)-2-((3-((3-(trifluorométhyl)phényl)éthynyl)imid azo[1,2-a]pyridin-2-yl)méthoxy)acétamide |
| **56** | 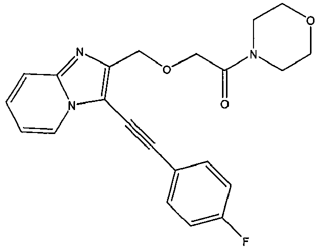 | 2-((3-((4-fluorophényl)éthynyl)imidazo[1,2-a]pyridin-2-yl)méthoxy)-1-morpholinoéthanone |
| **57** | 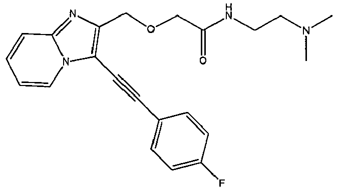 | N-(2-(diméthylamino)éthyl)-2-((3-((4-fluorophényl)éthynyl)imidazo[1,2-a]pyridin-2-yl)méthoxy) acétamide |
| **58** | 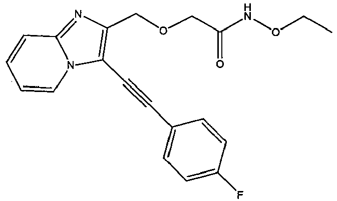 | N-éthoxy-2-((3-((4-fluorophényl)éthynyl)imidazo[1,2-a]pyridin-2-yl)méthoxy) acétamide |
| **59** | 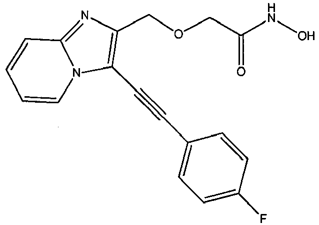 | 2-((3-((4-fluorophényl)éthynyl)imidazo[1,2-a]pyridin-2-yl)méthoxy)-N-hydroxy acétamide |
| **60** | 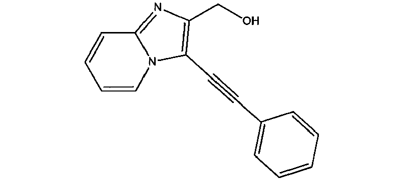 | (3-(phényléthynyl)imidazo[1,2-a]pyridin-2-yl)méthanol |
| **61** | 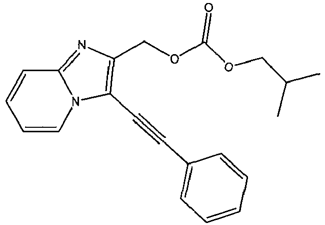 | ((3-(phényléthynyl)imidazo[1,2-a]pyridin-2-yl)méthyl) carbonate d'isobutyle |
| **62** | 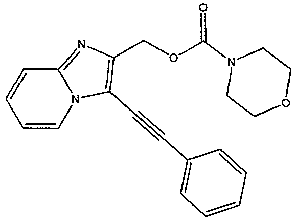 | (3-(phényléthynyl)imidazo[1,2-a]pyridin-2-yl)méthyl morpholine-4-carboxylate |
| **63** | 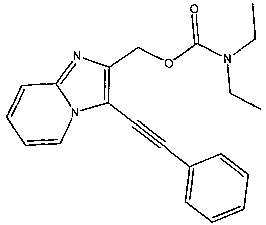 | (3-(phényléthynyl)imidazo[1,2-a]pyridin-2-yl)méthyl diéthylcarbamate |
| **64** | 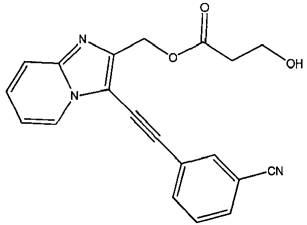 | (3-((3-cyanophényl)éthynyl)imidazo[1,2-a]pyridin-2-yl)méthyl 3-hydroxypropanoate |
| **65** | 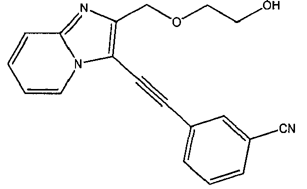 | 3-((2-((2-hydroxyéthoxy)méthyl)imidazo[1,2-a]pyridin-3-yl)éthynyl)benzonitrile |
| **66** | 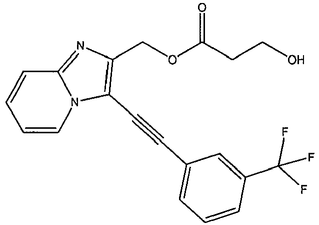 | (3-((3-(trifluorométhyl)phényl)éthynyl)imid azo[1,2-a]pyridin-2-yl)méthyl 3-hydroxypropanoate |
| **67** | 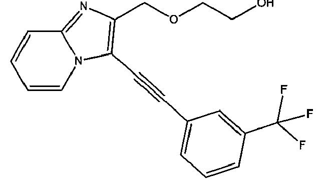 | 2-((3-((3-(trifluorométhyl)phényl)éthynyl)imid azo[1,2-a]pyridin-2-yl)méthoxy)éthanol |
| **68** | 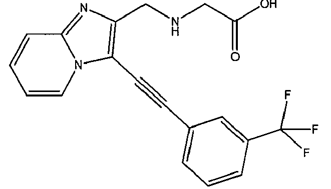 | Acide 2-(((3-((3-(trifluorométhyl)phényl)éthynyl)imid azo[1,2-a]pyridin-2-yl)méthyl)amino) acétique |
| **69** | 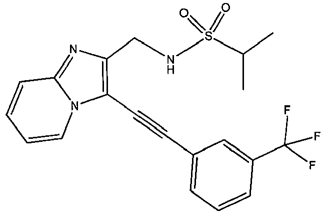 | N-((3-((3-(trifluorométhyl)phényl)éthynyl)imid azo[1,2-a]pyridin-2-yl)méthyl)propane-2-sulfonamide |
| **70** | 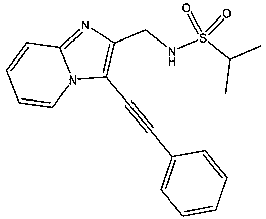 | N-((3-(phényléthynyl)imidazo[1,2-a]pyridin-2-yl)méthyl)propane-2-sulfonamide |
| **71** | 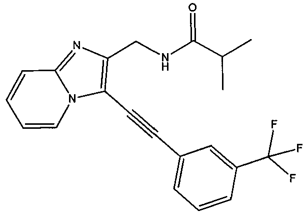 | N-((3-((3-(trifluorométhyl)phényl)éthynyl)imid azo[1,2-a]pyridin-2-yl)méthyl)isobutyramide |
| **72** | 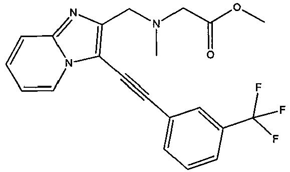 | 2-(méthyl((3-((3-(trifluorométhyl)phényl)éthynyl)imid azo[1,2-a]pyridin-2-yl)méthyl)amino) acétate de méthyle |
| **73** | 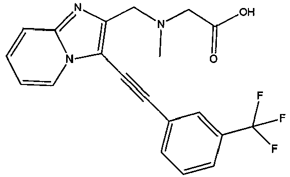 | Acide 2-(méthyl((3-((3-(trifluorométhyl)phényl)éthynyl)imid azo[1,2-a]pyridin-2-yl)méthyl)amino) acétique |
| **74** | 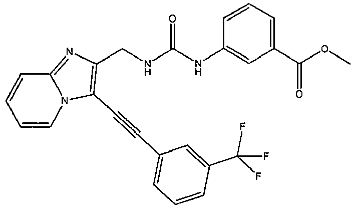 | 3-(3-((3-((3-(trifluorométhyl)phényl)éthynyl)imid azo[1,2-a]pyridin-2-yl)méthyl)ureido)benzoate de méthyle |
| **75** | 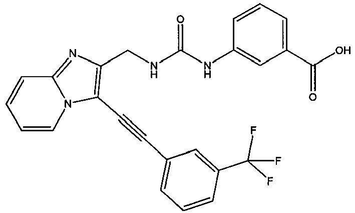 | Acide 3-(3-((3-((3-(trifluorométhyl)phényl)éthynyl)imid azo[1,2-a]pyridin-2-yl)méthyl)ureido) benzoïque |
| **76** | 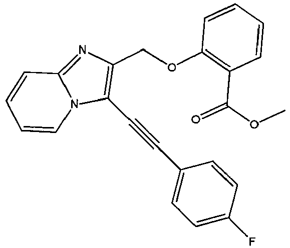 | 2-((3-((4-fluorophényl)éthynyl)imidazo[1,2-a]pyridin-2-yl)méthoxy) benzoate de méthyle |
| **77** | 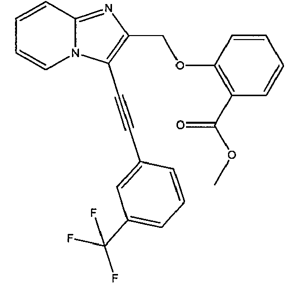 | 2-((3-((3-(trifluorométhyl)phényl)éthynyl)imid azo[1,2-a]pyridin-2-yl)méthoxy) benzoate de méthyle |
| **78** | 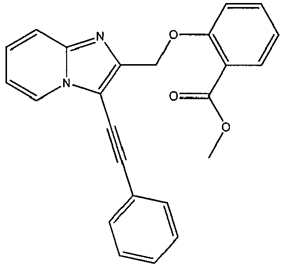 | 2-((3-(phényléthynyl)imidazo[1,2-a]pyridin-2-yl)méthoxy) benzoate de méthyle |
| **79** | 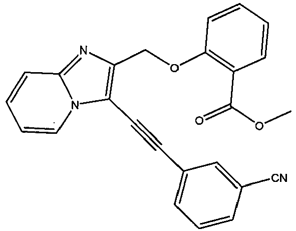 | 2-((3-((3-cyanophényl)éthynyl)imidazo[1,2-a]pyridin-2-yl)méthoxy) benzoate de méthyle |
| **80** | 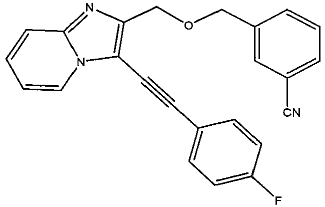 | 3-(((3-((4-fluorophényl)éthynyl)imidazo[1,2-a]pyridin-2-yl)méthoxy)méthyl)benzonitrile |
| **81** | 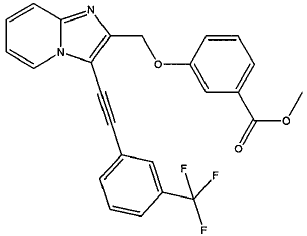 | 3-((3-((3-(trifluorométhyl)phényl)éthynyl)imid azo[1,2-a]pyridin-2-yl)méthoxy) benzoate de méthyle |
| **82** | 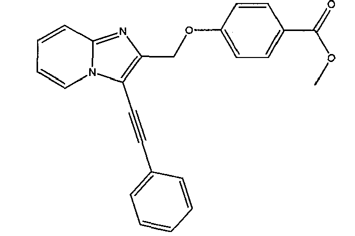 | 4-((3-(phényléthynyl)imidazo[1,2-a]pyridin-2-yl)méthoxy) benzoate de méthyle |
| **83** | 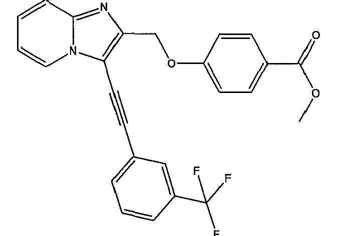 | 4-((3-((3-(trifluorométhyl)phényl)éthynyl)imid azo[1,2-a]pyridin-2-yl)méthoxy) benzoate de méthyle |
| **84** | 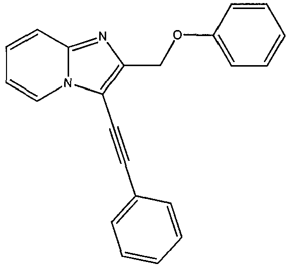 | 2-(phénoxyméthyl)-3-(phényléthynyl)imidazo[1,2-a]pyridine |
| **85** | 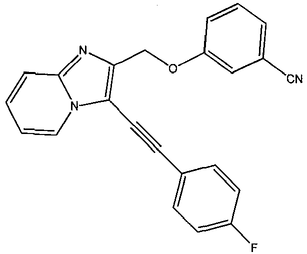 | 3-((3-((4-fluorophényl)éthynyl)imidazo[1,2-a]pyridin-2-yl)méthoxy)benzonitrile |
| **86** | 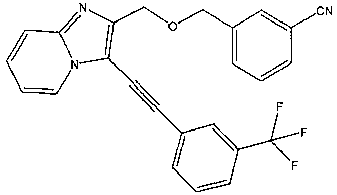 | 3-(((3-((3-(trifluorométhyl)phényl)éthynyl)imid azo[1,2-a]pyridin-2-yl)méthoxy)méthyl)benzonitrile |
| **87** | 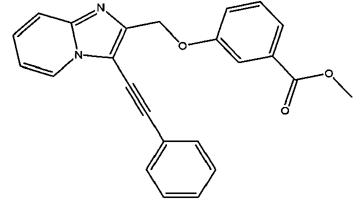 | 3-((3-(phényléthynyl)imidazo[1,2-a]pyridin-2-yl)méthoxy) benzoate de méthyle |
| **88** | 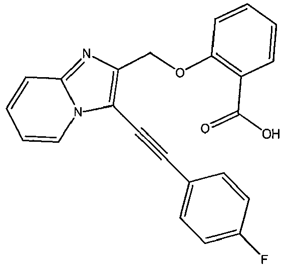 | Acide 2-((3-((4-fluorophényl)éthynyl)imidazo[1,2-a]pyridin-2-yl)méthoxy) benzoïque |
| **89** | 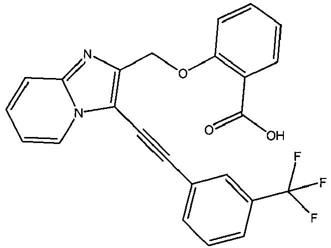 | Acide 2-((3-((3-(trifluorométhyl)phényl)éthynyl)imid azo[1,2-a]pyridin-2-yl)méthoxy) benzoïque |
| **90** | 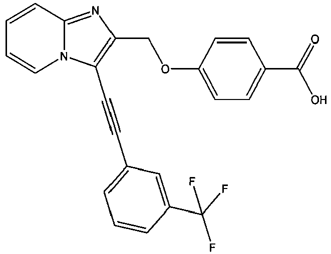 | Acide 4-((3-((3-(trifluorométhyl)phényl)éthynyl)imid azo[1,2-a]pyridin-2-yl)méthoxy) benzoïque |
| **91** | 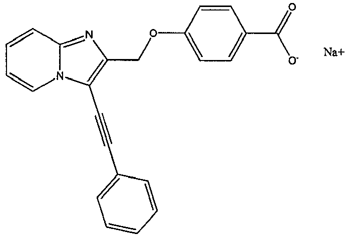 | 4-((3-(phényléthynyl)imidazo[1,2-a]pyridin-2-yl)méthoxy)benzoate de sodium |
| **92** | 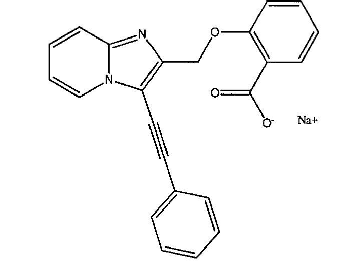 | 2-((3-(phényléthynyl)imidazo[1,2-a]pyridin-2-yl)méthoxy)benzoate de sodium |
| **93** | 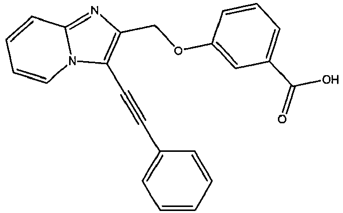 | Acide 3-((3-(phényléthynyl)imidazo[1,2-a]pyridin-2-yl)méthoxy) benzoïque |
| **94** | 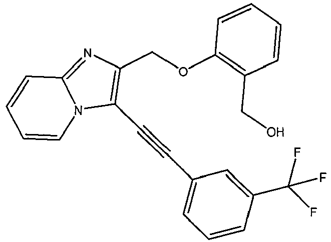 | (2-((3-((3-(trifluorométhyl)phényl)éthynyl)imid azo[1,2-a]pyridin-2-yl)méthoxy)phényl)méthanol |
| **95** | 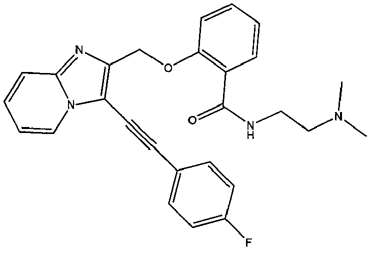 | N-(2-(diméthylamino)éthyl)-2-((3-((4-fluorophényl)éthynyl)imidazo[1,2-a]pyridin-2-yl)méthoxy)benzamide |
| **96** | 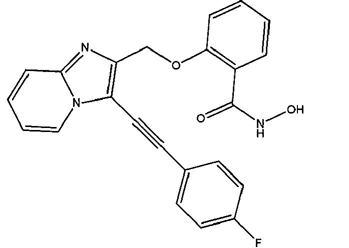 | 2-((3-((4-fluorophényl)éthynyl)imidazo[1,2-a]pyridin-2-yl)méthoxy)-N-hydroxybenzamide |
| **97** | 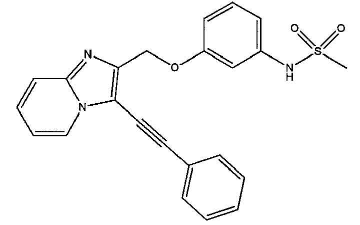 | N-(3-((3-(phényléthynyl)imidazo[1,2-a]pyridin-2-yl)méthoxy)phényl)méthanesulfona mide |
| **98** | 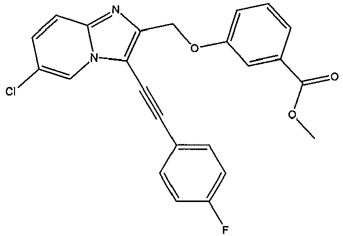 | 3-((6-chloro-3-((4-fluorophényl)éthynyl)imidazo[ 1,2-a]pyridin-2-yl)méthoxy)benzoate de méthyle |
| **99** | 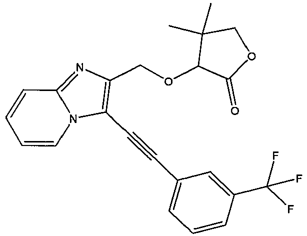 | 4,4-diméthyl-3-[[3-[2-[3-(trifluorométhyl)phényl]éthyny l]imidazo[1,2-a]pyridin-2-yl]méthoxy]tetrahydrofuran-2-one |
| **100** | 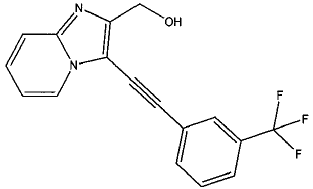 | [3-[2-[3-(trifluorométhyl)phényl]éthyny l]imidazo[1,2-a]pyridin-2-yl]méthanol |
| **101** | 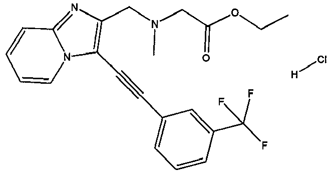 | chlorhydrate de 2-[méthyl-[[3-[2-[3-(trifluorométhyl)phényl]éthyny I]imidazo[1,2-a]pyridin-2-yl]méthyl]amino]acétate d'éthyle |
| **102** | 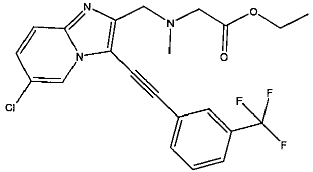 | 2-[[6-chloro-3-[2-[3-(trifluorométhyl)phényl]éthyny l]imidazo[1,2-a]pyridin-2-yl]méthyl-méthyl-amino]acétate d'éthyle |
| **103** | 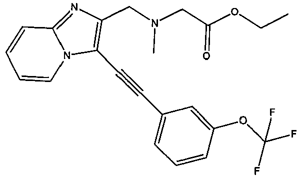 | 2-(méthyl((3-((3-(trifluorométhoxy)phényl)éthy nyl)imidazo[1,2-a]pyridin-2-yl)méthyl)amino)acétate d'éthyle |
| **104** | 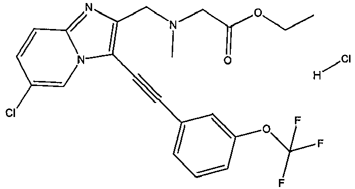 | Chlorhydrate de 2-(((6-chloro-3-((3-(trifluorométhoxy)phényl)éthy nyl)imidazo[1,2-a]pyridin-2-yl)méthyl)(méthyl)amino)acét ate d'éthyle |
| **105** | 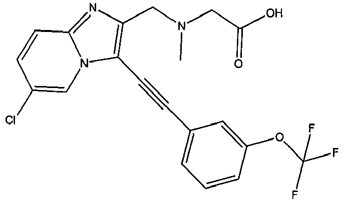 | Acide 2-(((6-chloro-3-((3-(trifluorométhoxy)phényl)éthy nyl)imidazo[1,2-a]pyridin-2-yl)méthyl)(méthyl)amino)acéti que |
| **106** | 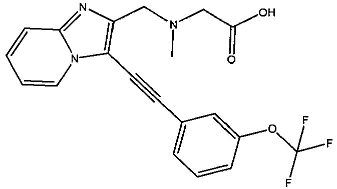 | Acide 2-(méthyl((3-((3-(trifluorométhoxy)phényl)éthy nyl)imidazo[1,2-a]pyridin-2-yl)méthyl)amino)acétique |
| **107** | 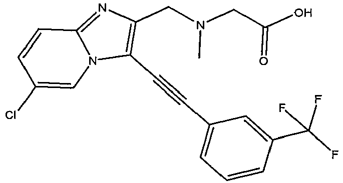 | Acide 2-(((6-chloro-3-((3-(trifluorométhyl)phényl)éthyny I)imidazo[1,2-a]pyridin-2-yl)méthyl)(méthyl)amino)acéti que |
| **108** | 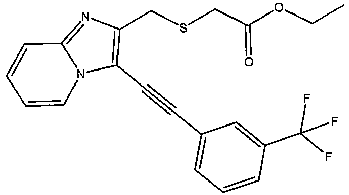 | 2-[[3-[2-[3-(trifluorométhyl)phényl]éthyny l]imidazo[1,2-a]pyridin-2-yl]méthylsulfanyl]acétate d'éthyle |
| **109** | 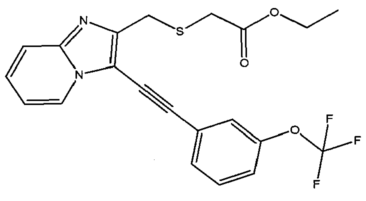 | 2-[[3-[2-[3-(trifluorométhoxy)phényl]éthy nyl]imidazo[1,2-a]pyridin-2-yl]méthylsulfanyl]acétate d'éthyle |
| **110** | 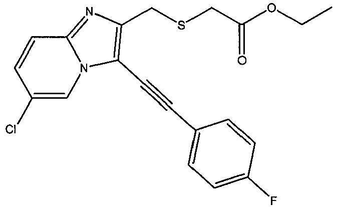 | 2-[[6-chloro-3-[2-[4-(fluoro)phényl]éthynyl]imidaz o[1,2-a]pyridin-2-yl]méthylsulfanyl]acétate d'éthyle |
| **111** | 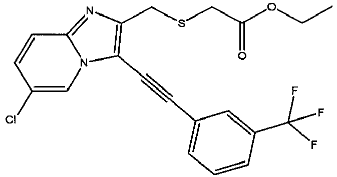 | 2-[[6-chloro-3-[2-[3-(trifluorométhyl)phényl]éthyny I]imidazo[1,2-a]pyridin-2-yl]méthylsulfanyl]acétate d'éthyle |
| **112** | 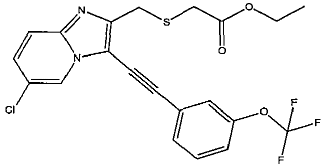 | 2-[[6-chloro-3-[2-[3-(trifluorométhoxy)phényl]éthy nyl]imidazo[1,2-a]pyridin-2-yl]méthylsulfanyl]acétate d'éthyle |
| **113** | 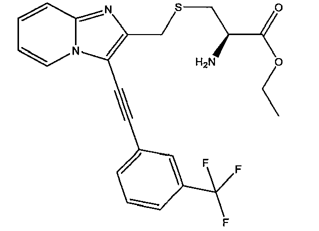 | (2R)-2-amino-3-[[3-[2-[3-(trifluorométhyl)phényl]éthyny l]imidazo[1,2-a]pyridin-2-yl]méthylsulfanyl]propanoate d'éthyle |
| **114** | 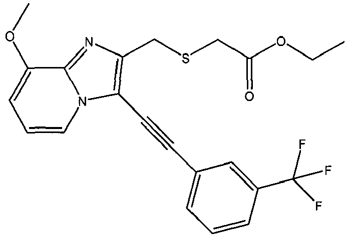 | 2-[[8-méthoxy-3-[2-[3-(trifluorométhyl)phényl]éthyny l]imidazo[1,2-a]pyridin-2-yl]méthylsulfanyl]acétate d'éthyle |
| **115** | 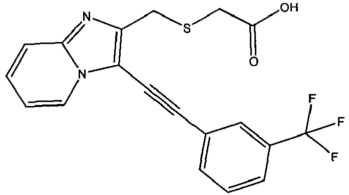 | Acide 2-[[3-[2-[3-(trifluorométhyl)phényl]éthyny l]imidazo[1,2-a]pyridin-2-yl]méthylsulfanyl]acétique |
| **116** | 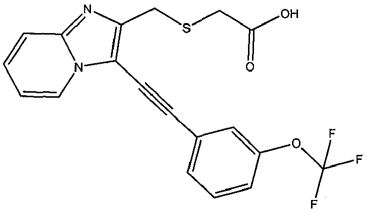 | Acide 2-[[3-[2-[3-(trifluorométhoxy)phényl]éthy nyl]imidazo[1,2-a]pyridin-2-yl]méthylsulfanyl]acétique |
| **117** | 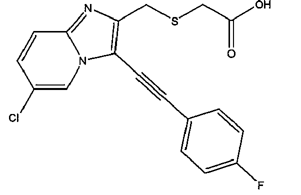 | Acide 2-[[6-chloro-3-[2-(4-fluorophényl)éthynyl]imidazo[ 1,2-a]pyridin-2-yl]méthylsulfanyl]acétique |
| **118** | 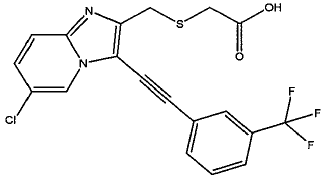 | Acide 2-[[6-chloro-3-[2-[3-(trifluorométhyl)phényl]éthyny l]imidazo[1,2-a]pyridin-2-yl]méthylsulfanyl]acétique |
| **119** | 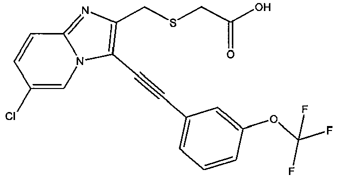 | Acide 2-[[6-chloro-3-[2-[3-(trifluorométhoxy)phényl]éthy nyl]imidazo[1,2-a]pyridin-2-yl]méthylsulfanyl]acétique |
| **120** | 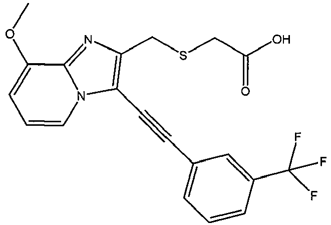 | Acide 2-[[8-méthoxy-3-[2-[3-(trifluorométhyl)phényl]éthyny l]imidazo[1,2-a]pyridin-2-yl]méthylsulfanyl]acétique |
| **121** | 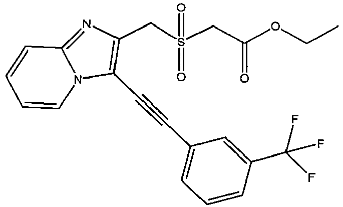 | 2-[[3-[2-[3-(trifluorométhyl)phényl]éthyny l]imidazo[1,2-a]pyridin-2-yl]méthylsulfonyl]acétate d'éthyle |
| **122** | 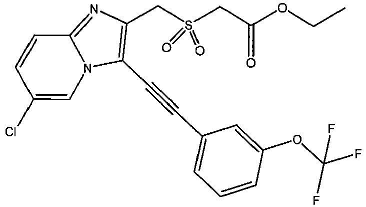 | 2-[[6-chloro-3-[2-[3-(trifluorométhoxy)phényl]éthy nyl]imidazo[1,2-a]pyridin-2-yl]méthylsulfonyl]acétate d'éthyle |
| **123** | 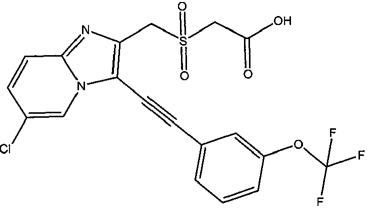 | Acide 2-[[6-chloro-3-[2-[3-(trifluorométhoxy)phényl]éthy nyl]imidazo[1,2-a]pyridin-2-yl]méthylsulfonyl]acétique |
| **124** | 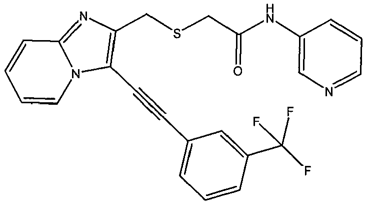 | N-(3-pyridyl)-2-[[3-[2-[3-(trifluorométhyl)phényl]éthyny l]imidazo[1,2-a]pyridin-2-yl]méthylsulfanyl]acétamide |
| **125** | 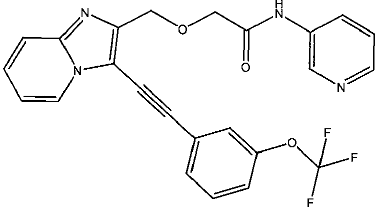 | N-(3-pyridyl)-2-[[3-[2-[3-(trifluorométhoxy)phényl]éthy nyl]imidazo[1,2-a]pyridin-2-yl]méthoxy]acétamide |
| **126** | 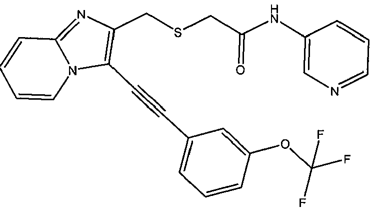 | N-(3-pyridyl)-2-[[3-[2-[3-(trifluorométhoxy)phényl]éthy nyl]imidazo[1,2-a]pyridin-2-yl]méthylsulfanyl]acétamide |
| **127** | 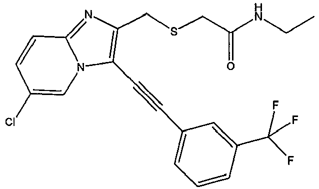 | 2-[[6-chloro-3-[2-[3-(trifluorométhyl)phényl]éthyny l]imidazo[1,2-a]pyridin-2-yl]méthylsulfanyl]-N-éthyl-acétamide |
| **128** | 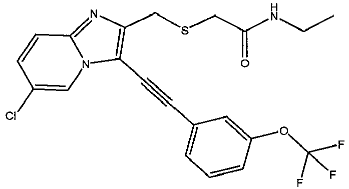 | 2-[[6-chloro-3-[2-[3-(trifluorométhoxy)phényl]éthy nyl]imidazo[1,2-a]pyridin-2-yl]méthylsulfanyl]-N-éthyl-acétamide |
| **129** | 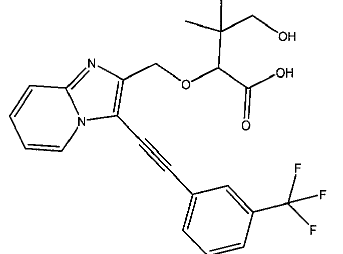 | Acide 4-hydroxy-3,3-diméthyl-2-[[3-[2-[3-(trifluorométhyl)phényl]éthyny l]imidazo[1,2-a]pyridin-2-y)]méthoxy]butanoïque |
| **130** | 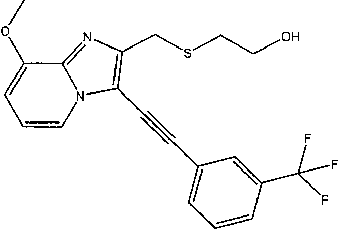 | 2-[[8-méthoxy-3-[2-[3-(trifluorométhyl)phényl]éthyny I]imidazo[1,2-a]pyridin-2-yl]méthylsulfanyl]éthanol |
| **131** | 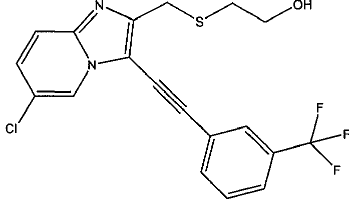 | 2-[[6-chloro-3-[2-[3-(trifluorométhyl)phényl]éthyny l]imidazo[1,2-a]pyridin-2-yl]méthylsulfanyl]éthanol |
| **132** | 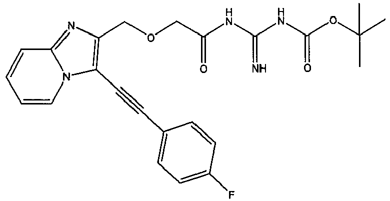 | N-[N-[2-[[3-[2-(4-fluorophényl)éthynyl]imidazo[ 1,2-a]pyridin-2-yl]méthoxy]acétyl]carbamimid oyl]carbamate de tert-butyle |
| **133** | 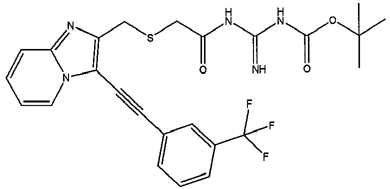 | N-[N-[2-[[3-[2-[3-(trifluorométhyl)phényl]éthyny I]imidazo[1,2-a]pyridin-2-yl]méthylsulfanyl]acétyl]carba mimidoyl]carbamate de tert-butyle |
| **134** | 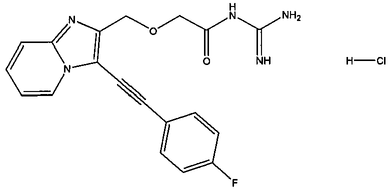 | Chlorhydrate de N-carbamimidoyl-2-[[3-[2-(4-fluorophényl)éthynyl]imidazo[ 1,2-a]pyridin-2-yl]méthoxy]acétamide |
| **135** | 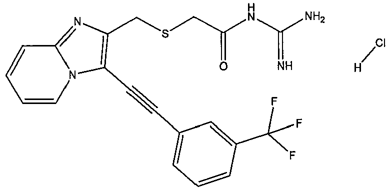 | Chlorhydrate de N-carbamimidoyl-2-[[3-[2-[3-(trifluorométhyl)phényl]éthyny I]imidazo[1,2-a]pyridin-2-yl]méthylsulfanyl]acétamide |
| **136** | 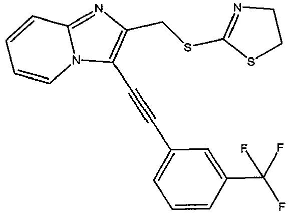 | 2-((3-((3-(trifluorométhyl)phényl)éthyny l)imidazo[1,2-a]pyridin-2-yl)méthylthio)-4,5-dihydrothiazole |
| **137** | 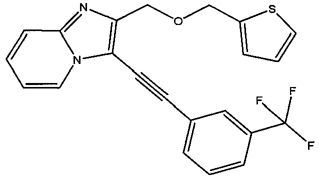 | 2-((thiophén-2-ylméthoxy)méthyl)-3-((3-(trifluorométhyl)phényl)éthyny l)imidazo[1,2-a]pyridine |
| **138** | 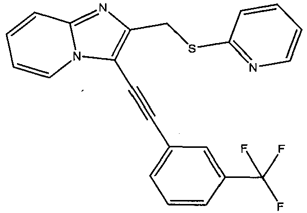 | 2-((pyridin-2-ylthio)méthyl)-3-((3-(trifluorométhyl)phényl)éthyny l)imidazo[1,2-a]pyridine |

### Exemple 1 : Préparation du dérivé N°1 : 2-((3-(phényléthynyl) imidazo[1,2-a]pyridin-2-yl)méthoxy)acétate d'éthyle

### Etape 1 : Préparation de la 2-(chlorométhyl)imidazo[1,2-a] pyridine

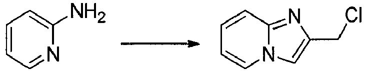

7,09 g (54,1 mmol) de 1,3-dichloroacétone ont été solubilisés dans 13 ml de DME, à cette solution ont été additionnés sous agitation magnétique, 5 g (52,1 mmol) de 2-aminopyridine. Le mélange a été agité à t.a. pendant 16 h. Le solide formé a été isolé par filtration et lavé avec 30 ml d'éther diisopropylique. Ce solide jaune a été mis en suspension dans 125 ml d'éthanol absolu et le mélange a été agité à reflux pendant 2 h. Le mélange a été concentré sous vide puis le résidu obtenu a été repris dans 150 ml d'un mélange composé d'eau et de glace. Le pH de la phase aqueuse a été basifié jusqu'à pH 8-9 par addition d'une solution aqueuse saturée en NaHCO₃. La phase aqueuse a été extraite avec 3 x 100 ml de Dichlorométhane. Les phases organiques combinées ont été lavées avec 200 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : gradient d'heptane / acétate d'éthyle, de 50% à 40% d'heptane, v/v). 4,77 g (rendement = 55%) de 2-(chlorométhyl)imidazo[1,2-a]pyridine ont été obtenus sous forme d'un solide blanc. LC-MS: m/z = 167 (MH⁺) ; pureté UV à 254 nm = 99%. RMN ¹H (300 MHz, DMSO) δ 8.52 (d, J = 6.8 Hz, 1H), 7.99 (s, 1H), 7.51 (d, J = 9.1 Hz, 1H), 7.32 - 7.16 (m, 1H), 6.89 (t, J = 6.8 Hz, 1H), 4.84 (s, 2H)

### Etape 2 : Préparation de l'acide 2-(imidazo[1,2-a]pyridin-2-ylméthoxy)acétique

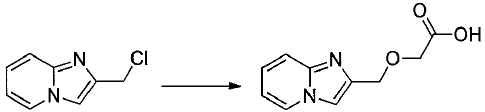

4,24 g (25,4 mmol) de 2-(chlorométhyl)imidazo[1,2-a]pyridine ont été dissous dans 150 ml de tétrahydrofurane sous agitation magnétique puis 4,2 g (28 mmol) d'iodure de sodium ont été ajoutés. Le mélange a été agité à t.a. pendant 3 h. Dans un autre ballon, 9,84 ml (101,8 mmol) de 2-hydroxyacétate d'éthyle ont été solubilisés dans 100 ml de tétrahydrofurane sous agitation magnétique, puis le mélange a été refroidi à 0°C et 4,072 g (101,8 mmol) d'hydrure de sodium ont été ajoutés par portions. Le mélange a été agité 15 min à 0°c puis la solution fraîchement préparée de 2-(iodométhyl)imidazo[1,2-a]pyridine dans le tétrahydrofurane a été ajoutée goutte à goutte en 15 min. Le mélange a été agité à t.a. pendant 24 h avant d'être filtré sur silice (éluant : tétrahydrofurane 100%). Le filtrat a été concentré sous vide et directement purifié par chromatographie flash sur une cartouche de gel de silice (éluant : gradient de Dichlorométhane / méthanol, de 100% à 70% de Dichlorométhane, v/v ; puis lavage de la cartouche avec 100% de méthanol). 3,62 g (rendement = 69%) d'acide 2-(imidazo[1,2-a]pyridin-2-ylméthoxy)acétique ont été obtenus sous forme d'un solide beige. LC-MS: m/z = 207 (MH⁺) ; pureté UV à 254 nm = 98%.

### Etape 3 : Préparation du 2-(imidazo[1,2-a]pyridin-2-ylméthoxy)acétate d'éthyle

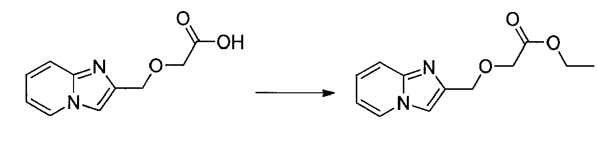

3,62 g (15,58 mmol) d'acide 2-(imidazo[1,2-a]pyridin-2-ylméthoxy) acétique ont été solubilisés sous agitation magnétique dans 100 ml d'éthanol et 0,094 ml (1,758mmol) d'acide sulfurique ont été ajoutés. Le mélange a été agité à reflux pendant 5 j. Le milieu réactionnel a été concentré sous vide et directement purifié par chromatographie flash sur une cartouche de gel de silice (éluant : acétate d'éthyle 100% puis Dichlorométhane / méthanol, 9/1, v/v). Le milieu réactionnel a été lavé avec 2 x 10 ml d'eau, puis extrait avec 2 x 30 ml d'acétate d'éthyle. Les phases organiques combinées ont été lavées avec 30 ml d'une solution aqueuse saturée en NaCl, séchées sur Na₂SO₄ qui a ensuite été éliminé par filtration. Le filtrat obtenu a été concentré sous vide. 0,162 mg (rendement = 91%) de 2-((3-((4-fluorophényl)éthynyl)imidazo[1,2-a] pyridin-2-yl)méthoxy)acétate d'isopropyle ont été obtenus sous forme d'une huile marron. MS: m/z = 235 (MH⁺). RMN ¹H (300 MHz, DMSO) δ 8.52 (d, J = 7.9 Hz, 1H), 7.91 (s, 1H), 7.50 (d, J = 9.1 Hz, 1H), 7.30 - 7.13 (m, 1H), 6.87 (t, J = 6.7 Hz, 1H), 4.65 (s, 2H), 4.20 (s, 2H), 4.12 (q, J = 7.1 Hz, 2H), 1.19 (t, J = 7.2 Hz, 3H).

### Etape 4 : Préparation du 2-((3-iodoimidazo[1,2-a]pyridin-2-yl)méthoxy)acétate d'éthyle

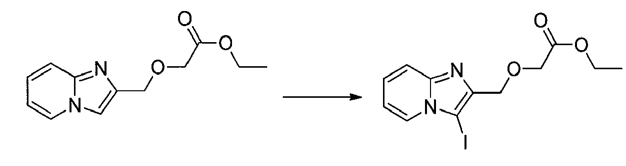

0,45 g (1,921 mmol) de 2-(imidazo[1,2-a]pyridin-2-ylméthoxy)acétate d'éthyle ont été solubilisés dans 6 ml d'acétonitrile sous agitation magnétique, à cette solution ont été ajoutés 0,475 g (2,113 mmol) de N-iodosuccinimide. Le mélange a été agité à t.a. pendant 16 h. Un solide a éliminé par filtration et le filtrat obtenu a été concentré sous vide. Le résidu a été trituré avec 10 ml d'éther diisopropylique et un précipité a été isolé par filtration. 0,465 g (rendement = 67%) de 2-((3-iodoimidazo[1,2-a]pyridin-2-yl)méthoxy)acétate d'éthyle ont été obtenus sous forme d'un solide jaune. LC-MS: m/z = 361 (MH⁺) ; pureté UV à 254 nm = 75%. RMN ¹H (300 MHz, DMSO) δ 8.34 (d, J = 6.9 Hz, 1H), 7.59 (d, J = 8.1 Hz, 1H), 7.47 - 7.28 (m, 1H), 7.07 (d, J = 5.7 Hz, 1H), 4.67 (s, 2H), 4.14 (t, J = 10.7 Hz, 4H), 1.22 (d, J = 1.9 Hz, 3H)

### Etape 5 : Préparation du 2-((3-(phényléthynyl)imidazo[1,2-a]pyridin-2-yl)méthoxy)acétate d'éthyle (dérivé numéro 1)

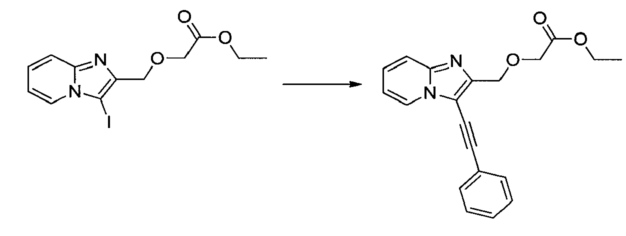

Dans un ballon placé sous un flux d'argon, 0,1 g (0,278 mmol) de 2-((3-iodoimidazo[1,2-a]pyridin-2-yl)méthoxy)acétate d'éthyle ont été solubilisés dans 0,3 ml de diméthylformamide sous agitation magnétique. A cette solution, ont été additionnés : 0,005 g (0,028 mmol) d'iodure de cuivre, 0,037 ml (0,333 mmol) de phénylacétylène et 0,3 ml (2,152 mmol) de triéthylamine. Le milieu réactionnel a été dégazé à l'argon pendant 10 min avant d'ajouter 0,025 g (0,028 mmol) de Pd₂(dba)₃. Le mélange a été agité à t.a. pendant 16 h avant d'être filtré sur célite. La célite a été lavée avec 20 ml d'acétate d'éthyle, puis le filtrat a été lavé avec 2 x 50 ml d'eau. Après séparation, la phase aqueuse a été extraite avec 30 ml d'acétate d'éthyle, les phases organiques combinées ont été lavées avec 30 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : heptane / acétate d'éthyle, 1/1, v/v). 0,038 g (rendement = 41%) de 2-((3-(phényléthynyl)imidazo[1,2-a] pyridin-2-yl)méthoxy) acétate d'éthyle ont été obtenus sous forme d'un solide jaune clair. LC-MS: m/z = 335 (MH⁺) ; pureté UV à 254 nm = 99%. RMN ¹H (300 MHz, DMSO) 5 8.63 (d, J = 5.6 Hz, 1H), 7.68 (d, J = 8.0 Hz, 3H), 7.54 - 7.39 (m, 4H), 7.14 (t, J = 6.8 Hz, 1H), 4.79 (s, 2H), 4.24 (s, 2H), 4.09 (q, J = 7.1 Hz, 2H), 1.15 (t, J = 7.1 Hz, 3H).

Les dérivés numéro **2** à **26, 98** et **99** ont été obtenus à partir du 2-((3-iodoimidazo[1,2-a]pyridin-2-yl)méthoxy)acétate d'éthyle selon le même protocole.

| **No** | **Poids moléculaire** | **Apparence** | **Pureté LCMS** | **Spectrométri e de masse** | | **RMN ¹H (300 MHz, DMSO) δ** |
|---|---|---|---|---|---|---|
| | **g/mol** | | **UV à 254 nm** | **MH⁺** | **M-H⁺** | |
| **2** | 348,40 | huile jaune | >99 | 349 | | 8.60 (d, *J* = 6.7 Hz, 1H), 7.67 (d, *J* = 9.0 Hz, 1H), 7.56 (d, *J* = 8.1 Hz, 2H), 7.49 - 7.38 (m, 1H), 7.28 (d, *J* = 7.9 Hz, 2H), 7.12 (t, *J* = 6.8 Hz, 1H), 4.77 (s, 2H), 4.23 (s, 2H), 4.09 (q, *J* = 7.1 Hz, 2H), 2.35 (s, 3H), 1.14 (t, *J* = 7.1 Hz, 3H). |
| **3** | 352,36 | solide gris clair | >99 | 353 | | 8.63 (d, *J* = 6.8 Hz, 1H), 7.80 - 7.62 (m, 3H), 7.50 - 7.39 (m, 1H), 7.33 (t, *J =* 8.9 Hz, 2H), 7.12 (t, *J =* 7.3 Hz, 1H), 4.77 (s, 2H), 4.22 (s, 2H), 4.08 *(q, J =* 7.1 Hz, 2H), 1.13 (t, *J* = 7.1 Hz, 3H). |
| **4** | 364,39 | huile marron | >99 | 365 | | 8.59 (d, *J =* 6.7 Hz, 1H), 7.77 - 7.56 (m, 3H), 7.50 - 7.36 (m, 1H), 7.22 - 6.97 (m, 3H), 4.77 (s, 2H), 4.23 (s, 2H), 4.18 - 4.02 (m, 2H), 1.15 (t, *J* = 7.1 Hz, 3H). |
| **5** | 379,37 | solide jaune | >99 | 380 | | 8.77 (d, *J* = 6.7 Hz, 1H), 8.33 (d, *J* = 9.0 Hz, 2H), 7.95 (d, *J* = 9.0 Hz, 2H), 7.72 (d, *J =* 9.0 Hz, 1H), 7.56 - 7.44 (m, 1H), 7.18 (t, *J* = 6.8 Hz, 1H), 4.82 (s, 2H), 4.27 (s, 2H), 4.11 (q, *J =* 7.1 Hz, 2H), 1.16 (t, *J =* 7.1 Hz, 3H). |
| **6** | 402,37 | solide marron | >99 | 403 | | 8.73 (d, *J* = 6.7 Hz, 1H), 7.89 (q, *J* = 8.4 Hz, 4H), 7.72 (d, *J* = 9.0 Hz, 1H), 7.54 - 7.42 (m, 1H), 7.18 (t, *J =* 6.3 Hz, 1H), 4.82 (s, 2H), 4.27 (s, 2H), 4.12 (q, *J* = 7.1 Hz, 2H), 1.16 (t, *J =* 7.1 Hz, 3H). |
| **7** | 402,37 | solide marron | 98,8 | 403 | | 8.76 (d, *J* = 6.7 Hz, 1H), 8.11 (s, 1H), 7.97 (d, *J* = 7.6 Hz, 1H), 7.79 (d, *J* = 7.9 Hz, 1H), 7.75 - 7.64 (m, 2H), 7.51-7.41 (m, 1H), 7.15 (t, *J* = 6.3 Hz, 1H), 4.80 (s, 2H), 4.25 (s, 2H), 4.09 (q, *J* = 7.1 Hz, 2H), 1.14 (t, *J* = 7.1 Hz, 3H). |
| **8** | 335,36 | solide jaune pâle | >99 | 336 | | 8.88 (d, *J* = 1.4 Hz, 1H), 8.69 (d, *J =* 6.8 Hz, 1H), 8.60 (dd, *J* = 4.8, 1.5 Hz, 1H), 8.09 (dt, *J* = 7.9, 1.9 Hz, 1H), 7.70 (d, *J* = 9.0 Hz, 1H), 7.56 - 7.41 (m, 2H), 7.15 (td, *J* = 6.8, 1.0 Hz, 1H), 4.79 (s, 2H), 4.25 (s, 2H), 4.06 (s, 2H), 1.14 (t, *J =* 7.1 Hz, 3H). |
| **9** | 340,40 | solide marron | >99 | 341 | | 8.57 (d, *J* = 6.7 Hz, 1H), 8.02 (dd, *J* = 2.9, 1.1 Hz, 1H), 7.78 - 7.63 (m, 2H), 7.50 - 7.34 (m, 2H), 7.13 (t, *J* = 6.8 Hz, 1H), 4.76 (s, 2H), 4.23 (s, 2H), 4.10 (q, *J* = 7.1 Hz, 2H), 1.16 (t, *J* = 7.1 Hz, 3H). |
| **10** | 370,35 | solide | >99 | 371 | | 8.68 (d, *J* = 6.8 Hz, 1H), 7.96 - 7.76 (m, 1H), 7.69 (d, *J* = 9.0 Hz, 1H), 7.63 - 7.52 (m, 2H), 7.52 - 7.39 (m, 1H), 7.14 (td, *J* = 6.8, 1.1 Hz, 1H), 4.78 (s, 2H), 4.24 (s, 2H), 4.10 (q, *J* = 7.1 Hz, 2H), 1.15 (t, *J* = 7.1 Hz, 3H). |
| **11** | 436,36 | solide | 97,3 | 437 | | 8.71 (d, *J* = 6.7 Hz, 1H), 7.93 (dd, J = 11.1, 1.8 Hz, 1H), 7.79 - 7.54 (m, 3H), 7.54-7.36 (m, 1H), 7.16 (td, *J* = 6.8, 1.1 Hz, 1H), 4.79 (s, 2H), 4.25 (s, 2H), 4.10 (q, *J* = 7.1 Hz, 2H), 1.15 (t, *J* = 7.1 Hz, 3H). |
| **12** | 364,40 | huile marron | >99 | 365 | | 8.58 (d, *J* = 6.7 Hz, 1H), 7.62 (d, *J* = 9.0 Hz, 1H)+, 7.45-7.26 (m, 2H), 7.18 (t, J = 4.2 Hz, 2H), 7.08 (t, *J* = 6.6 Hz, 1H), 7.00 - 6.90 (m, 1H), 4.72 (s, 2H), 4.18 (s, 2H), 4.04 (q, *J* = 7.1 Hz, 2H), 3.76 (s, 3H), 1.09 (t, *J* = 7.1 Hz, 3H). |
| **13** | 350,37 | solide marron | >99 | 351 | 349 | 9.75 (s, 1H), 8.58 (d, *J* = 6.8 Hz, 1H), 7.68 (d, *J* = 9.0 Hz, 1H), 7.53 - 7.35 (m, 1H), 7.26 (t, *J* = 7.9 Hz, 1H), 7.12 (ddd, *J* = 8.9, 7.3, 1.2 Hz, 2H), 7.05 - 6.98 (m, 1H), 6.85 (ddd, *J =* 8.2, 2.4, 0.9 Hz, 1H), 4.77 (s, 2H), 4.23 (s, 2H), 4.10 (q, *J* = 7.1 Hz, 2H), 1.15 (t, *J* = 7.1 Hz, 3H). |
| **14** | 359,38 | solide | 98,9 | 360 | | 8.68 (d, *J* = 6.7 Hz, 1H), 8.19 (t, *J* = 1.4 Hz, 1H), 7.97-7.89 (m, 1H), 7.89 - 7.78 (m, 1H), 7.63 (dd, *J* = 13.3, 5.4 Hz, 2H), 7.48 - 7.35 (m, 1H), 7.08 (dd, *J* = 16.4, 9.7 Hz, 1H), 4.74 (s, 2H), 4.20 (s, 2H), 4.06 (p, *J* = 7.0 Hz, 2H), 1.16 - 1.07 (m, 3H). |
| **15** | 373,41 | huile marron | 97,7 | 374 | | 8.64 (d, *J* = 6.5 Hz, 1H), 7.70 (d, *J* = 8.3 Hz, 3H), 7.45 (d, *J* = 8.4 Hz, 3H), 7.14 (t, *J* = 6.8 Hz, 1H), 4.78 (s, 2H), 4.24 (s, 2H), 4.09 (dd, *J* = 13.4, 6.3 Hz, 4H), 1.15 (t, *J* = 7.1 Hz, 3H). |
| **16** | 378,38 | solide beige | 93,8 | 379 | 377 | 13.28 (s, 1H), 8.72 (d, *J* = 6.8 Hz, 1H), 8.21 (d, *J* = 1.4 Hz, 1H), 8.07 - 7.85 (m, 2H), 7.78 - 7.54 (m, 2H), 7.54 - 7.33 (m, 1H), 7.15 (td, J= 6.8, 1.1 Hz, 1H), 4.80 (s, 2H), 4.26 (d, *J* = 8.7 Hz, 2H), 4.11 (q, *J* = 7.1 Hz, 2H), 1.15 (t, *J* = 7.1 Hz, 3H). |
| **17** | 418,37 | solide beige | 96 | 419 | - | 8.65 (d, *J* = 6.7 Hz, 1H), 7.87 - 7.74 (m, 2H), 7.68 (d, *J* = 9.0 Hz, 1H), 7.55 - 7.42 (m, 3H), 7.14 (td, *J* = 6.8, 1.0 Hz, 1H), 4.78 (s, 2H), 4.23 (s, 2H), 4.08 (q, *J* = 7.1 Hz, 2H), 1.13 (t, *J* = 7.1 Hz, 3H). |
| **18** | 418,37 | huile jaune | >99 | 419 | | 8.71 (dd, *J* = 5.8, 1.0 Hz, 1H), 7.80 - 7.66 (m, 3H), 7.60 (t, *J* = 8.0 Hz, 1H), 7.54 - 7.39 (m, 2H), 7.15 (td, *J* = 6.8, 1.1 Hz, 1H), 4.79 (s, 2H), 4.24 (s, 2H), 4.09 (q, *J* = 7.1 Hz, 2H), 1.14 (t, *J* = 7.1 Hz, 3H). |
| **19** | 391,42 | huile jaune | 95,1 | 392 | 390 | 10.09 (s, 1H), 8.56 (d, *J* = 6.8 Hz, 1H), 7.90 (s, 1H), 7.68 (d, *J* = 9.1 Hz, 1H), 7.56 (d, *J* = 7.8 Hz, 1H), 7.52 - 7.26 (m, 3H), 7.14 (t, *J* = 6.8 Hz, 1H), 4.77 (s, 2H), 4.22 (s, 2H), 4.09 (q, *J* = 7.1 Hz, 2H), 2.06 (s, 3H), 1.14 (t, *J* = 7.1 Hz, 3H). |
| **20** | 400,38 | Solide | 96,9 | 401 | | 8.68 (d, J = 6.8 Hz, 1H), 7.69 (d, J = 9.0 Hz, 1H), 7.60-7.40 (m, 4H), 7.32 (s, 1H), 7.30 - 7.22 (m, 1H), 7.15 (td, J = 6.8, 1.1 Hz, 1H), 4.79 (s, 2H), 4.24 (s, 2H), 4.13 - 4.05 (m, 2H), 1.15 (t, J = 7.1 Hz, 3H). |
| **21** | 394,42 | huile marron | >99 | 395 | | 8.60 (s, 1H), 7.64 (s, 1H), 7.38 (s, 1H), 7.18 (d, J = 6.9 Hz, 2H), 7.06 (t, J = 6.6 Hz, 1H), 6.98 (d, J = 8.9 Hz, 1H), 4.69 (d, J = 17.6 Hz, 2H), 4.25 (d, J = 31.2 Hz, 2H), 4.04 (q, J = 7.1 Hz, 2H), 3.75 (d, J = 4.6 Hz, 6H), 1.09 (t, J = 7.1 Hz, 3H). |
| **22** | 377,44 | Solide | >99 | 378 | | 8.52 (d, J = 6.7 Hz, 1H), 7.64 (d, J = 8.9 Hz, 1H), 7.52-7.33 (m, 3H), 7.09 (t, J = 6.7 Hz, 1H), 6.75 (d, J = 9.0 Hz, 2H), 4.74 (s, 2H), 4.21 (s, 2H), 4.09 (q, J = 7.1 Hz, 2H), 2.97 (s, 6H), 1.15 (t, J = 7.1 Hz, 3H). |
| **23** | 352,36 | huile marron | >99 | 353 | | 8.68 (d, J = 6.7 Hz, 1H), 7.69 (d, J = 9.0 Hz, 1H), 7.64-7.39 (m, 4H), 7.37 - 7.22 (m, 1H), 7.14 (t, J = 6.5 Hz, 1H), 4.79 (s, 2H), 4.24 (s, 2H), 4.10 (q, J = 7.1 Hz, 2H), 1.15 (t, J = 7.1 Hz, 3H). |
| **24** | 364,40 | huile marron | >99 | 365 | | 8.50 (d, J = 6.8 Hz, 1H), 7.68 (d, J = 9.0 Hz, 1H), 7.58 (dd, J = 7.6, 1.7 Hz, 1H), 7.49 - 7.39 (m, 2H), 7.16 (ddd, J = 8.2, 6.0, 2.6 Hz, 2H), 7.02 (td, J = 7.5, 0.8 Hz, 1H), 4.76 (s, 2H), 4.24 (s, 2H), 4.09 (q, J = 7.1 Hz, 2H), 3.92 (s, 3H), 1.14 (t, J = 7.1 Hz, 3H). |
| **25** | 352,36 | huile marron | 98,7 | 353 | | 8.56 (d, J = 6.6 Hz, 1H), 7.85 - 7.65 (m, 2H), 7.61 - 7.47 (m, 2H), 7.43 (dd, J = 9.6, 8.8 Hz, 1H), 7.39 - 7.32 (m, 1H), 7.18 (t, J = 6.8 Hz, 1H), 4.79 (s, 2H), 4.24 (s, 2H), 4.10 (q, J = 7.1 Hz, 2H), 1.15 (t, J = 7.1 Hz, 3H). |
| **26** | 402,37 | huile marron | 95 | 403 | | 8.60 (s, 1H), 7.96 (d, J = 7.6 Hz, 1H), 7.74 (ddd, J = 43.4, 18.7, 7.7 Hz, 4H), 7.50 (s, 1H), 7.22 (t, J = 6.5 Hz, 1H), 4.79 (s, 2H), 4.26 (s, 2H), 4.10 (q, J = 7.0 Hz, 2H), 1.15 (t, J = 7.1 Hz, 3H). |
| **98** | 434,85 | solide beige | 99 | 435 | | 8.87 (d, J = 1.7 Hz, 1H), 7.84 - 7.72 (m, 3H), 7.62 - 7.29 (m, 7H), 5.40 (s, 2H), |
| **99** | 428,40 | Solide marron | 99 | 429 | | 8.77 (d, J = 6.6 Hz, 1H), 8.08 (s, 1H), 7.94 (d, J = 7.6 Hz, 1H), 7.79 (d, J = 7.9 Hz, 1H), 7.71 (t, J = 7.7 Hz, 2H), 7.53 - 7.41 (m, 1H), 7.16 (t, J = 6.8 Hz, 1H), 5.13 (d, J = 11.9 Hz, 1H), 4.95 (d, J = 11.8 Hz, 1H), 4.30 (s, 1H), 3.99 - |

### Exemple 2 : Préparation du dérivé N°27 : acide 2-((3-((3-cyano phényl)éthynyl)imidazo[1,2-a]pyridin-2-yl)méthoxy)acétique

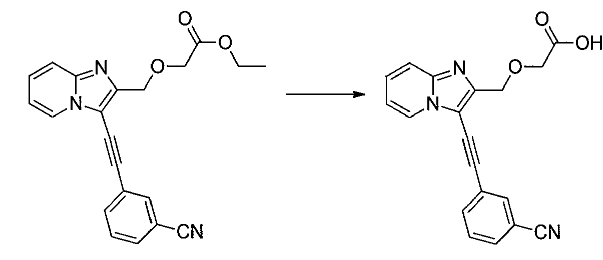

0,05 g (0,139 mmol) de 2-((3-((3-cyanophényl)éthynyl)imidazo[1,2-a] pyridin-2-yl)méthoxy) acétate d'éthyle ont été solubilisés sous agitation magnétique dans 4 ml d'un mélange éthanol / tétrahydrofurane (1/1, v/v) puis 0,167 ml (0,167 mmol) d'une solution aqueuse de NaOH 1N ont été ajoutés. Le mélange a été agité à t.a. pendant 16 h avant d'ajouter 20 ml d'eau puis 0,016 ml (0,278 mmol) d'acide acétique. La phase aqueuse a été extraite avec 3 x 20 ml d'acétate d'éthyle. Les phases organiques combinées ont été séchées sur Na₂SO₄ qui a ensuite été éliminé par filtration. Le filtrat obtenu a été concentré sous vide. 0,042 g (rendement = 91%) de acide 2-((3-((3-cyanophényl)éthynyl)imidazo[1,2-a]pyridin-2-yl)méthoxy) acétique ont été obtenus sous forme d'un solide jaune pâle. LC-MS: m/z = 332 (MH⁺) ; pureté UV à 254 nm = 99%. RMN ¹H (300 MHz, DMSO) δ 12.70 (s, 1H), 8.73 (d, J = 6.7 Hz, 1H), 8.22 (t, J = 1.3 Hz, 1H), 8.07 - 7.94 (m, 1H), 7.94 - 7.78 (m, 1H), 7.78 - 7.55 (m, 2H), 7.54 - 7.38 (m, 1H), 7.15 (td, J = 6.8, 0.9 Hz, 1H), 4.79 (s, 2H), 4.15 (s, 2H).

Les dérivés numéro **28** à **44** ont été préparés selon le même protocole.

| **No** | **Poids moléculaire** | **Apparence** | **Pureté LCMS** | **Spectrométr ie de masse** | | **RMN ¹H (300 MHz, DMSO) δ** |
|---|---|---|---|---|---|---|
| | **g/mol** | | **UV à 254 nm** | **MH⁺** | **M-H⁺** | |
| **28** | 349,38 | solide | 97,3 | 350 | | 8.52 (d, J = 6.8 Hz, 1H), 7.64 (d, *J* = 9.0 Hz, 1H), 7.46 (d, *J* = 8.9 Hz, 2H), 7.43 - 7.33 (m, 1H), 7.09 (td, *J* = 6.8, 1.1 Hz, 1H), 6.74 (d, J = 9.0 Hz, 2H), 4.74 (s, 2H), 4.12 (s, 2H), 2.97 (s, 6H). |
| **29** | 390,31 | solide | >99 | 391 | 389 | 12.68 (s, 1H), 8.72 (d, J = 6.7 Hz, 1H), 7.83 - 7.65 (m, 3H), 7.60 (t, J = 8.0 Hz, 1H), 7.46 (t, *J* = 7.8 Hz, 2H), 7.15 (t, J = 6.7 Hz, 1H), 4.79 (s, 2H), 4.15 (s, 2H). |
| **30** | 336,34 | solide | >99 | 337 | 335 | 12.70 (s, 1H), 8.65 (d, *J =* 6.7 Hz, 1H), 7.69 (d, *J =* 9.0 Hz, 1H), 7.54 - 7.33 (m, 2H), 7.26 (dd, *J =* 4.1, 2.6 Hz, 2H), 7.15 (t, *J =* 6.7 Hz, 1H), 7.02 (dd, *J =* 7.9, 2.0 Hz, 1H), 4.79 (s, 2H), 4.16 (s, 2H), 3.83 (s, 3H). |
| **31** | 350,33 | solide | >99 | | 349 | 13.00 - 11.49 (m, 1H), 9.77 (s, 1H), 8.59 (d, *J =* 6.7 Hz, 1H), 7.68 (d, *J =* 9.0 Hz, 1H), 7.51 - 7.39 (m, 1H), 7.26 (t, *J* = 7.9 Hz, 1H), 7.20 - 7.00 (m, 3H), 6.86 (ddd, *J* = 8.2, 2.4, 0.9 Hz, 1H), 4.77 (s, 2H), 4.14 (s, 2H). |
| **32** | 390,31 | solide | 94 | | 389 | 12.53 (s, 1H), 8.62 (t, *J* = 9.6 Hz, 1H), 7.81 - 7.73 (m, 2H), 7.61 (t, *J* = 7.9 Hz, 1H), 7.44 - 7.36 (m, 3H), 7.15 - 7.02 (m, 1H), 4.71 (d, *J* = 3.8 Hz, 2H), 4.06 (d, *J* = 12.9 Hz, 2H). |
| **33** | 322,32 | solide | 92,7 | | 321 | 9.75 (s, 1H), 8.57 (d, *J =* 6.7 Hz, 1H), 7.67 (d, *J =* 9.0 Hz, 1H), 7.49 - 7.37 (m, 1H), 7.25 (t, *J* = 7.9 Hz, 1H), 7.18 - 7.07 (m, 2H), 7.07 - 6.97 (m, 1H), 6.84 (dd, *J* = 8.2, 1.5 Hz, 1H), 4.76 (s, 2H), 4.10 (s, 2H). |
| **34** | 363,37 | solide | 95,3 | | 362 | 12.60 (s, 1H), 10.08 (s, 1H), 8.56 (d, J = 6.7 Hz, 1H), 7.89 (s, 1H), 7.68 (d, J = 9.0 Hz, 1H), 7.58 (d, J = 7.5 Hz, 1H), 7.52 - 7.33 (m, 3H), 7.14 (t, J = 6.5 Hz, 1H), 4.77 (s, 2H), 4.14 (s, 2H), 2.06 (s, 3H). |
| **35** | 372,32 | solide | >99 | 373 | 371 | 12.68 (s, 1H), 8.68 (d, *J* = 6.7 Hz, 1H), 7.69 (d, *J* = 9.0 Hz, 1H), 7.63 - 7.39 (m, 4H), 7.32 (s, 1H), 7.29 - 7.19 (m, 1H), 7.19 - 7.11 (m, 1H), 7.08 (s, 1H), 4.78 (s, 1H), 4.15 (s, 2H). |
| **36** | 366,37 | solide | >99 | 367 | | 12.67 (s, 8H), 8.60 (d, J = 6.3 Hz, 10H), 7.66 (d, J = 9.0 Hz, 13H), 7.52 - 7.34 (m, 16H), 7.24 (s, 23H), 7.12 (t, J = 6.6 Hz, 12H), 7.03 (d, J = 8.8 Hz, 12H), 4.76 (s, 20H), 4.15 (s, 21H), 3.81 (d, J = 3.9 Hz, 60H). |
| **37** | 345,35 | solide | 94,6 | 346 | 344 | 8.63 (d, *J* = 6.7 Hz, 1H), 7.69 (t, *J* = 8.3 Hz, 3H), 7.45 (t, *J* = 6.4 Hz, 3H), 7.14 (t, *J* = 6.3 Hz, 1H), 4.78 (s, 2H), 4.13 (d, *J* = 4.8 Hz, 4H). |
| **38** | 342,30 | solide marron | >99 | 343 | 341 | 12.38 (s, 1H), δ 8.69 (s, 1H), 7.84 (dd, J = 11.1, 8.0 Hz, 1H), 7.69 (d, J = 8.3 Hz, 1H), 7.63 - 7.51 (m, 2H), 7.46 (s, 1H), 7.15 (t, J = 6.2 Hz, 1H), 4.78 (s, 2H), 4.16 (s, 2H). |
| **39** | 408,30 | solide marron | >99 | 409 | 407 | 12.66 (s, 1H) 8.70 (d, J = 6.6 Hz, 1H), 7.97 - 7.83 (m, 1H), 7.66 (q, J = 9.1 Hz, 3H), 7.47 (t, J = 7.8 Hz, 1H), 7.15 (t, J = 6.7 Hz, 1H), 4.79 (s, 2H), 4.15 (s, 2H). |
| **40** | 324,31 | solide marron | >99 | 325 | 323 | 12.62 (s, 1H) 8.67 (d, J = 6.8 Hz, 1H), 7.68 (d, J = 9.0 Hz, 1H), 7.65 - 7.39 (m, 4H), 7.35 - 7.23 (m, 1H), 7.14 (td, J = 6.8, 1.0 Hz, 1H), 4.78 (s, 2H), 4.15 (s, 2H). |
| **41** | 336,34 | solide marron | 99 | 337 | 335 | 12.63 (s, 1H) 8.58 - 8.44 (m, 1H), 7.68 (d, J = 9.0 Hz, 1H), 7.59 (dd, J = 7.6, 1.7 Hz, 1H), 7.51 - 7.36 (m, 2H), 7.16 (ddd, J = 8.2, 6.1, 2.7 Hz, 2H), 7.02 (td, J = 7.5, 0.9 Hz, 1H), 4.77 (s, 2H), 4.16 (s, 2H), 3.93 (s, 3H). |
| **42** | 324,31 | solide marron | >99 | 325 | 323 | 8.52 (d, J = 6.7 Hz, 1H), 7.74 (ddd, J = 22.0, 11.0, 5.3 Hz, 2H), 7.58 - 7.25 (m, 4H), 7.16 (t, J = 6.7 Hz, 1H), 4.78 (s, 2H), 4.13 (s, 2H). 1H under H2O peak (3,3) |
| **43** | 374,31 | huile jaune | >99 | 375 | 373 | 12.65 (s, 1H), 8.52 (s, 1H), 7.96 (d, J = 7.0 Hz, 1H), 7.86 (d, J = 7.8 Hz, 1H), 7.76 (t, J = 7.4 Hz, 2H), 7.61 (d, J = 7.6 Hz, 1H), 7.49 (s, 1H), 7.21 (s, 1H), 4.78 (s, 2H), 4.15 (s, 2H). |
| **44** | 324,31 | solide | 99 | 325 | 323 | 12.75 (s, 1H), 8.65 (d, *J* = 6.7 Hz, 1H), 7.93 - 7.60 (m, 3H), 7.47 (m, 1H), 7.34 (t, *J* = 8.9 Hz, 2H), 7.20 - 7.03 (m, 1H), 4.78 (s, 2H), 4.15 (s, 2H). |

### Exemple 3: Préparation du dérivé N°45: 2-((3-(phényl éthynyl)imidazo[1,2-a]pyridin-2-yl)méthoxy)acétate de sodium

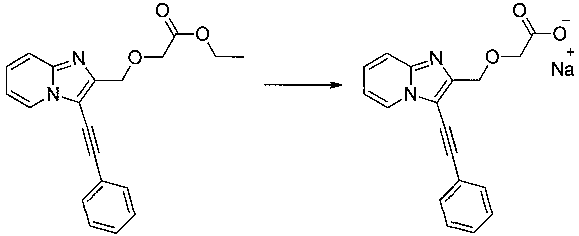

0,061 g (0,182 mmol) de 2-((3-(phényléthynyl)imidazo[1,2-a]pyridin-2-yl) méthoxy)acétate d'éthyle ont été solubilisés sous agitation magnétique dans 3 ml d'un mélange composé de méthanol et de tétrahydrofurane (1/1, v/v) puis 0,173 ml (0,173 mmol) d'une solution aqueuse de NaOH 1N ont été ajoutés. Le mélange a été agité à t.a. pendant 2 j avant d'être concentré sous vide. Le résidu brut a été trituré dans 3 ml d'éther diisopropylique, le solide obtenu a été isolé par filtration et séché sous cloche à vide pour donner 0,027 g (rendement = 45%) de 2-((3-(phényl éthynyl)imidazo[1,2-a]pyridin-2-yl)méthoxy)acétate de sodium. LC-MS: m/z = 307 (MH⁺) ; pureté UV à 254 nm = 99%. RMN ¹H (300 MHz, DMSO) δ 8.62 (d, J = 6.7 Hz, 1H), 7.79 - 7.58 (m, 3H), 7.45 (dd, J = 8.8, 7.2 Hz, 4H), 7.13 (t, J = 6.5 Hz, 1H), 4.74 (s, 2H), 3.70 (s, 2H)

Les dérivés numéro **46** à **53** ont été préparés selon le même protocole.

| **No** | **Poids moléculaire** | **Apparence** | **Pureté LCMS** | **Spectrométr ie de masse** | | **RMN ¹H (300 MHz, DMSO) δ** |
|---|---|---|---|---|---|---|
| | **g/mol** | | **UV à 254 nm** | **MH⁺** | **M-H⁺** | |
| **46** | 346,29 | solide | >99 | 325 | | 8.62 (d, *J* = 6.7 Hz, 1H), 7.77 (dd, *J* = 8.9, 5.5 Hz, 2H), 7.64 (d, *J* = 9.0 Hz, 1H), 7.48 - 7.38 (m, 1H), 7.31 (t, *J* = 8.9 Hz, 2H), 7.11 (t, *J* = 6.8 Hz, 1H), 4.72 (s, 2H), 3.67 (s, 2H). |
| **47** | 342,32 | solide | >99 | 321 | | 8.59 (d, *J* = 6.7 Hz, 1H), 7.62 (dd, *J* = 17.6, 8.5 Hz, 3H), 7.51 - 7.38 (m, 1H), 7.28 (d, *J* = 8.0 Hz, 2H), 7.12 (t, *J* = 6.8 Hz, 1H), 4.73 (s, 2H), 3.70 (s, 2H), 2.36 (s, 3H). |
| **48** | 373,30 | solide orangé | >99 | 352 | 350 | 8.75 (d, *J* = 6.4 Hz, 1H), 8.30 (d, *J* = 8.8 Hz, 2H), 7.99 (d, *J* = 8.8 Hz, 2H), 7.69 (d, *J* = 8.9 Hz, 1H), 7.49 (t, *J* = 7.6 Hz, 1H), 7.17 (t, *J* = 6.8 Hz, 1H), 4.79 (s, 2H), 3.69 (d, *J* = 21.8 Hz, 2H). |
| **49** | 358,32 | solide | >99 | 337 | 335 | 8.56 (s, 1H), 7.63 (d, *J* = 8.6 Hz, 3H), 7.42 (t, *J* = 7.1 Hz, 1H), 7.17 - 6.93 (m, 3H), 4.71 (s, 2H), 3.81 (s, 3H), 3.69 (s, 2H). |
| **50** | 396,30 | solide | 98,7 | 375 | 373 | 8.69 (d, *J* = 6.7 Hz, 1H), 7.93 (d, *J* = 8.1 Hz, 2H), 7.81 (d, *J* = 8.3 Hz, 2H), 7.66 (d, *J* = 9.0 Hz, 1H), 7.52 - 7.41 (m, 1H), 7.20 - 7.09 (m, 1H), 4.76 (s, 2H), 3.70 (s, 2H). |
| **51** | 396,30 | solide | 99 | 375 | 373 | 8.75 (dd, *J* = 6.7, 1.0 Hz, 1H), 8.13 (s, 1H), 8.01 (d, *J* = 7.6 Hz, 1H), 7.78 (d, *J* = 7.9 Hz, 1H), 7.74 - 7.62 (m, 2H), 7.51 - 7.41 (m, 1H), 7.14 (td, *J* = 6.8, 1.1 Hz, 1H), 4.76 (s, 2H), 3.70 (s, 2H). |
| **52** | 329,29 | solide | >99 | 308 | 306 | 8.91 (d, *J* = 1.4 Hz, 1H), 8.68 (d, *J* = 6.7 Hz, 1H), 8.60 (dd, *J* = 4.9, 1.6 Hz, 1H), 8.14 (dt, *J* = 7.9, 1.8 Hz, 1H), 7.67 (d, *J* = 9.0 Hz, 1H), 7.55 - 7.40 (m, 2H), 7.14 (t, *J* = 6.3 Hz, 1H), 4.76 (s, 2H), 3.70 (s, 2H). |
| **53** | 334,33 | solide | 98,4 | 313 | 311 | 8.55 (d, *J* = 5.4 Hz, 1H), 8.03 (dd, *J* = 2.9, 1.0 Hz, 1H), 7.68 (dd, *J* = 5.0, 3.0 Hz, 2H), 7.38 (dd, *J* = 5.0, 1.0 Hz, 2H), 7.10 (t, *J* = 6.8 Hz, 1H), 4.70 (s, 2H), 3.69 (s, 2H). |

### Exemple 4: Préparation du dérivé N°54: 2-((3-((4-fluoro phényl)éthynyl)imidazo[1,2-a]pyridin-2-yl)méthoxy)acétate d'isopropyle

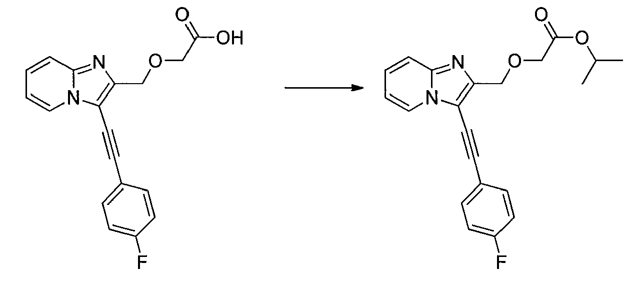

0,155 g (0,478 mmol) d'acide 2-((3-((4-fluorophényl)éthynyl)imidazo[1,2-a]pyridin-2-yl)méthoxy)acétique ont été solubilisés sous agitation magnétique dans 5 ml d'isopropanol et une goutte d'acide sulfurique a été ajoutée. Le mélange a été agité à 60°C pendant 3 h. Le milieu réactionnel a été lavé avec 2 x 10 ml d'eau, puis extrait avec 2 x 30 ml d'acétate d'éthyle. Les phases organiques combinées ont été lavées avec 30 ml d'une solution aqueuse saturée en NaCl, séchées sur Na₂SO₄ qui a ensuite été éliminé par filtration. Le filtrat obtenu a été concentré sous vide. 0,162 mg (rendement = 91%) de 2-((3-((4-fluorophényl)éthynyl)imidazo[1,2-a] pyridin-2-yl)méthoxy)acétate d'isopropyle ont été obtenus sous forme d'une huile marron. LC-MS: m/z = 367 (MH⁺) ; pureté UV à 254 nm = 98%. RMN ¹H (300 MHz, DMSO) δ 8.62 (d, *J* = 6.8 Hz, 1H), 7.74 (dd, *J* = 8.7, 5.5 Hz, 2H), 7.67 (d, *J* = 9.0 Hz, 1H), 7.45 (d, *J* = 7.0 Hz, 1H), 7.33 (t, *J* = 8.9 Hz, 2H), 7.12 (t, *J* = 6.5 Hz, 1H), 4.93 (dt, *J* = 12.5, 6.3 Hz, 1H), 4.78 (s, 2H), 4.19 (s, 2H), 1.15 (d, *J* = 6.3 Hz, 6H).

### Exemple 5: Préparation du dérivé numéro 55: N-(2-(diméthylamino)éthyl)-2-((3-((3-(trifluorométhyl)phényl) éthynyl)imidazo[1,2-a]pyridin-2-yl)méthoxy)acétamide

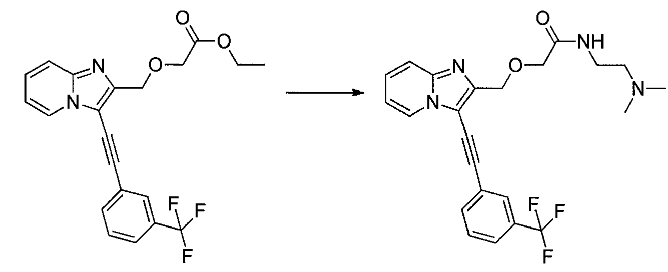

0,15 g (0,373 mmol) de 2-((3-((3-(trifluorométhyl)phényl)éthynyl)imidazo [1,2-a]pyridin-2-yl)méthoxy) acétate d'éthyle ont été solubilisés dans 2 ml de pyridine sous agitation magnétique, à cette solution ont été ajoutés 0,409 ml (3,73 mmol) de N,N-diméthyléthane-1,2-diamine et 0,249 mg (1,864 mmol) de trichlorure d'aluminium. Le mélange a été agité à t.a. pendant 18 h avant d'être traité avec 30 ml d'une solution aqueuse saturée en NaCl. La phase aqueuse a été extraite avec 3 x 30 ml d'acétate d'éthyle. Les phases organiques combinées ont été séchées sur Na₂SO₄ qui a ensuite été éliminé par filtration. Le filtrat obtenu a été concentré sous vide. 0,056 g (rendement = 34%) de N-(2-(diméthylamino)éthyl)-2-((3-((3-(trifluorométhyl)phényl)éthynyl)imidazo[1,2-a]pyridin-2-yl) méthoxy) acétamide ont été obtenus sous forme d'un solide beige. LC-MS: m/z = 445 (MH⁺) ; pureté UV à 254 nm = 96%. RMN ¹H (300 MHz, CDCl₃) δ 7.85 (s, 1H), 7.78 (d, *J* = 7.7 Hz, 2H), 7.70 (s, 2H), 7.58 (t, *J* = 7.8 Hz, 2H), 7.23 (s, 1H), 5.11 (s, 2H), 4.31 (dd*, J* = 26.6, 11.2 Hz, 2H), 3.83 (s, 2H), 3.33 (s, 2H), 2.89 (s, 6H).

### Exemple 6 : Préparation du dérivé N°56 : 2-((3-((4-fluoro phényl) éthynyl)imidazo[1,2-a]pyridin-2-yl)méthoxy)-1-morpholino éthanone

### Etape 1 : Préparation du chlorure de 2-((3-((4-fluorophényl)éthynyl)imidazo[1,2-a]pyridin-2-yl)méthoxy) acétyle

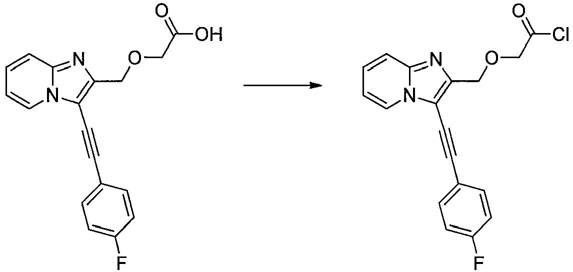

1,5 g (4,63 mmol) d'acide 2-((3-((4-fluorophényl)éthynyl)imidazo[1,2-a] pyridin-2-yl)méthoxy)acétique ont été mis en suspension dans 40 ml de Dichlorométhane sous agitation magnétique, le mélange a été placé dans un bain à 0°C puis 1,215 ml (13,88 mmol) de chlorure d'oxalyle ont été additionnés goutte à goutte, enfin, quelques gouttes de diméthylformamide ont été ajoutées. Le bain froid a été retiré puis le mélange a été agité à t.a. pendant 1 h. Le milieu réactionnel a été concentré sous vide et utilisé immédiatement dans l'étape suivante.

### Etape 2 : Préparation du 2-((3-((4-fluorophényl)éthynyl)imidazo [1,2-a]pyridin-2-yl)méthoxy)-1-morpholinoéthanone (dérivé N°56)

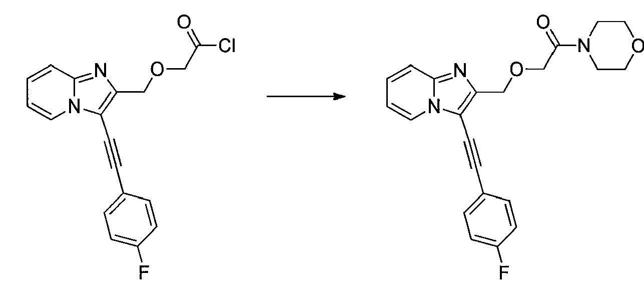

0,806 ml (9,25 mmol) de morpholine ont été solubilisés dans 4 ml de Dichlorométhane sous agitation magnétique, une solution de chlorure de 2-((3-((4-fluorophényl)éthynyl)imidazo[1,2-a]pyridin-2-yl)méthoxy)acétyle dans 4 ml de Dichlorométhane a été ajoutée puis le mélange a été additionné à t.a. pendant 1 h avant d'être traité avec 10 ml d'eau. La phase aqueuse a été extraite avec 2 x 5 ml de Dichlorométhane. Les phases organiques combinées ont été lavées avec 5 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : acétate d'éthyle 100%). 0,211 g (rendement = 58%) de 2-((3-((4-fluorophényl)éthynyl)imidazo[1,2-a]pyridin-2-yl)méthoxy)-1-morpholino éthanone ont été obtenus sous forme d'un solide jaune clair. LC-MS: m/z = 394 (MH⁺) ; pureté UV à 254 nm = 99%. RMN ¹H (300 MHz, DMSO) δ 8.62 (d, *J* = 6.8 Hz, 1H), 7.74 (dd, *J* = 8.7, 5.5 Hz, 2H), 7.67 (d, *J* = 9.0 Hz, 1H), 7.50 - 7.39 (m, 1H), 7.33 (t, *J* = 8.9 Hz, 2H), 7.12 (t, *J* = 6.8 Hz, 1H), 4.75 (s, 2H), 4.27 (s, 2H), 3.55 - 3.46 (m, 4H), 3.39 (s, 4H).

Les dérivés numéro **57** à **59** ont été obtenus à partir du chlorure de 2-((3-((4-fluorophényl)éthynyl)imidazo[1,2-a]pyridin-2-yl)méthoxy)acétyle selon le même protocole.

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **57** | 394,44 | solide | 99 | 395 | | 8.64 (d, J = 6.8 Hz, 1H), 7.82 - 7.59 (m, 4H), 7.51 - 7.41 (m, 1H), 7.34 (t, *J* = 8.9 Hz, 2H), 7.19 - 7.05 (m, 1H), 4.79 (s, 2H), 4.03 (s, 2H), 3.17 (dd, *J* = 12.6, 6.5 Hz, 2H), 2.24 (t, *J* = 6.7 Hz, 2H), 2.09 (s, 6H). |
| **58** | 367,37 | solide | 99 | 368 | | 11.10 (s, 1H), 8.63 (d, *J =* 6.7 Hz, 1H), 7.76 (dd, *J =* 8.6, 5.5 Hz, 2H), 7.68 (d, *J* = 9.0 Hz, 1H), 7.49 - 7.41 (m, 1H), 7.33 (t, *J* = 8.8 Hz, 2H), 7.13 (t, *J =* 6.7 Hz, 1H), 4.77 (s, 2H), 4.02 (s, 2H), 3.78 (q, *J =* 7.0 Hz, 2H), 1.10 (t, *J* = 7.0 Hz, 3H). |
| **59** | 339,32 | solide | 97,4 | 340 | | 10.60 (s, 1H), 8.84 (s, 1H), 8.63 (d, *J* = 6.6 Hz, 1H), 7.87 - 7.60 (m, 3H), 7.36 (dd, *J* = 31.0, 22.2 Hz, 3H), 7.13 (t, *J* = 6.7 Hz, 1H), 4.76 (s, 2H), 4.01 (s, 2H). |

### Exemple 7 : Préparation du dérivé numéro 60: 2-((3-((4-fluorophényl)éthynyl)imidazo[1,2-a]pyridin-2-yl)méthoxy)-1-morpholinoéthanone

### Etape 1 : Préparation du 2-(((triisopropylsilyl)oxy)méthyl) imidazo[1,2-a]pyridine

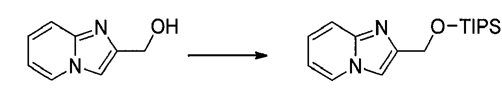

1 g (6,55 mmol) de imidazo [1,2-a]pyridin-2-ylméthanol ont été solubilisés dans 25 ml de Dichlorométhane sous agitation magnétique puis 2,145 ml (9,82 mmol) de chlorure de triisopropylsilyle ont été ajoutés lentement. Le mélange a été agité à t.a. pendant 1 h avant d'être traité avec 100 ml d'eau. La phase aqueuse a été extraite avec 3 x 50 ml de Dichlorométhane. Les phases organiques combinées ont été lavées avec 100 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : Dichlorométhane 100% puis acétate d'éthyle 100%). 1,85 g (rendement = 92%) de 2-(((triisopropylsilyl)oxy) méthyl)imidazo[1,2-a] pyridine ont été obtenus sous forme d'une huile incolore. LC-MS: m/z = 305 (MH⁺) ; pureté UV à 254 nm = 99%. RMN ¹H (300 MHz, DMSO) δ 8.57 - 8.50 (m, 1H), 7.81 (s, 1H), 7.46 (dd, *J* = 9.1, 0.6 Hz, 1H), 7.19 (ddd, *J* = 9.1, 6.7, 1.3 Hz, 1H), 6.84 (td, J = 6.8, 1.1 Hz, 1H), 4.88 (d, *J* = 0.6 Hz, 2H), 1.23 - 1.11 (m, 3H), 1.11 - 1.02 (m, 18H).

### Etape 2 : Préparation du 3-iodo-2-(((triisopropylsilyl)oxy) méthyl)imidazo[1,2-a]pyridine

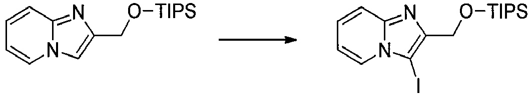

1,85 g (6,08 mmol) de 2-(((triisopropylsilyl)oxy)méthyl)imidazo[1,2-a] pyridine ont été solubilisés dans 25 ml d'acétonitrile sous agitation magnétique, à cette solution ont été ajoutés 1,409 g (6,08 mmol) de N-iodosuccinimide. Le mélange a été agité à t.a. pendant 16 h avant d'être traité par 100 ml d'eau. La phase aqueuse a été extraite avec 3 x 50 ml de Dichlorométhane. Les phases organiques combinées ont été lavées avec 100 ml d'une solution aqueuse saturée en NaCl, séchées sur Na₂SO₄ qui a ensuite été éliminé par filtration. Le filtrat obtenu a été concentré sous vide. 2,29 g (rendement = 83%) de 3-iodo-2-(((triisopropylsilyl)oxy) méthyl)imidazo[1,2-a]pyridine ont été obtenus sous forme d'un solide jaune clair. LC-MS: m/z = 431 (MH⁺) ; pureté UV à 254 nm = 95%. RMN ¹H (300 MHz, DMSO) δ 8.30 (dd, *J =* 5.6, 2.2 Hz, 1H), 7.59 (dd, *J =* 14.3, 9.1 Hz, 1H), 7.33 (tt, *J =* 6.8, 4.1 Hz, 1H), 7.14 - 6.96 (m, 1H), 4.80 (s, 2H), 1.27 - 0.90 (m, 21H).

### Etape 3 : Préparation du 3-(phényléthynyl)-2-(((triisopropylsilyl) oxy)méthyl)imidazo[1,2-a]pyridine

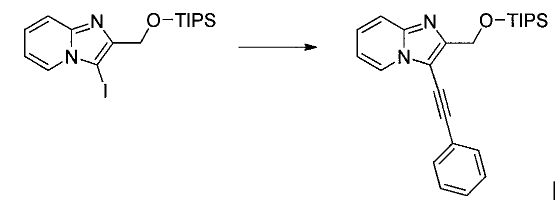

Dans un ballon placé sous un flux d'argon, 2,29 g (5,32 mmol) de 3-iodo-2-(((triisopropylsilyl)oxy)méthyl)imidazo[1,2-a]pyridine ont été solubilisés dans 11 ml de diméthylformamide sous agitation magnétique. A cette solution, ont été additionnés : 0,103 g (0,532 mmol) d'iodure de cuivre, 0,715 ml (6,38 mmol) de phénylacétylène et 11,12 ml (80 mmol) de triéthylamine. Le milieu réactionnel a été dégazé à l'argon pendant 10 min avant d'ajouter 0,487 g (0,532 mmol) de Pd₂(dba)₃. Le mélange a été agité à t.a. pendant 16 h avant d'être filtré sur célite. La célite a été lavée avec 200 ml d'acétate d'éthyle. Le filtrat a été lavé avec 2 x 100 ml d'eau et 100 ml d'une solution aqueuse saturée en NaCl puis séché sur Na₂SO₄ qui a ensuite été éliminé par filtration. Le filtrat obtenu a été concentré sous vide. Le résidu brut a été purifié par chromatographie flash sur une cartouche de gel de silice (éluant : gradient d'heptane / acétate d'éthyle, de 90% à 80% d'heptane, v/v). 1,56 g (rendement = 72%) de 2 3-(phényléthynyl)-2-(((triisopropylsilyl)oxy)méthyl)imidazo[1,2-a]pyridine ont été obtenus sous forme d'un solide marron. LC-MS: m/z = 405 (MH⁺) ; pureté UV à 254 nm = 99%. RMN ¹H (300 MHz, DMSO) δ 8.60 (d, *J* = 6.7 Hz, 1H), 7.72 - 7.55 (m, 3H), 7.52 - 7.36 (m, 4H), 7.17 - 7.06 (m, 1H), 4.94 (s, 2H), 1.14 (m, 3H), 1.04 (d, *J =* 6.5 Hz, 18H).

### Etape 4 : Préparation du (3-(phényléthynyl)imidazo[1,2-a]pyridin-2-yl)méthanol (dérivé numéro 60)

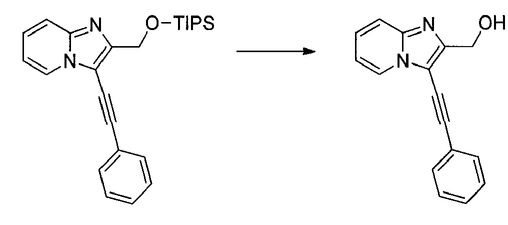

1,56 g (3,86 mmol) de 2-(((triisopropylsilyl)oxy)méthyl)imidazo[1,2-a]pyridine ont été solubilisés dans 30 ml de tétrahydrofurane sous agitation magnétique, à cette solution ont été ajoutés 4,24 ml (4,24 mmol) d'une solution de fluorure de tetrabutylamonium 1M dans le tétrahydrofurane. Le mélange a été agité à t.a. pendant 1 heure avant d'être concentré sous vide. Le résidu brut a été directement purifié par chromatographie flash sur une cartouche de gel de silice (éluant : acétate d'éthyle 100%). 0,83 g (rendement = 86%) de (3-(phényléthynyl)imidazo [1,2-a]pyridin-2-yl)méthanol ont été obtenus sous forme d'un solide orangé. LC-MS: m/z = 249 (MH⁺) ; pureté UV à 254 nm = 99%. RMN ¹H (300 MHz, DMSO) δ 8.59 (dt, J= 6.7, 1.1 Hz, 1H), 7.77 - 7.56 (m, 3H), 7.56 - 7.32 (m, 4H), 7.11 (td, *J* = 6.8, 1.1 Hz, 1H), 5.33 (t, *J* = 5.9 Hz, 1H), 4.67 (d, *J* = 5.9 Hz, 2H).

Le dérivé numéro **100** a été obtenu selon le même protocole.

| **No** | **Poids moléculaire** | **Apparence** | **Pureté LCMS** | **Spectromé trie de masse m/z** | **RMN ¹H (300 MHz, DMSO) δ** |
|---|---|---|---|---|---|
| | **g/mol** | | **UV à 254** | **MH⁺** | |
| **100** | 316,28 | solide marron clair | 94 | 317,1 | 8.74 (d, J = 6.7 Hz, 1H), 8.10 (s, 1H), 7.96 (d, J = 7.6 Hz, 1H), 7.83 - 7.63 (m, 3H), 7.50 - 7.39 (m, 1H), 7.13 (t, J = 6.5 Hz, 1H), 5.36 (t, J = 5.9 Hz, 1H), 4.70 (d, J = 5.9 Hz, 2H) |

### Exemple 8: Préparation du dérivé N°61 : ((3-(phényl éthynyl)imidazo[1,2-a]pyridin-2-yl)méthyl) carbonate d'isobutyle

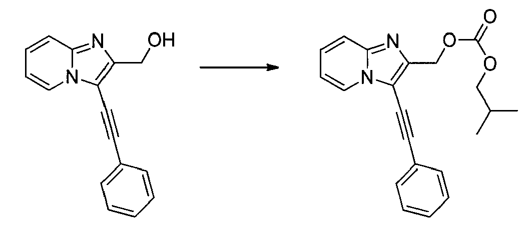

0,1 g (0,403 mmol) de (3-(phényléthynyl)imidazo[1,2-a]pyridin-2-yl) méthanol ont été solubilisés dans 3 ml de Dichlorométhane sous agitation magnétique. 0,084 ml (0,604 mmol) de triéthylamine et 0,059 ml (0,443 mmol) de chloroformiate d'isobutyle ont été ajoutés. Le mélange a été agité à t.a. pendant 15 min avant d'être concentré sous vide. Le résidu a été repris dans 10 ml d'éther diisopropylique et un solide a été isolé par filtration. Le solide a été purifié par HPLC préparative et 0,084 g (rendement = 59%) de ((3-(phényléthynyl)imidazo[1,2-a]pyridin-2-yl) méthyl) carbonate d'isobutyle ont été obtenus sous forme d'un solide jaune clair. LC-MS: m/z = 349 (MH⁺) ; pureté UV à 254 nm = 99%. RMN ¹H (300 MHz, DMSO) δ 8.70 (d, *J* = 6.8 Hz, 1H), 7.79 - 7.65 (m, 3H), 7.60 - 7.45 (m, 4H), 7.23 (td, *J* = 6.8, 1.1 Hz, 1H), 5.42 (s, 2H), 3.91 (d, *J* = 6.6 Hz, 2H), 1.95 - 1.80 (m, 1H), 0.86 (d, *J* = 6.7 Hz, 6H).

### Exemple 9 : Préparation du dérivé N°62: (3-(phényléthynyl) imidazo[1,2-a]pyridin-2-yl)méthyl morpholine-4-carboxylate

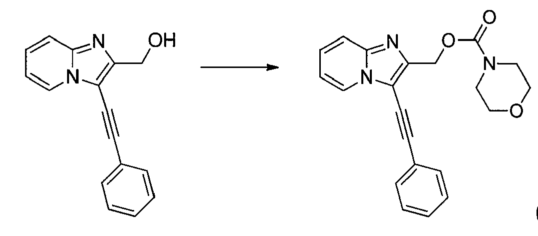

0,117 g (0,467 mmol) de (3-(phényléthynyl)imidazo[1,2-a]pyridin-2-yl) méthanol ont été solubilisés dans 4 ml de tétrahydrofurane sous agitation magnétique. 0,228 g (0,7 mmol) de carbonate de césium et 0,61 ml (0,513 mmol) de chlorure de morpholine-4-carbonyle ont été ajoutés. Le mélange a été agité à t.a. pendant 16 heures. 1,22 ml (1,026 mmol) de chlorure de morpholine-4-carbonyle supplémentaire ont été ajoutés et le mélange a été agité à t.a. 2 jours avant d'être traité avec 10 ml d'une solution aqueuse saturée en NaCl. La phase aqueuse a été extraite avec 3 x 10 ml d'acétate d'éthyle. Les phases organiques combinées ont été séchées sur Na₂SO₄ qui a ensuite été éliminé par filtration. Le résidu brut a été purifié par HPLC préparative et 0,078 g (rendement = 46%) de (3-(phényléthynyl)imidazo[1,2-a]pyridin-2-yl)méthylmorpholine-4-carboxylate ont été obtenus sous forme d'un solide beige. LC-MS: m/z = 362 (MH⁺) ; pureté UV à 254 nm = 99%. RMN ¹H (300 MHz, DMSO) δ 8.60 (d, J = 6.7 Hz, 1H), 7.66 (dd, J = 11.0, 5.4 Hz, 3H), 7.47 (dd, J = 4.1, 1.3 Hz, 4H), 7.14 (t, J = 6.4 Hz, 1H), 5.33 (s, 2H), 3.48 (s, 4H), 3.34 (s, 4H).

### Exemple 10 : Préparation du dérivé N°63: (3-(phényl éthynyl)imidazo[1,2-a]pyridin-2-yl)méthyl diéthylcarbamate

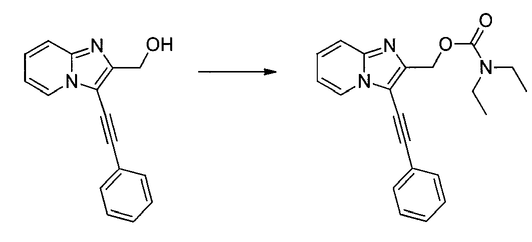

0,1 g (0,403 mmol) de (3-(phényléthynyl)imidazo[1,2-a]pyridin-2-yl) méthanol ont été solubilisés dans 3 ml de diméthylformamide sous agitation magnétique. Le mélange a été refroidi à 0°C, 0,048 g (1,208 mmol) d'hydrure de sodium (60% en masse) ont été additionnés par portion, le mélange a été agité 10 min à 0°C puis 0,204 ml (1,611 mmol) de chlorure de diéthylcarbamoyle ont été ajoutés. Le bain froid a été retiré, le mélange a été agité à t.a. pendant 2 h avant d'être traité avec 30 ml d'une solution aqueuse saturée en NaCl. La phase aqueuse a été extraite avec 3 x 10 ml d'acétate d'éthyle. Les phases organiques combinées ont été séchées sur Na₂SO₄ qui a ensuite été éliminé par filtration. Le résidu brut a été purifié par chromatographie flash sur une cartouche de gel de silice (éluant : heptane / acétate d'éthyle, 6/4, v/v). 0,101 g (rendement = 72%) de (3-(phényléthynyl)imidazo[1,2-a]pyridin-2-yl)méthyl diéthylcarbamate ont été obtenus sous forme d'un solide jaune pâle. LC-MS: m/z = 348 (MH⁺) ; pureté UV à 254 nm = 99%. RMN ¹H (300 MHz, DMSO) δ 8.61 (d, *J* = 6.8 Hz, 1H), 7.74 - 7.59 (m,3H), 7.53 - 7.38 (m, 4H), 7.13 (td, *J* = 6.8, 1.1 Hz, 1H), 5.30 (s, 2H), 3.22 (q, *J* = 7.1 Hz, 4H), 1.01 (t, *J* = 7.1 Hz, 6H).

### Exemple 11: Préparation du dérivé N°64: 3-((2-((2-hydroxy éthoxy)méthyl)imidazo[1,2-a]pyridin-3-yl)éthynyl)benzonitrile

### Etape 1 : Préparation du 2-(chlorométhyl)-3-iodoimidazo[1,2-a] pyridine

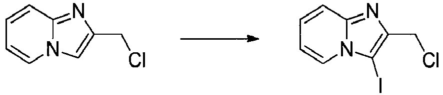

5 g (30 mmol) de 2-(chlorométhyl)imidazo[1,2-a]pyridine ont été solubilisés dans 60 ml d'acétonitrile sous agitation magnétique, 7,31 g (31,5 mmol) de N-iodosuccinimide ont été ajoutés et le mélange a été agité à t.a. pendant 15 min. Le solide formé a été isolé par filtration, lavé avec 30 ml d'eau et séché sous cloche à vide. 8,46 g (rendement = 95%) de 2-(chlorométhyl)-3-iodoimidazo[1,2-a]pyridine ont été obtenus sous forme d'un solide blanc. LC-MS: m/z = 293 (MH⁺) ; pureté UV à 254 nm = 99%. RMN ¹H (300 MHz, DMSO) δ 8.31 (d, *J* = 6.9 Hz, 1H), 7.58 (d, *J* = 9.1 Hz, 1H), 7.46 - 7.29 (m, 1H), 7.07 (td, *J* = 6.8, 1.0 Hz, 1H), 4.81 (s, 2H).

### Etape 2: Préparation du (3-iodoimidazo[1,2-a]pyridin-2-yl)méthyl 3-hydroxypropanoate

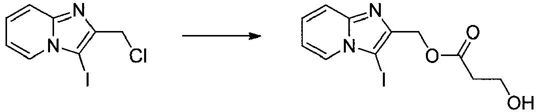

Dans un ballon placé sous un flux d'argon, 0,515 ml (3,42 mmol) de 2-(chlorométhyl)-3-iodoimidazo[1,2-a]pyridine ont été solubilisés sous agitation magnétique dans 8 ml de tétrahydrofurane puis 0,06 g (1,504 mmol) d'hydrure de sodium (60% en masse) ont été ajoutés, le mélange a été agité à t.a. pendant 30 min puis 0,4 g (1,368 mmol) de 2-(chlorométhyl)-3-iodoimidazo[1,2-a]pyridine et 0,05 ml (0,137 mmol) d'iodure de tétrabutyle ammonium ont été ajoutés. Le mélange a été agité à reflux pendant 2 j. Le milieu réactionnel a été traité avec 30 ml d'une solution aqueuse de chlorure d'ammonium saturé. La phase aqueuse a été extraite avec 2 x 30 ml d'acétate d'éthyle. Les phases organiques combinées ont été lavées avec 30 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : gradient d'heptane / acétate d'éthyle, 1/1, v/v). 0,17 g (rendement = 36%) de (3-iodoimidazo[1,2-a]pyridin-2-yl)méthyl 3-hydroxypropanoate ont été obtenus sous forme d'un solide marron. LC-MS: m/z = 347 (MH⁺) ; pureté UV à 254 nm = 99%. RMN ¹H (300 MHz, DMSO) δ 8.32 (d, *J* = 6.9 Hz, 1H), 7.57 (d, *J* = 9.1 Hz, 1H), 7.42 - 7.24 (m, 1H), 7.07 (t, *J* = 6.4 Hz, 1H), 5.16 (s, 2H), 4.73 (t, *J* = 5.3 Hz, 1H), 3.64 (dd, *J* = 11.6, 6.0 Hz, 2H), 2.46 (m, 2H).

### Etape 3: Préparation du (3-((3-cyanophényl)éthynyl)imidazo [1,2-a]pyridin-2-yl)méthy/3-hydroxypropanoate (dérivé N°64)

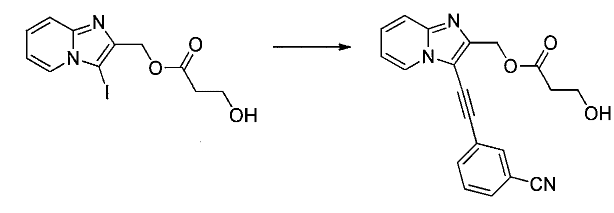

Dans un ballon placé sous un flux d'argon, 0,138 g (0,397 mmol) de (3-iodoimidazo[1,2-a]pyridin-2-yl)méthyl 3-hydroxypropanoate ont été solubilisés dans 4 ml de diméthylformamide sous agitation magnétique. A cette solution, ont été additionnés : 0,008 g (0,04 mmol) d'iodure de cuivre, 0,06 g (0,477 mmol) de 3-éthynylbenzonitrile et 0,831 ml (5,96 mmol) de triéthylamine. Le milieu réactionnel a été dégazé à l'argon pendant 10 min avant d'ajouter 0,036 g (0,04 mmol) de Pd₂(dba)₃. Le mélange a été agité à t.a. pendant 1 h avant d'être traité avec 50 ml d'une solution aqueuse saturée en NaCl, la phase aqueuse a été extraite avec 2 x 50 ml d'acétate d'éthyle. Les phases organiques combinées ont été lavées avec 50 ml d'une solution aqueuse saturée en NaCl puis séché sur Na₂SO₄ qui a ensuite été éliminé par filtration. Le filtrat obtenu a été concentré sous vide. Le filtrat obtenu a été concentré sous vide. Le résidu brut a été purifié par chromatographie flash sur une cartouche de gel de silice (éluant : heptane / acétate d'éthyle, 2/8, v/v). 0,12 g (rendement = 82%) de (3-((3-cyanophényl)éthynyl)imidazo[1,2-a]pyridin-2-yl)méthyl 3-hydroxy propanoate ont été obtenus sous forme d'un solide jaune. LC-MS: m/z = 346 (MH⁺) ; pureté UV à 254 nm = 93%. RMN ¹H (300 MHz, DMSO) δ 8.74 (d, J = 6.8 Hz, 1H), 8.26 (s, 1H), 7.98 (dd, J = 5.3, 3.9 Hz, 1H), 7.90 (dd, J = 5.2, 3.8 Hz, 1H), 7.76 - 7.63 (m, 2H), 7.57 - 7.41 (m, 1H), 7.18 (td, J = 6.8, 1.0 Hz, 1H), 5.35 (s, 2H), 4.76 (t, J = 5.3 Hz, 1H), 3.67 (dd, J = 11.6, 6.2 Hz, 2H), 2.54 -2.49 (m,2H).

Les dérivés numéro **65** à **67** ont été préparés selon le même enchaînement d'étapes 1 à 4.

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **65** | 317,34 | solide | 99 | 318 | | 8.72 (d, *J* = 6.7 Hz, 1H), 8.25 (s, 1H), 7.98 (d, *J* = 7.9 Hz, 1H), 7.88 (d, *J* = 7.8 Hz, 1H), 7.67 (t, *J* = 8.0 Hz, 2H), 7.54 - 7.38 (m, 1H), 7.14 (t, *J* = 6.7 Hz, 1H), 4.72 (s, 2H), 4.66 (t, *J* = 5.2 Hz, 1H), 3.56 (dd, *J* = 8.3, 4.6 Hz, 4H). |
| **66** | 388,34 | solide | 99 | 389 | | 8.76 (d, *J* = 6.7 Hz, 1H), 8.12 (s, 1H), 7.97 (d, *J* = 7.6 Hz, 1H), 7.80 (d, *J* = 7.9 Hz, 1H), 7.71 (dd, *J* = 8.3, 5.8 Hz, 2H), 7.55 - 7.43 (m, 1H), 7.17 (t, *J* = 6.7 Hz, 1H), 5.36 (s, 2H), 4.74 (t, *J* = 5.3 Hz, 1H), 3.66 (dd, *J =* 11.7, 6.0 Hz, 2H), 2.52 (d, *J* = 6.4 Hz, 2H). |
| **67** | 360,33 | solide | 96 | 363 | 361 | 8.77 (d, *J* = 6.7 Hz, 1H), 8.13 (s, 1H), 7.99 (d, *J =* 7.6 Hz, 1H), 7.81 (d, *J* = 7.8 Hz, 1H), 7.72 (t, *J =* 8.8 Hz, 2H), 7.56 - 7.42 (m, 1H), 7.16 (td, *J* = 6.8, 1.0 Hz, 1H), 4.75 (s, 2H), 4,70 (s,1H), 3.58 (td, *J* = 7.7, 4.0 Hz, 4H). |

### Exemple 12: Préparation du dérivé N°68: acide 2-(((3-((3-(trifluorométhyl)phényl)éthynyl)imidazo[1,2-a]pyridin-2-yl) méthyl) amino)acétique

### Etape 1: Préparation du 2-(imidazol[1,2-a]pyridin-2-ylméthyl) isoindoline-1,3-dione

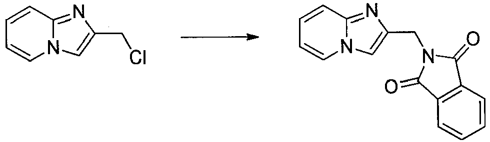

1,5 g (9 mmol) de 2-(chlorométhyl)imidazo[1,2-a]pyridine ont été solubilisés dans 55 ml de diméthylformamide sous agitation magnétique, 1,834 g (9,9 mmol) de phtalimide de potassium ont été ajoutés par portions puis le mélange a été agité à 60°C pendant 2 h. Le solide obtenu a été isolé par filtration, lavé avec 30 ml d'éther diisopropylique et séché sous cloche à vide pour donner 1,81 g (rendement = 67%) de 2-(imidazo [1,2-a]pyridin-2-ylméthyl)isoindoline-1,3-dione sous forme d'un solide blanc. LC-MS: m/z = 278 (MH⁺) ; pureté UV à 254 nm = 93%. RMN ¹H (300 MHz, DMSO) δ 8.45 (d, *J* = 6.8 Hz, 1H), 7.96 - 7.67 (m, 5H), 7.45 (dd, *J* = 9.1, 0.5 Hz, 1H), 7.33 - 7.14 (m, 1H), 6.84 (td, *J* = 6.8, 1.1 Hz, 1H), 4.88 (s, 2H).

### Etape 2: Préparation du 2-((3-iodoimidazo[1,2-a]pyridin-2-yl) méthyl)isoindoline-1,3-dione

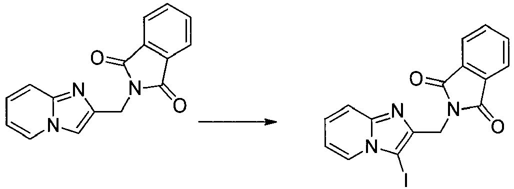

1,7 g (5,7 mmol) de 2-(chlorométhyl)imidazo[1,2-a]pyridine ont été solubilisés dans 65 ml d'acétonitrile sous agitation magnétique, 1,389 g (5,99 mmol) de N-iodosuccinimide ont été ajoutés et le mélange a été agité à t.a. pendant 1 h. Le solide formé a été isolé par filtration, lavé avec 20 ml d'éther diisopropylique et séché sous cloche à vide. 0,105 g (rendement = 87%) de 2-((3-iodoimidazo[1,2-a]pyridin-2-yl)méthyl) isoindoline-1,3-dione ont été obtenus sous forme d'un solide blanc. LC-MS: m/z = 404 (MH⁺) ; pureté UV à 254 nm = 78%. RMN ¹H (300 MHz, DMSO) δ 8.29 (d, *J* = 6.8 Hz, 1H), 7.89 (d, *J* = 5.6 Hz, 4H), 7.48 (d, *J* = 9.0 Hz, 1H), 7.36 - 7.24 (m, 1H), 7.03 (t, *J* = 6.4 Hz, 1H), 4.89 (s, 2H).

### Etape 3: Préparation du 2-((3-((3-(trifluorométhyl)phényl) éthynyl)imidazo[1,2-a]pyridin-2-yl)méthyl)isoindoline-1,3-dione

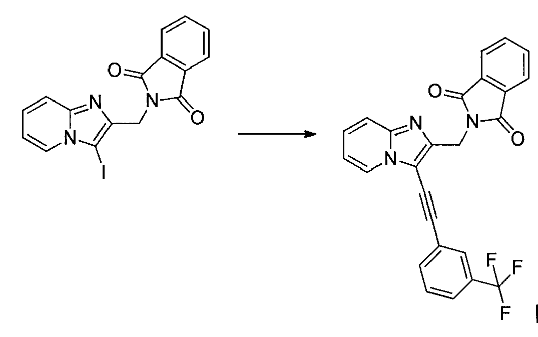

Dans un ballon placé sous un flux d'argon, 2,1 g (5,21 mmol) de 2-((3-iodoimidazo[1,2-a]pyridin-2-yl)méthyl)isoindoline-1,3-dione ont été solubilisés dans 11 ml de diméthylformamide sous agitation magnétique. A cette solution, ont été additionnés : 0,008 g (0,04 mmol) d'iodure de cuivre, 0,921 ml (6,25 mmol) de 3-trifluorométhylphényl acétylène et 10,89 ml (78 mmol) de triéthylamine. Le milieu réactionnel a été dégazé à l'argon pendant 10 min avant d'ajouter 0,477 g (0,521 mmol) de Pd₂(dba)₃. Le mélange a été agité à t.a. pendant 16 h avant d'être filtré sur une cartouche de gel de silice qui a ensuite été rincée avec 300 ml d'acétate d'éthyle. La phase organique a été lavée avec 3 x 150 ml d'une solution aqueuse saturée en NaCl, puis séché sur Na₂SO₄ qui a ensuite été éliminé par filtration. Le filtrat obtenu a été concentré sous vide. Le filtrat obtenu a été concentré sous vide. Le résidu brut a été purifié par chromatographie flash sur une cartouche de gel de silice (éluant : gradient d'heptane / acétate d'éthyle, de 50% à 40% d'heptane, v/v). 1,41 g (rendement = 59%) de 2-((3-((3-(trifluorométhyl)phényl)éthynyl)imidazo [1,2-a] pyridin-2-yl)méthyl) isoindoline-1,3-dione ont été obtenus sous forme d'un solide marron. LC-MS: m/z = 446 (MH⁺) ; pureté UV à 254 nm = 97%. RMN ¹H (300 MHz, DMSO) δ 8.67 (d, *J* = 6.7 Hz, 1H), 7.91 (dd, *J* = 5.6, 3.0 Hz, 2H), 7.82 (dd, *J* = 5.5, 3.1 Hz, 2H), 7.69 (dd, *J* = 12.5, 8.5 Hz, 2H), 7.63 - 7.52 (m, 2H), 7.52 - 7.42 (m, 2H), 7.13 (td, *J* = 6.8, 1.1 Hz, 1H), 5.09 (s, 2H).

### Etape 4: Préparation du (3-((3-(trifluorométhyl)phényl) éthynyl)imidazo[1,2-a]pyridin-2-yl)méthanamine

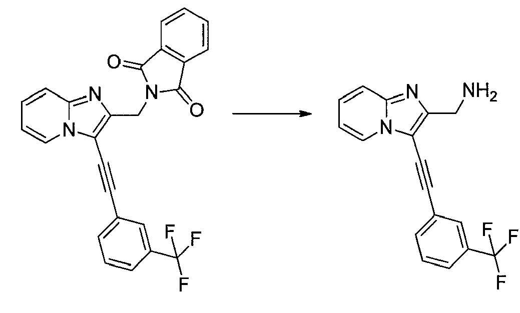

1,41 g (3,08 mmol) de 2-((3-((3-(trifluorométhyl)phényl)éthynyl) imidazo [1,2-a]pyridin-2-yl)méthyl) isoindoline-1,3-dione ont été solubilisés dans 30 ml d'éthanol sous agitation magnétique, 0,978 ml (30,8 mmol) de monohydrate d'hydrazine ont été ajoutés. Le mélange est progressivement devenu laiteux et a été agité à t.a. pendant 1,5 h. Le solide en suspension a été isolé par filtration et lavé avec 2 x 25 ml d'éthanol. Le filtrat a été concentré sous vide. Le résidu a été trituré dans 20 ml d'éthanol, le solide a été isolé par filtration et le filtrat concentré sous vide pour donner 0,715 g (rendement = 69%) de (3-((3-(trifluorométhyl)phényl)éthynyl)imidazo[1,2-a]pyridin-2-yl)méthanamine sous forme d'un solide jaune pâle. LC-MS: m/z = 316 (MH⁺) ; pureté UV à 254 nm = 94%. RMN ¹H (300 MHz, DMSO) δ 8.73 (d, *J* = 6.7 Hz, 1H), 8.12 (s, 1H), 7.98 (d, *J* = 7.7 Hz, 1H), 7.82 - 7.63 (m, 3H), 7.49 - 7.40 (m, 1H), 7.13 (td, *J* = 6.8, 1.1 Hz, 1H), 3.96 (s, 2H).

### Etape 5: Préparation de l'acide 2-(((3-((3-(trifluorométhyl) phényl)éthynyl)imidazo[1,2-a]pyridin-2-yl)méthyl)amino) acétique (dérivé numéro 68)

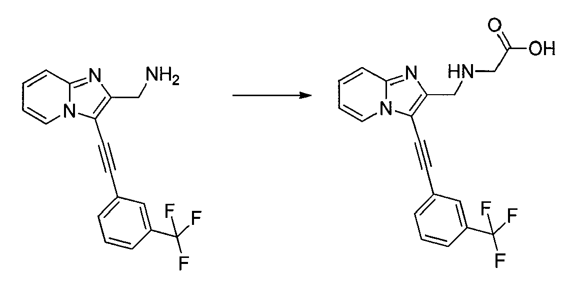

Dans un réacteur équipé d'un filtre et d'un système de vidange et placé sur un vortex, ont été introduits 0,33 g (0,656 mmol) de résine cyanoborohydrure. La résine a été gonflée dans 3 ml de Dichlorométhane, le mélange a été agité à t.a. pendant 10 min puis le Dichlorométhane a été éliminé par filtration. L'opération a été répétée une deuxième fois puis un mélange de 0,2 g de (3-((3-(trifluorométhyl)phényl)éthynyl) imidazo [1,2-a]pyridin-2-yl) méthanamine et 0,055 g (0,596 mmol) d'hydrate d'acide 2-oxoacétique solubilisés dans 3 ml de méthanol a été ajouté. Le mélange a été agité sous vortex lent à t.a. pendant 24 h. La résine a été éliminée par filtration, lavée avec 15 ml de méthanol, 15 ml de Dichlorométhane puis à nouveau 15 ml de méthanol. Le filtrat obtenu a été concentré sous vide et le résidu a été purifié par chromatographie flash sur une cartouche de gel de silice (éluant : Dichlorométhane / méthanol / ammoniaque aqueuse (20% en masse), 80/20/2, v/v/v). 0,049 g (rendement = 22%) d'acide 2-(((3-((3-(trifluorométhyl)phényl)éthynyl) imidazo[1,2-a]pyridin-2-yl)méthyl)amino) acétique ont été obtenus sous forme d'un solide blanc. LC-MS: m/z = 374 (MH⁺) ; pureté UV à 254 nm = 98%. RMN ¹H (300 MHz, DMSO) δ 8.76 (d, J = 6.7 Hz, 1H), 8.13 (s, 1H), 8.01 (d, J = 7.6 Hz, 1H), 7.78 (d, J = 7.9 Hz, 1H), 7.69 (dd, J = 8.3, 7.1 Hz, 2H), 7.53 - 7.42 (m, 1H), 7.16 (td, J = 6.8, 1.0 Hz, 1H), 4.17 (s, 2H), 3.31 (s, 6H).

### Exemple 13: Préparation du dérivé N°69: N-((3-((3-(trifluoro méthyl)phényl)éthynyl)imidazo[1,2-a]pyridin-2-yl)méthyl) propane-2-sulfonamide

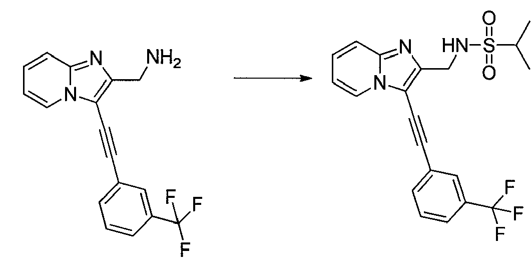

0,08 g (0,239 mmol) de (3-((3-(trifluorométhyl)phényl) éthynyl) imidazo [1,2-a]pyridin-2-yl) méthanamine (préparé selon les étapes 1 à 4 de l'exemple 11) ont été solubilisés dans 2 ml de Dichlorométhane sous agitation magnétique, 0,208 ml (1,193 mmol) de diisopropyléthylamine et 0,083 ml (0,716 mmol) de chlorure d'isopropylsulfonyle ont été ajoutés. Le mélange a été agité à t.a. pendant 16 h. Un supplément de 0,083 ml 0,083 ml (0,716 mmol) de chlorure d'isopropylsulfonyle a été ajouté et le mélange a été agité à t.a. pendant 1 h avant d'être concentré sous vide. Le résidu brut a été directement purifié par chromatographie flash sur une cartouche de gel de silice (éluant : Dichlorométhane / méthanol 95/5, v/v). 0,048 g (rendement = 22%) de N-((3-((3-(trifluorométhyl) phényl)éthynyl) imidazo[1,2-a]pyridin-2-yl)méthyl)propane-2-sulfonamide ont été obtenus sous forme d'un solide blanc. LC-MS: m/z = 422 (MH⁺) ; pureté UV à 254 nm = 98%. RMN ¹H (300 MHz, DMSO) δ 8.75 (d, J = 6.8 Hz, 1H), 8.13 (s, 1H), 7.99 (d, J = 7.5 Hz, 1H), 7.85 - 7.61 (m, 4H), 7.53 - 7.38 (m, 1H), 7.15 (t, J = 6.8 Hz, 1H), 4.44 (d, J = 5.9 Hz, 2H), 3.25 (dd, J = 13.6, 6.8 Hz, 1H), 1.21 (d, J = 6.8 Hz, 6H).

Les dérivés numéro **70** et **71** ont été préparés selon le même protocole.

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **70** | 353,44 | solide | 99 | 354 | | 8.79 (d, *J* = 6.7 Hz, 1H), 7.89 - 7.61 (m, 4H), 7.49 (dd, *J* = 5.1, 1.8 Hz, 3H), 7.33 (s, 1H), 4.49 (d, *J* = 5.8 Hz, 2H), 4.03 (s, 1H), 3.40 - 3.22 (m, 1H), 1.23 (d, *J* = 6.8 Hz, 6H). |
| **71** | 385,38 | | 99 | 386 | | 8.73 (d, *J* = 6.7 Hz, 1H), 8.38 (s, 1H), 8.12 (s, 1H), 7.97 (d, *J* = 7.5 Hz, 1H), 7.82 - 7.58 (m, 3H), 7.52 - 7.37 (m, 1H), 7.13 (t, *J* = 6.7 Hz, 1H), 4.55 (d, *J* = 5.4 Hz, 2H), 2.48 - 2.36 (m, 1H), 0.98 (d, *J* = 6.8 Hz, 6H). |

### Exemple 14 : Préparation du dérivé numéro 72 : 2-(méthyl((3-((3-(trifluorométhyl)phényl)éthynyl)imidazo[1,2-a]pyridin-2-yl)méthyl)amino)acétate de méthyle

### Etape 1: Préparation du 2-((imidazol[1,2-a]pyridin-2-ylméthyl)(méthyl)amino)acétate de méthyle

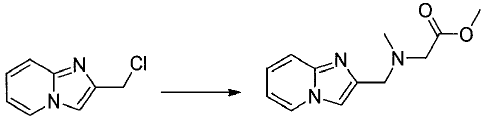

0,303 g (1,773 mmol) de 2-(chlorométhyl)imidazo[1,2-a]pyridine ont été dissous dans 15 ml de diméthylformamide sous agitation magnétique puis 0,253 g (1,773 mmol) d'hydrochlorure de sarcosine méthylester et 0,741 ml (5,32 mmol) de triéthylamine ont été ajoutés. Le mélange a été agité à t.a. pendant 16 h, puis traité avec 30 ml d'une solution aqueuse saturée en NaCl. La phase aqueuse a été concentrée sous vide. Le résidu a été trituré dans 75 ml d'isopropanol, les sels ont été éliminés par filtration et le filtrat a été concentré sous vide. Le résidu brut a été purifié par chromatographie flash sur une cartouche de gel de silice (éluant : Dichlorométhane / méthanol, 95/5, v/v). 0,107 g (rendement = 26%) de 2-((imidazo[1,2-a]pyridin-2-ylméthyl)(méthyl)amino)acétate de méthyle ont été obtenus sous forme d'une huile jaune clair. LC-MS: m/z = 234 (MH⁺) ; pureté UV à 254 nm = 99%. RMN ¹H (300 MHz, DMSO) δ 8.49 (d, *J* = 6.8 Hz, 1H), 7.79 (s, 1H), 7.47 (d, *J =* 9.0 Hz, 1H), 7.18 (ddd, *J =* 9.0, 6.7, 1.2 Hz, 1H), 6.84 (td, *J* = 6.7, 1.1 Hz, 1H), 3.76 (s, 2H), 3.61 (s, 3H), 2.32 (s, 3H).

### Etape 2: Préparation du 2-(((3-iodoimidazo[1,2-a]pyridin-2-yl)méthyl) (méthyl)amino)acétate de méthyle

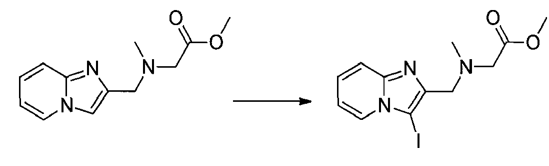

0,107 g (0,459 mmol) de 2-((imidazo[1,2-a]pyridin-2-ylméthyl)(méthyl) amino)acétate de méthyle ont été dissous dans 4 ml d'acétonitrile sous agitation magnétique, à cette solution ont été ajoutés 0,112 g (0,482 mmol) de N-iodosuccinimide. Le mélange a été agité à t.a. pendant 16 h. Le mélange réactionnel a été traité avec 10 ml d'eau puis extrait avec 3 x 10 ml d'acétate d'éthyle. Les phases organiques combinées ont été lavées avec 20 ml d'une solution aqueuse saturée en NaCl, puis séché sur Na₂SO₄ qui a ensuite été éliminé par filtration. Le filtrat obtenu a été concentré sous vide. Le résidu brut a été purifié par chromatographie flash sur une cartouche de gel de silice (éluant : acétate d'éthyle 100%). 0,093 g (rendement = 46%) de 2-(((3-iodoimidazo[1,2-a]pyridin-2-yl)méthyl) (méthyl)amino)acétate de méthyle ont été obtenus sous forme d'une huile jaune. LC-MS: m/z = 360 (MH⁺) ; pureté UV à 254 nm = 82%. RMN ¹H (300 MHz, DMSO) δ 8.30 (d, *J* = 6.8 Hz, 1H), 7.54 (d, *J* = 9.0 Hz, 1H), 7.41 - 7.23 (m, 1H), 7.03 (t, *J* = 6.7 Hz, 1H), 3.79 (s, 2H), 3.62 (s, 3H), 3.37 (s, 2H), 2.34 (s, 3H).

### Etape 3: Préparation du 2-(méthyl((3-((3-(trifluorométhyl) phényl)éthynyl)imidazo[1,2-a]pyridin-2-yl)méthyl)amino)acétate de méthyle (dérivé numéro 72)

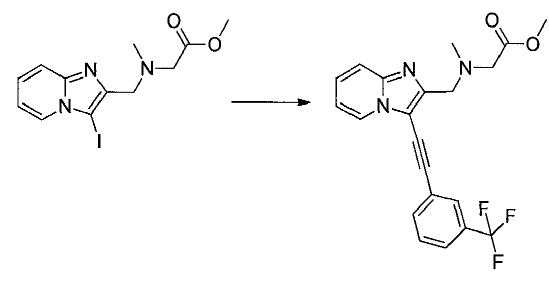

Dans un ballon placé sous un flux d'argon, 0,093 g (0,212 mmol) de 2-(((3-iodoimidazo[1,2-a]pyridin-2-yl)méthyl)(méthyl)amino)acétate de méthyle ont été dissous dans 0,5 ml de diméthylformamide sous agitation magnétique. A cette solution, ont été additionnés : 0,004 g (0,021 mmol) d'iodure de cuivre, 0,037 ml (0,255 mmol) de 3-trifluorométhylphénylacétylène et 0,444 ml (3,18 mmol) de triéthylamine. Le milieu réactionnel a été dégazé à l'argon pendant 10 min avant d'ajouter 0,019 g (0,021 mmol) de Pd₂(dba)₃. Le mélange a été agité à t.a. pendant 16 h avant d'être traité dilué avec 15 ml d'acétate d'éthyle et lavé avec 2 x 15 ml d'une solution aqueuse saturée en NaCl. La phase aqueuse a été extraite avec 20 ml d'acétate d'éthyle. Les phases organiques combinées ont été séchées sur Na₂SO₄ qui a ensuite été éliminé par filtration. Le filtrat obtenu a été concentré sous vide. Le résidu brut a été purifié par chromatographie flash sur une cartouche de gel de silice (éluant : acétate d'éthyle 100%). 0,05 g (rendement = 57%) de 2-(méthyl((3-((3-(trifluorométhyl)phényl)éthynyl) imidazo[1,2-a]pyridin-2-yl) méthyl)amino)acétate de méthyle ont été obtenus sous forme d'un solide marron clair. LC-MS: m/z = 402 (MH⁺) ; pureté UV à 254 nm = 96%. RMN ¹H (300 MHz, DMSO) δ 8.75 (d, *J* = 6.7 Hz, 1H), 8.11 (s, 1H), 7.97 (d, *J* = 7.6 Hz, 1H), 7.83 - 7.59 (m, 3H), 7.55 - 7.37 (m, 1H), 7.14 (td, *J* = 6.8, 0.9 Hz, 1H), 3.97 (s, 2H), 3.58 (s, 3H), 3.43 (s, 2H), 2.39 (s, 3H).

Les dérivés numéro **101** à **104** ont été préparés selon le même protocole.

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254** | **MH⁺** | **M-H⁺** | |
| **101** | 451,13 | Solide jaune | 99 | 416 | | 8.87 (d, J = 6.7 Hz, 1H), 8.20 (s, 1H), 8.08 (d, J = 7.5 Hz, 1H), 7.79 (dt, J = 15.6, 8.4 Hz, 3H), 7.65 - 7.53 (m, 1H), 7.26 (t, J = 6.7 Hz, 1H), 4.69 (s, 2H), 4.32 (s, 2H), 4.23 - 4.16 (m, 4H), 2.97 (s, 3H), 1.20 (t, J = 7.1 Hz, 3H). |
| **102** | 449,85 | Solide beige | | 450 | | 8.95 (s, 1H), 8.14 (s, 1H), 8.00 (d, J = 7.5 Hz, 1H), 7.73 (dt, J = 15.5, 7.8 Hz, 3H), 7.48 (d, J = 8.3 Hz, 1H), 4.04 (d, J = 7.0 Hz, 2H), 3.96 (s, 2H), 3.39 (s, 2H), 2.38 (s, 3H), 1.13 (t, J = 7.0 Hz, 3H). |
| **103** | 431,41 | Solide jaune | 99 | 432 | | 8.83 (d, J = 6.7 Hz, 1H), 7.88 - 7.77 (m, 3H), 7.59 (ddd, J = 27.9, 17.8, 8.1 Hz, 3H), 7.26 (t, J = 6.7 Hz, 1H), 4.68 (s, 2H), 4.32 (s, 2H), 4.20 (d, J = 7.1 Hz, 2H), 2.97 (s, 3H), 1.21 (t, J = 7.1 Hz, 3H). |
| **104** | 465,85 | solide marron | 99 | 466 | | 9.08 (s, 1H), 7.85 (dd, J = 13.6, 6.0 Hz, 3H), 7.69 - 7.60 (m, 2H), 7.50 (d, J = 8.3 Hz, 1H), 4.64 (s, 2H), 4.28 (s, 2H), 4.19 (dd, J = 14.1, 7.0 Hz, 2H), 2.93 (s, 3H), 1.20 (t, J = 7.1 Hz, 3H). |

### Exemple 15: Préparation du dérivé numéro 73 : acide 2-(méthyl((3-((3-(trifluorométhyl)phényl)éthynyl)imidazo[1,2-a]pyridin-2-yl)méthyl)amino)acétique

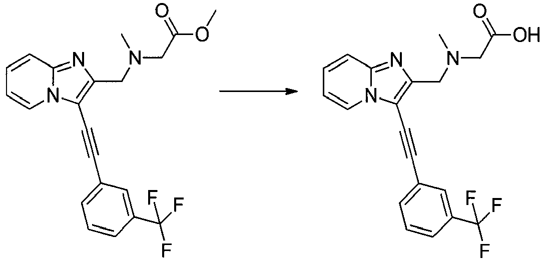

0,22 g (0,543 mmol) de 2-(méthyl((3-((3-(trifluorométhyl)phényl) éthynyl) imidazo[1,2-a]pyridin-2-yl)méthyl)amino)acétate ont été dissous dans 6 ml d'un mélange composé de méthanol et de tétrahydrofurane sous agitation magnétique puis 0,543 ml (0,543 mmol) d'une solution aqueuse de NaOH 1M ont été ajoutés. Le mélange a été agité à t.a. pendant 16 h. 0,163 ml (0,163 mmol) d'une solution aqueuse de NaOH 1M ont été ajoutés. Le mélange a été agité à t.a. pendant 24 h supplémentaires avant d'être traité par une solution de 0,04 ml (0,705 mmol) d'acide acétique dans 10 ml d'eau. Le mélange a été agité à t.a. pendant 30 min. Le solide formé a été isolé par filtration, lavé avec 5 ml d'eau et séché sous cloche à vide pour donner 0,155 g (rendement = 73%) d'acide 2-(méthyl((3-((3-(trifluorométhyl)phényl) éthynyl)imidazo [1,2-a]pyridin-2-yl)méthyl)amino) acétique sous forme d'un solide beige. LC-MS: m/z = 388 (MH⁺) ; pureté UV à 254 nm = 99%. RMN ¹H (300 MHz, DMSO) δ 8.74 (d, J = 6.7 Hz, 1H), 8.10 (s, 1H), 7.97 (d, J = 7.6 Hz, 1H), 7.77 (d, J = 7.8 Hz, 1H), 7.74 - 7.64 (m, 2H), 7.49 - 7.39 (m, 1H), 7.13 (t, J = 6.4 Hz, 1H), 4.02 (s, 2H), 3.59 (s,1H), 3.33 (s, 2H), 2.41 (s, 3H).

Les dérivés numéro **105** à **107** ont été préparés selon le même protocole.

| **No** | **Poids moléculaire** | **Apparence** | **Pureté LCMS** | **Spectrométrie de masse m/z** | | **RMN 1H (300 MHz, DMSO) δ** |
|---|---|---|---|---|---|---|
| | **g/mol** | | **UV à 254** | **MH⁺** | **M-H⁺** | |
| **105** | 437,8 | solide vert | 99 | 438 | | 9.08 (s, 1H), 7.91 - 7.80 (m, 3H), 7.63 (s, 2H), 7.50 (d, J = 7.2 Hz, 1H), 4.65 (s, 2H), 4.19 (s, 2H), 2.92 (s, 1H), 1.23 (s, 3H). |
| **106** | 403,36 | Solide marron | 99 | 404 | | 8.79 (d, J = 5.9 Hz, 1H), 7.88 - 7.71 (m, 3H), 7.63 (t, J = 7.8 Hz, 1H), 7.56 - 7.42 (m, 2H), 7.22 (d, J = 6.3 Hz, 1H), 4.48 (s, 2H), 3.97 (s, 2H), 2.79 (s, 1H), 1.03 (d, J = 5.8 Hz, 3H). |
| **107** | 421,8 | Solide marron | 99 | 422 | | 8.96 (s, 1H), 8.15 (s, 1H), 8.01 (s, 1H), 7.74 (dd, J = 24.9, 7.0 Hz, 3H), 7.52 (s, 1H), 3.94 (s, 2H), 3.03 (s, 2H), 2.33 (s, 3H). |

### Exemple 16: Préparation du dérivé numéro 74: 3-(3-((3-((3-(trifluorométhyl)phényl)éthynyl)imidazo[1,2-a]pyridin-2-yl)méthyl)ureido)benzoate de méthyle

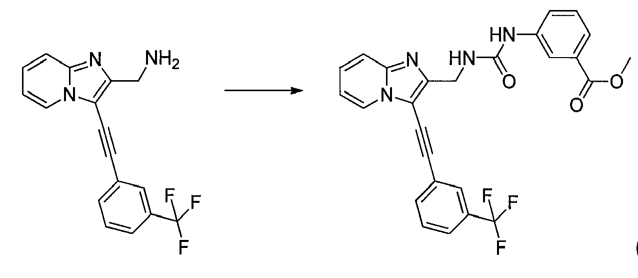

0,15 g (0,477 mmol) de (3-((3-(trifluorométhyl)phényl)éthynyl)imidazo [1,2-a]pyridin-2-yl)méthanamine (préparé selon les étapes 1 à 4 de l'exemple 11) ont été dissous dans 4 ml de Dichlorométhane sous agitation magnétique puis 0,09 g (0,492 mmol) de benzoate-3-isocyanate de méthyle. Le mélange a été agité à t.a. pendant 15 min. Le solide formé a été isolé par filtration et lavé avec 10 ml de Dichlorométhane. Le solide a ensuite été purifié par HPLC préparative pour donner 0,057 g (rendement = 25%) de 3-(3-((3-((3-(trifluorométhyl)phényl)éthynyl)imidazo[1,2-a]pyridin-2-yl)méthyl) ureido)benzoate de méthyle sous forme d'une poudre blanche. LC-MS: m/z = 493 (MH⁺) ; pureté UV à 254 nm = 98%. RMN ¹H (300 MHz, DMSO) δ ), 8.74 (d, *J* = 6.7 Hz, 1H), 8.11 (t, *J* = 1.8 Hz, 1H), 8.04 (s, 1H), 7.91 (d, *J* = 7.8 Hz, 1H), 7.70 (dd, *J* = 13.0, 8.5 Hz, 2H), 7.58 (t, *J* = 7.9 Hz, 2H), 7.53 - 7.42 (m, 2H), 7.34 (t, *J* = 7.9 Hz, 1H), 7.15 (td, *J* = 6.8, 1.1 Hz, 1H), 6.81 (t, *J* = 5.7 Hz, 1H), 4.64 (d, *J* = 5.7 Hz, 2H), 3.83 (s, 3H).

### Exemple 17 : Préparation du dérivé numéro 75 : acide 3-(3-((3-((3-(trifluorométhyl)phényl)éthynyl)imidazo[1,2-a]pyridin-2-yl)méthyl)ureido)benzoïque

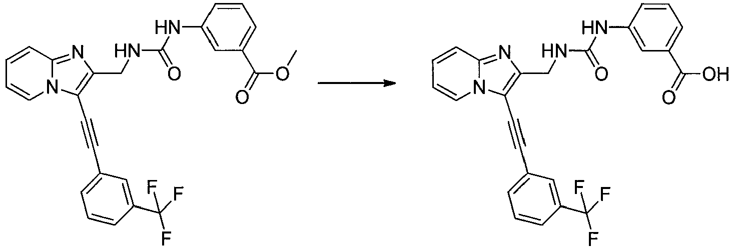

0,038 g (0,078 mmol) de 3-(3-((3-((3-(trifluorométhyl)phényl)éthynyl) imidazo [1,2-a]pyridin-2-yl)méthyl)ureido)benzoate de méthyle ont été dissous dans 2 ml d'un mélange méthanol / tétrahydrofurane (1/1, v/v) sous agitation magnétique puis 0,931 ml (0,931 mmol) d'une solution aqueuse de NaOH (1 M) ont été ajoutés et le mélange a été agité à t.a. pendant 16 h avant d'être traité par une solution de 0,053 ml (0,931 mmol) d'acide acétique dans 10 ml d'eau. Le mélange a été agité à t.a. pendant 30 min. Le solide formé a été isolé par filtration, lavé avec 5 ml d'eau et séché sous cloche à vide pour donner 0,026 g (rendement = 70%) d'acide 3-(3-((3-((3-(trifluorométhyl)phényl)éthynyl)imidazo[1,2-a]pyridin-2-yl)méthyl) ureido)benzoïque sous forme d'un solide blanc. LC-MS: m/z = 479 (MH⁺) ; pureté UV à 254 nm = 99%. RMN ¹H (300 MHz, DMSO) δ 12.83 (s, 1H), 8.96 (s, 1H), 8.73 (d, J = 6.7 Hz, 1H), 8.04 (s, 2H), 7.90 (d, J = 7.5 Hz, 1H), 7.69 (dd, J = 13.0, 8.4 Hz, 2H), 7.58 (d, J = 7.7 Hz, 2H), 7.45 (d, J = 7.5 Hz, 2H), 7.29 (t, J = 7.9 Hz, 1H), 7.13 (t, J = 6.8 Hz, 1H), 6.80 (t, J = 5.4 Hz, 1H), 4.62 (d, J = 5.6 Hz, 2H).

### Exemple 18 : Préparation du dérivé N°76: 2-((3-((4-fluoro phényl)éthynyl)imidazo[1,2-a]pyridin-2-yl)méthoxy)benzoate de méthyle

### Etape 1: Préparation du 2-((3-iodoimidazo[,2-a]pyridin-2-yl)méthoxy)benzoate de méthyle

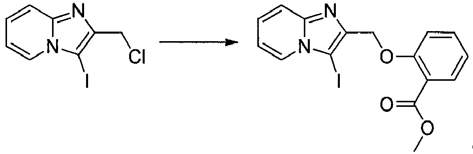

4 g (13,68 mmol) de 2-(chlorométhyl)-3-iodoimidazo[1,2-a]pyridine ont été dissous dans le diméthylformamide sous agitation magnétique puis 2,71 ml (20,51 mmol) de 2-hydroxybenzoate de méthyle et 6,68 g (20,51 mmol) de carbonate de césium ont été ajoutés. Le mélange a été agité à 60°C pendant 2 h avant d'être versé dans 200 ml d'eau. La phase aqueuse a été extraite avec 2 x 100 ml d'acétate d'éthyle. Les phases organiques combinées ont été lavées avec 100 ml d'une solution aqueuse saturée en NaHCO₃, 100 ml d'une solution aqueuse saturée en NaCl, séchées sur Na₂SO₄ qui a ensuite été éliminé par filtration. Le filtrat obtenu a été concentré sous vide. Le résidu brut a été trituré dans 50 ml d'éther diisopropylique, le solide obtenu a été isolé par filtration. 2,55 g de produit attendu ont été isolés. D'autre part, une suspension en phase aqueuse a été isolée par filtration et lavée avec 2 x 30 ml d'eau. Les analyses ont confirmé qu'il s'agissait également du produit attendu. Les deux lots ont donc été rassemblés et 5,58 g (rendement = 76%) de 2-((3-iodoimidazo [1,2-a]pyridin-2-yl) méthoxy)benzoate de méthyle ont été obtenus sous forme d'une poudre blanche. LC-MS: m/z = 409 (MH⁺) ; pureté UV à 254 nm = 98%. RMN ¹H (300 MHz, DMSO) δ 8.33 (d, *J* = 6.9 Hz, 1H), 7.69 - 7.44 (m, 3H), 7.45 - 7.28 (m, 2H), 7.16 - 6.93 (m, 2H), 5.23 (s, 2H), 3.73 (s, 3H).

### Etape 2 : Préparation du 2-((3-((4-fluorophényl)éthynyl)imidazo [1,2-a]pyridin-2-yl)méthoxy)benzoate de méthyle (dérivé N° 76)

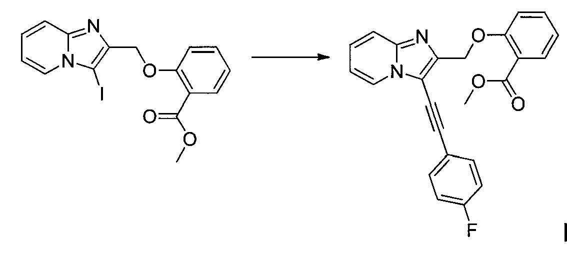

Dans un ballon placé sous un flux d'argon, 2 g (3,82 mmol) de 2-((3-iodoimidazo[1,2-a]pyridin-2-yl)méthoxy)benzoate de méthyle ont été dissous dans 8 ml de diméthylformamide sous agitation magnétique. A cette solution, ont été additionnés : 0,074 g (0,382 mmol) d'iodure de cuivre, 0,551 g (4,59 mmol) de 1-éthynyl-4-fluoronenzène et 8 ml (57,3 mmol) de triéthylamine. Le milieu réactionnel a été dégazé à l'argon pendant 10 min avant d'ajouter 0,35 g (0,382 mmol) de Pd₂(dba)₃. Le mélange a été agité à t.a. pendant 16 h avant d'être traité par 100 ml d'une solution aqueuse saturée en NaCl. La phase aqueuse a été extraite avec 2 x 100 ml d'acétate d'éthyle. Les phases organiques combinées ont été lavées avec 100 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : heptane / acétate d'éthyle, 1/1, v/v). 1,36 g (rendement = 84%) de 2-((3-((4-fluorophényl)éthynyl) imidazo[1,2-a]pyridin-2-yl)méthoxy)benzoate de méthyle ont été obtenus sous forme d'un solide marron clair. LC-MS: m/z = 401 (MH⁺) ; pureté UV à 254 nm = 98%. RMN ¹H (300 MHz, DMSO) δ 8.63 (d, J = 6.7 Hz, 1H), 7.75 - 7.59 (m, 4H), 7.48 (ddd, J = 15.9, 11.5, 4.8 Hz, 2H), 7.40 - 7.26 (m, 3H), 7.13 (t, J = 6.8 Hz, 1H), 7.01 (t, J = 7.4 Hz, 1H), 5.40 (s, 2H), 3.68 (s, 3H).

Les dérivés numéro **77** à **86** ont été préparés selon le même enchaînement d'étapes 1 et 2.

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **77** | 450,41 | solide marron | 99 | 451 | | 8.77 (d, *J =* 6.7 Hz, 1H), 8.03 (s, 1H), 7.91 (d, *J =* 7.6 Hz, 1H), 7.83 - 7.59 (m, 4H), 7.57 - 7.44 (m, 2H), 7.36 (d, *J* = 8.4 Hz, 1H), 7.15 (dd, *J* = 14.3, 7.5 Hz, 1H), 7.02 (t, *J* = 7.5 Hz, 1H), 5.46 (s, 2H), 3.68 (s, 3H). |
| **78** | 382.,11 | solide jaune | 95 | 383 | | 1H NMR (DMSO-D6): 8.63 (d, *J =* 6.8 Hz, 1H), 7.79 - 7.57 (m, 4H), 7.57 - 7.35 (m, 6H), 7.14 (tt, *J =* 10.1, 5.0 Hz, 1H), 7.02 (td, *J =* 7.6, 0.9 Hz, 1H), 5.41 (s, 2H), 3.68 (s, 3H). |
| **79** | 407,21 | solide | 92 | 408 | | 8.73 (d, *J* = 6.7 Hz, 1H), 8.15 (s, 1H), 8.00 - 7.82 (m, 2H), 7.79 - 7.59 (m, 3H), 7.50 (td, *J* = 8.1, 3.9 Hz, 2H), 7.36 (d, *J =* 8.3 Hz, 1H), 7.16 (t, *J =* 6.8 Hz, 1H), 7.02 (t, *J =* 7.5 Hz, 1H), 5.44 (s, 2H), 3.69 (s, 3H). |
| **80** | 381,40 | huile marron | 93,1 | 382 | | 8.52 (d, J = 6.8 Hz, 1H), 7.69 (s, 1H), 7.62 - 7.52 (m, 5H), 7.45 - 7.34 (m, 2H), 7.22 (d, J = 8.9 Hz, 2H), 7.02 (t, J = 6.8 Hz, 1H), 4.68 (s, 2H), 4.58 (s, 2H). |
| **81** | 450,41 | solide marron | 99 | 451 | | 8.81 - 8.71 (m, 1H), 8.04 (s, 1H), 7.94 (d, J = 7.7 Hz, 1H), 7.84 - 7.61 (m, 4H), 7.58 - 7.30 (m, 4H), 7.16 (td, J = 6.8, 1.1 Hz, 1H), 5.45 (s, 2H), 3.78 (s, 3H). |
| **82** | 382,41 | solide marron | 95 | 383 | | 8.64 (d, *J* = 6.8 Hz, 1H), 7.97 - 7.85 (m, 2H), 7.75 - 7.59 (m, 3H), 7.52 - 7.39 (m, 4H), 7.26 - 7.10 (m, 3H), 5.42 (s, 2H), 3.79 (s, 3H). |
| **83** | 450,41 | solide marron | 96 | 451 | | 8.78 (d, *J* = 6.7 Hz, 1H), 8.04 (s, 1H), 7.92 (t, *J* = 8.1 Hz, 3H), 7.73 (dt, *J* = 13.9, 7.8 Hz, 3H), 7.56 - 7.41 (m, 1H), 7.27 - 7.09 (m, 3H), 5.47 (s, 2H), 3.80 (s, 3H). |
| **84** | 324,38 | solide orangé | 99 | 325 | | 8.64 (dd, J = 6.8, 1.0 Hz, 1H), 7.77 - 7.59 (m, 3H), 7.53 - 7.40 (m, 4H), 7.30 (ddd, *J* = 7.5, 5.8, 2.2 Hz, 2H), 7.21 - 7.04 (m, 3H), 6.95 (t, *J* = 7.3 Hz, 1H), 5.33 (s, 2H). |
| **85** | 367,38 | solide | 97 | 368 | | 8.66 (d, *J* = 6.8 Hz, 1H), 7.79 - 7.68 (m, 3H), 7.63 (s, 1H), 7.55 - 7.40 (m, 4H), 7.33 (t, *J* = 8.9 Hz, 2H), 7.15 (td, *J* = 6.8, 1.0 Hz, 1H), 5.42 (s, 2H). |
| **86** | 431,41 | solide beige | >99 | 432 | | 8.75 (d, J = 5.9 Hz, 1H), 7.97 (s, 1H), 7.91 - 7.62 (m, 7H), 7.58 - 7.40 (m, 2H), 7.15 (t, J = 6.8 Hz, 1H), 4.82 (s, 2H), 4.70 (s, 2H). |

### Exemple 19: Préparation du dérivé numéro 87: 3-((3-(phényléthynyl)imidazo[1,2-a]pyridin-2-yl)méthoxy)benzoate de méthyle

### Etape 1: Préparation de l'imidazo[1,2-a]pyridine-2-carboxylate d'éthyle

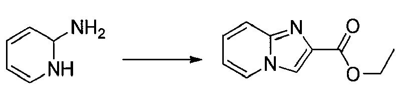

3 g (31,9 mmol) de 2-aminopyridine ont été dissous dans 65 ml de tétrahydrofurane sous agitation magnétique. L'ajout de 4,44 ml (31,9 mmol) de bromopyruvate d'éthyle a provoqué la précipitation d'un solide. Le mélange hétérogène a été agité à reflux pendant 4 h. Après retour à t.a., le solide a été isolé par filtration et repris dans 65 ml d'éthanol pour être agité à reflux dans 65 ml d'éthanol pendant 16 h. Après retour à t.a., le solide a été isolé par filtration. Le filtrat a été placé dans la glace afin de faciliter la cristallisation du produit qui a été collecté par filtration et ainsi de suite de suite jusqu'à ce que la phase liquide reste limpide. Le solide a été rincé avec 30 ml d'éther diisopropylique et séché sous cloche à vide pour donner 5,14 g (rendement = 84%) d'imidazo[1,2-a]pyridine-2-carboxylate d'éthyle sous forme d'une poudre blanche. LC-MS: m/z = 191 (MH⁺) ; pureté UV à 254 nm = 99%. RMN ¹H (300 MHz, DMSO) δ 8.95 (s, 1H), 8.84 (d, J = 6.8 Hz, 1H), 7.98 - 7.80 (m, 2H), 7.46 (t, J = 7.2 Hz, 1H), 4.43 (q, J = 7.1 Hz, 2H), 1.35 (d, J = 7.1 Hz, 3H).

### Etape 2: Préparation du 3-iodoimidazo[1,2-a]pyridine-2-carboxylate d'éthyle

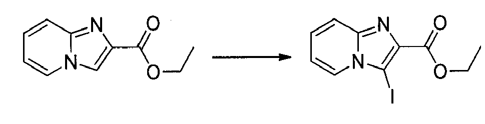

2,405 g (12,16 mmol) d'imidazo[1,2-a]pyridine-2-carboxylate d'éthyle ont été dissous dans 60 ml d'acétonitrile sous agitation magnétique, à cette solution ont été ajoutés 3,01 g (13,38 mmol) de N-iodosuccinimide. Le mélange a été agité à t.a. pendant 1 h. Le mélange réactionnel a été traité avec 50 ml d'une solution aqueuse de Na₂S₂O₅ à 100 mg/ml. La phase aqueuse a été extraite avec 2 x 100 ml d'acétate d'éthyle. La phase aqueuse a été neutralisée par addition de NaHCO₃ par portions puis extraite avec 3 x 100 ml d'acétate d'éthyle. L'ensemble des phases organiques a été combiné puis lavé avec 200 ml d'une solution aqueuse saturée en NaCl, puis séché sur Na₂SO₄ qui a ensuite été éliminé par filtration. Le filtrat obtenu a été concentré sous vide pour donner 1,59 g (rendement = 41%) de 3-iodoimidazo[1,2-a]pyridine-2-carboxylate d'éthyle sous forme d'un solide jaune pâle. LC-MS: m/z = 317 (MH⁺) ; pureté UV à 254 nm = 99%. RMN ¹H (300 MHz, DMSO) δ 8.45 (d, J = 6.9 Hz, 1H), 7.66 (d, J = 9.1 Hz, 1H), 7.46 (d, J = 7.0 Hz, 1H), 7.15 (d, J = 6.6 Hz, 1H), 4.30 (d, J = 7.1 Hz, 2H), 1.36 (d, J = 7.1 Hz, 3H).

### Etape 3: Préparation du 3-(phényléthynyl)imidazo[1,2-a]pyridine-2-carboxylate d'éthyle

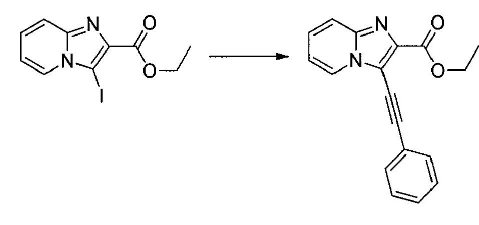

Dans un ballon placé sous un flux d'argon, 1,82 g (5,77 mmol) de 3-iodoimidazo[1,2-a]pyridine-2-carboxylate d'éthyle ont été dissous dans 6 ml de diméthylformamide sous agitation magnétique. A cette solution, ont été additionnés : 0,11 g (0,577 mmol) d'iodure de cuivre, 0,776 ml (6,93 mmol) de phénylacétylène et 6 ml (43 mmol) de triéthylamine. Le milieu réactionnel a été dégazé à l'argon pendant 10 min avant d'ajouter 0,529 g (0,577 mmol) de Pd₂(dba)₃. Le mélange a été agité à t.a. pendant 16 h avant d'être filtré sur célite. La célite a été lavée avec 50 ml d'acétate d'éthyle, puis le filtrat a été lavé avec 2 x 50 ml d'eau. Après séparation, la phase aqueuse a été extraite avec 2 x 50 ml d'acétate d'éthyle, les phases organiques combinées ont été lavées avec 70 ml d'une solution aqueuse saturée en NaCl, séchées sur Na₂SO₄ qui a ensuite été éliminé par filtration. Le filtrat obtenu a été concentré sous vide. Le résidu brut a été purifié par chromatographie flash sur une cartouche de gel de silice (éluant : heptane / acétate d'éthyle, 6/4, v/v). 1,28 g (rendement = 57%) de 3-(phényléthynyl)imidazo[1,2-a]pyridine-2-carboxylate d'éthyle ont été obtenus sous forme d'un solide jaune clair. LC-MS: m/z = 291 (MH⁺) ; pureté UV à 254 nm = 93%. RMN ¹H (300 MHz, DMSO) δ 8.70 (d, J = 6.8 Hz, 1H), 7.74 (dd, *J* = 22.1, 6.2 Hz, 3H), 7.53 (dd, *J* = 17.6, 6.9 Hz, 4H), 7.23 (t, *J* = 6.3 Hz, 1H), 4.36 (d, *J* = 4.7 Hz, 2H), 1.36 (d, *J* = 7.1 Hz, 3H).

### Etape 4 : Préparation du (3-(phényléthynyl)imidazo[1,2-a]pyridin-2-yl)méthanol

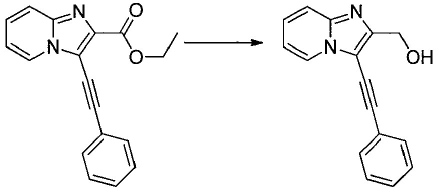

Dans un ballon placé sous un flux d'argon, 0,078 g (1,984 mmol) de LAH ont été dissous dans 1,4 ml de tétrahydrofurane puis sous une agitation magnétique vigoureuse, une solution de 0,3 g (0,992 mmol) de 3-(phényléthynyl)imidazo[1,2-a]pyridine-2-carboxylate d'éthyle dans 3,3 ml de tétrahydrofurane a été ajoutée goutte à goutte. Le mélange a été agité à t.a. pendant 15 min puis 0,2 ml d'eau ont été ajouté précautionneusement, suivis d'une solution de 0,2 ml d'acide sulfurique dans 1,5 ml d'eau. Après séparation, la phase organique a été lavée avec 50 ml d'eau. La phase aqueuse a été extraite avec 3 x 50 ml d'acétate d'éthyle puis les phases organiques combinées ont été lavées avec 100 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur une cartouche de gel de silice (éluant : gradient d'heptane / acétate d'éthyle, de 40% à 30% d'heptane, v/v). 0,076 g (rendement = 30%) de (3-(phényléthynyl)imidazo[1,2-a]pyridin-2-yl)méthanol ont été obtenus sous forme d'un solide jaune pâle. LC-MS: m/z = 249 (MH⁺) ; pureté UV à 254 nm = 97%. RMN ¹H (300 MHz, DMSO) δ 8.59 (dt, J = 6.7, 1.1 Hz, 1H), 7.77 - 7.56 (m, 3H), 7.56 - 7.32 (m, 4H), 7.11 (td, *J*= 6.8, 1.1 Hz, 1H), 5.33 (t, *J*= 5.9 Hz, 1H), 4.67 (d, *J* = 5.9 Hz, 2H).

### Etape 5: Préparation du 3-((3-(phényléthynyl)imidazo[1,2-a]pyridin-2-yl)méthoxy) benzoate de méthyle (dérivé N°87)

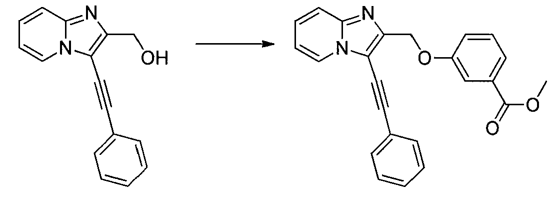

0,09 g (0,594 mmol) de 3-hydroxybenzoate de méthyle ont été dissous dans 1 ml de tétrahydrofurane sous agitation magnétique puis 0,312 g (1,188 mmol) de triphénylphosphine et 0,231 ml (1,188 mmol) de DIAD ont été ajoutés. Le mélange a été agité à t.a. pendant 15 min puis une solution de 0,076 g (0,297 mmol) de (3-(phényléthynyl)imidazo[1,2-a]pyridin-2-yl)méthanol dans 4 ml de tétrahydrofurane a été ajoutée et le mélange a été agité à t.a. pendant 16 h. Un mélange de 0,09 g (0,594 mmol) de 3-hydroxybenzoate de méthyle, 0,312 g (1,188 mmol) de triphénylphosphine et 0,231 ml (1,188 mmol) de DIAD dissous dans 1 ml de tétrahydrofurane a été additionné et le milieu réactionnel a été agité à t.a. pendant 2 h supplémentaires. Le milieu réactionnel a été traité avec 10 ml. La phase aqueuse a été extraite avec 3 x 10 ml d'acétate d'éthyle puis les phases organiques combinées ont été lavées avec 10 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur une cartouche de gel de silice (éluant : gradient d'heptane / acétate d'éthyle, de 70% à 40% d'heptane, v/v). 0,048 g (rendement = 42%) de 3-((3-(phényléthynyl)imidazo[1,2-a]pyridin-2-yl)méthoxy) benzoate de méthyle ont été obtenus sous forme d'un solide jaune pâle. LC-MS: m/z = 383 (MH⁺) ; pureté UV à 254 nm = 99%. RMN ¹H (300 MHz, DMSO) δ 8.64 (d, J = 6.8 Hz, 1H), 7.68 (dd, J = 17.0, 7.2 Hz, 4H), 7.55 (d, J = 8.7 Hz, 1H), 7.52 - 7.34 (m, 6H), 7.15 (t, J = 7.3 Hz, 1H), 5.42 (s, 2H), 3.80 (s, 3H).

### Exemple 20 : Préparation du dérivé N°88 : acide 2-((3-((4-fluoro phényl)éthynyl)imidazo[1,2-a]pyridin-2-yl)méthoxy)benzoïque

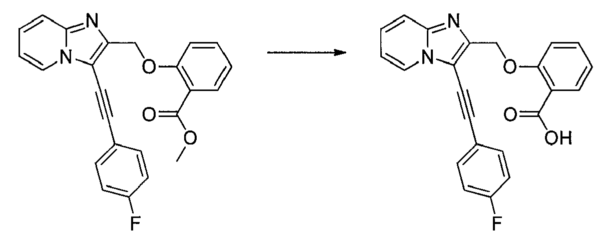

0,65 g (1,542 mmol) de 2-((3-((4-fluorophényl)éthynyl)imidazo[1,2-a] pyridin-2-yl)méthoxy) benzoate de méthyle ont été solubilisés sous agitation magnétique dans 30 ml d'un mélange composé d'éthanol et de tétrahydrofurane (1/1, v/v) puis 15,42 ml (15,42 mmol) d'une solution aqueuse de NaOH 1N ont été ajoutés. Le mélange a été agité à t.a. pendant 16 h avant d'ajouter 20 ml d'eau puis 0,883 ml (15,42 mmol) d'acide acétique. Le précipité formé a été isolé par filtration, lavé avec 10 ml d'eau et séché sous cloche à vide pour donner 0,518 g (rendement = 81%) d'acide 2-((3-((4-fluorophényl)éthynyl)imidazo[1,2-a]pyridin-2-yl) méthoxy) benzoïque sous forme d'un solide jaune pâle. LC-MS: m/z = 387 (MH⁺) ; pureté UV à 254 nm = 99%. RMN ¹H (300 MHz, DMSO) δ 12.72 (s, 1H), 8.64 (d, J = 5.5 Hz, 1H), 7.78 - 7.59 (m, 4H), 7.45 (d, J = 6.2 Hz, 2H), 7.41 - 7.24 (m, 3H), 7.14 (t, J = 6.8 Hz, 1H), 7.00 (t, J = 7.3 Hz, 1H), 5.42 (s, 2H).

Les dérivés numéros **89** à **93** ont été préparés selon le même protocole.

| **No** | **Poids moléculaire g/mol** | **Apparence** | **Pureté LCMSUV à 254 nm** | **Spectrométrie de masse m/z** | | **RMN 1H (300 MHz, DMSO) δ** |
|---|---|---|---|---|---|---|
| | | | | **MH⁺** | **M-H⁺** | |
| **89** | 436,38 | solide | 99 | 437 | 435 | 12.71 (s, 1H), 8.76 (d, *J* = 6.5 Hz, 1H), 8.05 (s, 1H), 7.93 (d, *J* = 7.6 Hz, 1H), 7.82 - 7.58 (m, 5H), 7.48 (dd, *J* = 13.1, 6.6 Hz, 3H), 7.36 (d, *J* = 8.3 Hz, 1H), 7.17 (t, *J* = 6.7 Hz, 1H), 7.01 (t, *J* = 7.4 Hz, 1H), 5.47 (s, 2H). |
| **90** | 436,38 | solide marron | 99 | 437 | | 12.66 (s, 1H), 8.78 (d, *J* = 6.8 Hz, 1H), 8.05 (s, 1H), 8.01 - 7.84 (m, 3H), 7.84 - 7.60 (m, 3H), 7.57 - 7.42 (m, 1H), 7.30 - 7.05 (m, 3H), 5.45 (s, 2H). |
| **91** | 390,37 | solide marron | 98 | 369 | | 8.63 (d, *J* = 6.8 Hz, 1H), 7.79 (d, *J* = 8.7 Hz, 2H), 7.65 (s, 3H), 7.45 (s, 4H), 7.15 (td, *J* = 6.8, 1.0 Hz, 1H), 6.96 (d, *J* = 8.8 Hz, 2H), 5.31 (s, 2H). |
| **92** | 390,37 | solide jaune | 98 | 369 | | 8.64 (d, *J =* 6.7 Hz, 1H), 7.85 - 7.62 (m, 3H), 7.56 - 7.37 (m, 4H), 7.29 (t, *J* = 7.7 Hz, 1H), 7.23 - 6.98 (m, 3H), 6.94 - 6.72 (m, 1H), 5.28 (s, 2H). |
| **93** | 368,39 | solide jaune | 98,6 | 369 | | 13.02 (s, 1H), 8.64 (d, *J* = 6.8 Hz, 1H), 7.76 - 7.59 (m, 4H), 7.59 - 7.32 (m, 7H), 7.15 (t, *J* = 6.3 Hz, 1H), 5.41 (s, 2H). |

### Exemple 21 : Préparation du dérivé N°94: (2-((3-((3-(trifluoro méthyl)phényl)éthynyl)imidazo[1,2-a]pyridin-2-yl)méthoxy) phényl)méthanol

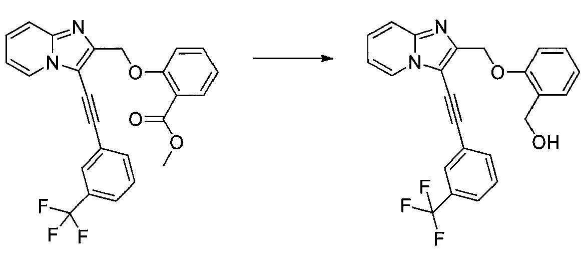

Dans un ballon placé sous un flux d'argon, 0,16 g (0,352 mmol) de 2-((3-((3-(trifluorométhyl)phényl)éthynyl)imidazo[1,2-a]pyridin-2-yl)méthoxy) benzoate de méthyle ont été dissous dans 4 ml de tétrahydrofurane sous agitation magnétique, le mélange a été refroidi à 0°C puis 0,148 ml (0,492 mmol) de RED-AI ont été ajoutés. Le mélange a été agité à 0°C pendant 15 min avant d'être dilué avec 15 ml d'acétate d'éthyle et versé dans 20 ml d'eau. Sous agitation vigoureuse, 1 ml de d'une solution aqueuse de NaOH (32% en masse) a été ajouté lentement. Après séparation la phase organique a été lavée avec 2 x 10 ml d'eau. Les phases aqueuses ont été extraites avec 2 x 5 ml d'acétate d'éthyle. Les phases organiques combinées ont été lavées avec 20 ml d'une solution aqueuses saturée en NaCl puis séchées sur Na₂SO₄ qui a ensuite été éliminé par filtration. Le filtrat obtenu a été concentré sous vide. Le résidu brut a été purifié par trituration dans 10 ml d'éther diisopropylique. Le solide a été isolé par filtration et séché sous vide. 0,091 g (rendement = 58%) de (2-((3-((3-(trifluorométhyl)phényl)éthynyl)imidazo[1,2-a]pyridin-2-yl)méthoxy) phényl) méthanol ont été obtenus sous forme d'un solide marron clair. LC-MS: m/z = 425 (M+2H⁺); pureté UV à 254 nm = 94%. RMN ¹H (300 MHz, DMSO) δ 8.77 (d, J = 6.8 Hz, 1H), 8.05 (s, 1H), 7.92 (d, J = 7.6 Hz, 1H), 7.73 (dt, J = 14.3, 7.8 Hz, 3H), 7.55 - 7.43 (m, 1H), 7.38 (d, J = 7.4 Hz, 1H), 7.20 (d, J = 3.6 Hz, 3H), 7.03 - 6.86 (m, 1H), 5.39 (s, 2H), 4.98 (t, J = 5.7 Hz, 1H), 4.55 (d, J = 5.6 Hz, 2H).

### Exemple 22: Préparation du dérivé numéro 95: N-(2-(diméthylamino)éthyl)-2-((3-((4-fluorophényl)éthynyl) imidazo [1,2-a]pyridin-2-yl)méthoxy)benzamide

### Etape 1: Préparation du chlorure de 2-((3-((4-fluorophényl) éthynyl)imidazo[1,2-a]pyridin-2-yl)méthoxy)benzoyle

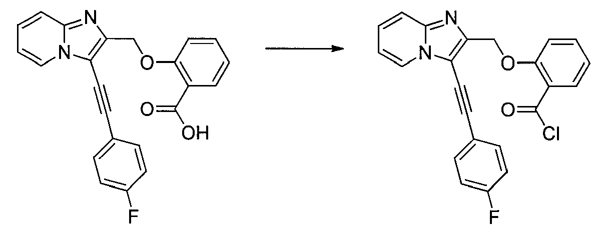

0,6 g (1,553 mmol) d'acide 2-((3-((4-fluorophényl)éthynyl)imidazo[1,2-a] pyridin-2-yl) méthoxy)benzoïque ont été mis en suspension dans 20 ml de Dichlorométhane sous agitation magnétique, le mélange a été placé dans un bain à 0°C puis 0,408 ml (4,66 mmol) de chlorure d'oxalyle ont été additionnés goutte à goutte, enfin, quelques gouttes de diméthylformamide ont été ajoutées. Le bain froid a été retiré puis le mélange a été agité à t.a. pendant 2 h. Le milieu réactionnel a été concentré sous vide et utilisé immédiatement dans l'étape suivante.

### Etape 2: Préparation du N-(2-(diméthylamino)éthyl)-2-((3-((4-fluorophényl)éthynyl)imidazo[1,2-a]pyridin-2-yl)méthoxy) benzamide (dérivé N° 95)

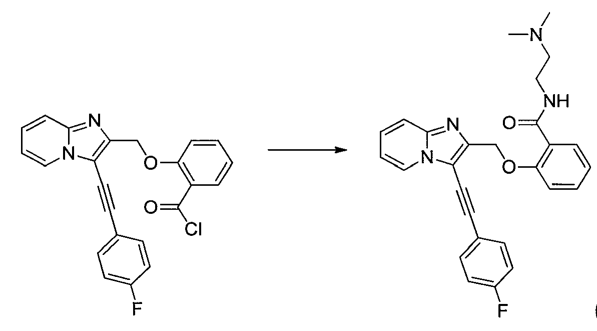

0,896 ml (7,76 mmol) de N,N-diméthyléthane-1,2-diamine ont été solubilisés dans 8 ml de Dichlorométhane sous agitation magnétique, 0,449 g (0,776 mmol) de chlorure de 2-((3-((4-fluorophényl)éthynyl) imidazo[1,2-a]pyridin-2-yl) méthoxy)benzoyle ont été additionnés et le mélange a été agité à t.a. pendant 16 h avant d'être concentré sous vide. Le résidu brut a été purifié par trituration dans 20 ml d'éther diisopropylique. Le solide a été isolé par filtration lavé avec 10 ml d'eau et séché sous vide. 0,178 g (rendement = 50%) de N-(2-(diméthylamino) éthyl)-2-((3-((4-fluorophényl)éthynyl) imidazo[1,2-a]pyridin-2-yl) méthoxy) benzamide ont été obtenus sous forme d'un solide jaune pâle. LC-MS: m/z = 457 (MH⁺) ; pureté UV à 254 nm = 98%. RMN ¹H (300 MHz, DMSO) δ 8.68 (d, J = 6.7 Hz, 1H), 8.47 (d, J = 4.8 Hz, 1H), 7.91 (dd, J = 7.7, 1.5 Hz, 1H), 7.72 (dd, J = 8.6, 5.2 Hz, 3H), 7.57 - 7.39 (m, 3H), 7.32 (t, J = 8.9 Hz, 2H), 7.17 (t, J = 6.6 Hz, 1H), 7.09 (t, J = 7.3 Hz, 1H), 5.51 (s, 2H),2,5 (1 H masqué sous le pic de solvant), 2.25 (t, J = 6.5 Hz, 2H), 1.92 (s, 6H).

Le dérivé numéro **96** a été préparé selon le même protocole.

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **96** | 401,39 | solide | 97,5 | 402 | | 10.97 (s, 1H), 9.12 (s, 1H), 8.66 (d, *J* = 6.8 Hz, 1H), 7.77 (dd, *J* = 8.8, 5.5 Hz, 2H), 7.70 (dd, *J* = 5.4, 3.5 Hz, 2H), 7.55 - 7.26 (m, 5H), 7.16 (t, *J* = 6.4 Hz, 1H), 7.06 (t, *J* = 6.9 Hz, 1H), 5.52 (s, 2H). |

### Exemple 23: Préparation du dérivé N°97: N-(3-((3-(phényl éthynyl)imidazo[1,2-a]pyridin-2-yl)méthoxy)phényl) méthane sulfonamide

### Etape 1: Préparation du 2-(chlorométhyl)-3-(phényléthynyl) imidazo[1,2-a]pyridine

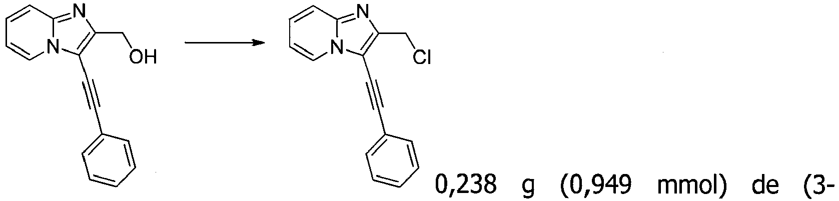

(phényléthynyl)imidazo[1,2-a]pyridin-2-yl) méthanol ont été dissous dans 5 ml de dichlorométhane sous agitation magnétique puis 0,346 ml (4,75 mmol) de chlorure de thionyle ont été ajoutés. Le mélange a été agité à t.a. pendant 2 h avant d'être concentré sous vide. Le résidu a été repris dans 20 ml d'un mélange composé d'acétate d'éthyle et d'une solution aqueuse saturée en NaHCO₃ (1/1, v/v). Après séparation, la phase aqueuse a été extraite avec 10 ml d'acétate d'éthyle. Les phases organiques combinées ont été lavées avec 10 ml d'une solution aqueuses saturée en NaCl puis séchées sur Na₂SO₄ qui a ensuite été éliminé par filtration. Le filtrat obtenu a été concentré sous vide. 0,246 g (rendement = 96%) de 2-(chlorométhyl)-3-(phényl éthynyl)imidazo[1,2-a]pyridine ont été obtenus sous forme d'un solide marron. LC-MS: m/z = 267 (MH⁺) ; pureté UV à 254 nm = 98%.

### Etape 2: Préparation du N-(3-hydroxyphényl) méthanesulfonamide

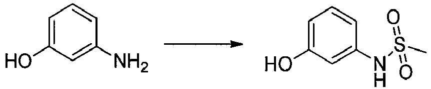

2 g (18,33 mmol) de 3-aminophénol ont été dissous dans 6 ml de pyridine sous agitation magnétique, le mélange a été refroidi à 10°C puis 1,8 ml (23,26 mmol) de chlorure de méthanesufonyle ont été ajoutés. Le bain froid a été retiré et le mélange a été agité à t.a. pendant 18 h avant d'être dilué avec 100 ml d'une solution aqueuse saturée en NaCl. La phase aqueuse a été extraite avec 100 ml d'acétate d'éthyle. La phase organique a été lavée avec 50 ml d'une solution aqueuse saturée en NaHCO₃, extraite avec 50 ml d'une solution aqueuse de NaOH (0,5 N). La phase aqueuse basique a été lavée avec 2 x 100 ml d'acétate d'éthyle puis acidifiée jusqu'à pH 2-3 par ajout d'une solution aqueuse d'HCl 1N. La phase aqueuse acidifiée a été extraite avec 2 x 100 ml d'acétate d'éthyle. Les phases organiques combinées ont été lavées avec 100 ml d'une solution aqueuses saturée en NaCl puis séchées sur Na₂SO₄ qui a ensuite été éliminé par filtration. Le filtrat obtenu a été concentré sous vide. 3,3 g (rendement = 80%) de N-(3-hydroxyphényl)méthanesulfonamide ont été obtenus sous forme d'une huile. LC-MS: m/z = 186 (M-H⁺) ; pureté UV à 254 nm = 81%.

### Etape 3: Préparation du N-(3-((3-(phényléthynyl)imidazo[1,2-a]pyridin-2-yl)méthoxy)phényl)méthanesulfonamide (dérivé numéro 97)

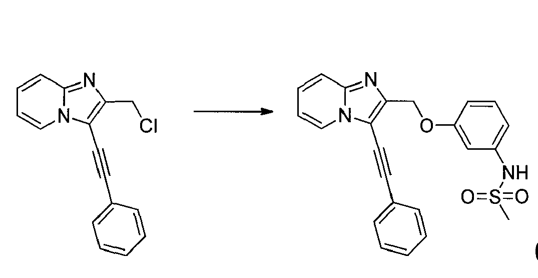

0,094 g (0,352 mmol) de 2-(chlorométhyl)-3-(phényléthynyl)imidazo[1,2-a] pyridine ont été dissous dans 2 ml de diméthylformamide sous agitation magnétique, 121 mg (0,529 mmol) de N-(3-hydroxyphényl) méthanesulfonamide et 0,172 g (0,529 mmol) de carbonate de césium ont été ajoutés. Le mélange a été agité à 60°C pendant 3 h avant d'être versé sur 15 ml d'une solution aqueuses saturée en NaCl. La phase aqueuse a été extraite avec 2 x 20 ml d'acétate d'éthyle. Les phases organiques combinées ont été séchées sur Na₂SO₄ qui a ensuite été éliminé par filtration. Le filtrat obtenu a été concentré sous vide. Le résidu brut a été purifié par HPLC préparative et 0,028 g (rendement = 18%) de N-(3-((3-(phényléthynyl)imidazo[1,2-a]pyridin-2-yl)méthoxy)phényl) méthane sulfonamide ont été obtenus sous forme d'un solide blanc. LC-MS: m/z = 418 (MH⁺) ; pureté UV à 254 nm = 95%. RMN ¹H (300 MHz, DMSO) δ 9.53 (s, 1H), 8.55 (d, J = 6.8 Hz, 1H), 7.76 - 7.57 (m, 3H), 7.54 - 7.33 (m, 4H), 7.17 - 7.02 (m, 2H), 6.89 (dd, J = 8.0, 1.1 Hz, 1H), 6.79 (t, J = 2.1 Hz, 1H), 6.69 - 6.54 (m, 1H), 5.07 (s, 2H), 3.14 (s, 3H).

### Exemple 24 : Préparation du dérivé N°111 : 2-((6-chloro-3-((3-(trifluorométhyl)phényl)éthynyl)imidazo[1,2-a]pyridin-2-yl) méthylthio)acétate d'éthyle

### Etape 1: Préparation de 2-((6-chloroimidazo[1,2-a]pyridin-2-yl)méthylthio)acétate d'éthyle

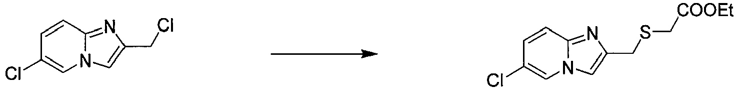

20 g (99 mmol) de 6-chloro-2-(chlorométhyl)imidazo[1,2-a]pyridine, 0,746 g (4,97 mmol de iodure de sodium et 64,8 g (199 mmol de carbonate de césium ont été chargés dans 500 ml d'acétonitrile. La solution a été agitée à température ambiante (t.a.) pendant 3 heures. Le minéral précipité a été filtré, et le solvant évaporé sous vide. Le solide marron obtenu a été dissout dans 300 ml d'acétate d'éthyle, lavé avec 3 fois 100 ml d'eau et directement purifié sur un gâteau de silice, en éluant avec de l'acétate d'éthyle. 28,74 g (rendement = 100%) de 2-((6-chloroimidazo[1,2-a] pyridin-2-yl)méthylthio)acétate d'éthyle ont été obtenus sous forme d'un solide orange. LC-MS: m/z = 285 (MH⁺) pureté UV à 254 nm = 99%. RMN 1H (300 MHz, DMSO) δ 8.79 (dd, J = 2.1, 0.8 Hz, 1H), 7.82 (s, 1H), 7.52 (d, J = 9.6 Hz, 1H), 7.25 (dd, J = 9.6, 2.1 Hz, 1H), 4.06 (q, J = 7.1 Hz, 2H), 3.90 (s, 2H), 3.36 (s, 2H), 1.16 (t, J = 7.1 Hz, 3H).

### Etape 2 : Préparation de 3-iodo-2-(iodométhyl)imidazo[1,2-a]pyridine

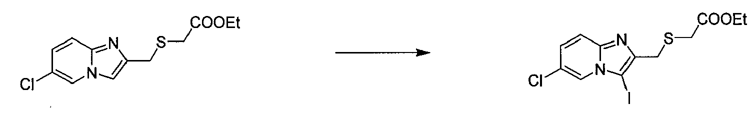

24,5 g (86 mmol) de 2-((6-chloroimidazo[1,2-a]pyridin-2-yl)méthylthio) acétate d'éthyle ont été ajoutés à 400 ml d'acétonitrile suivi par l'addition de 20,32 g (90 mmol) de N-iodosuccinimide. La solution a été agitée à t.a. pendant 1 heure. Le solvant a été évaporé sous vide, et le résidu obtenu a été fortement agité dans 250 ml d'eau et 200 ml d'éther diisopropylique. Le solide précipité a été filtré et lavé avec 2 fois 30 ml d'eau et 2 fois 30 ml d'éther diisopropylique. 33,93 g (rendement = 88%) de 3-iodo-2-(iodométhyl)imidazo[1,2-a]pyridine ont été obtenus sous forme d'un solide marron. LC-MS: m/z = 411 (MH⁺) pureté UV à 254 nm = 91.3%. RMN 1H 300 MHz, DMSO) δ 8.40 (dd, J = 1.9, 0.7 Hz, 1H), 7.61 (dd, J = 9.5, 0.6 Hz, 1H), 7.38 (dd, J = 9.5, 2.0 Hz, 1H), 4.07 (q, J = 7.1 Hz, 2H), 3.92 (s, 2H), 3.39 (s, 2H), 1.18 (t, J = 7.1 Hz, 3H).

### Etape 3 : Préparation de 2-((6-chloro-3-((3-(trifluorométhyl) phényl)éthynyl)imidazo[1,2-a]pyridin-2-yl)méthylthio)acétate d'éthyle

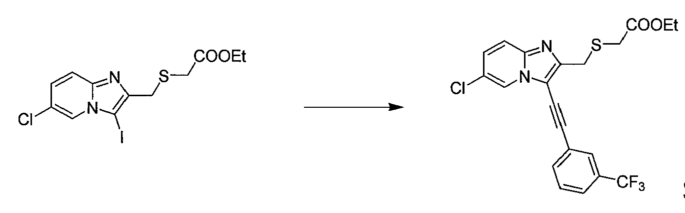

Sous atmosphère d'argon, 27 g (59,8 mmol) de 2-((6-chloro-3-iodoimidazo[1,2-a]pyridin-2-yl)méthylthio)acétate d'éthyle, 9,51 ml (65,8 mmol) de 1-éthynyl-3-(trifluorométhyl)benzène, 1,139 g (5,98 mmol de iodure de cuivre et 125 ml (897 mmol) de triéthylamine sont chargés dans 100 ml de DMF. On a fait buller de l'argon dans la solution pendant 10 mn, puis 2,74g (2,99 mmol) de Pd₂(dba)₃ ont été additionnés. Le mélange a été agité pendant 1 heure à t.a. Le catalyseur a été filtré sur papier plissé, la solution a été versée sur 300 ml d'eau, extraite avec 2 fois 200 ml d'acétate d'éthyle, les phases organiques réunies ont été lavées avec 3 fois 150 ml d'eau et 1 fois 150 ml de saumure, séchées sur Na₂SO₄ qui a ensuite été éliminé par filtration et le filtrat obtenu a été concentré sous vide. Le résidu a été purifié sur gâteau de silice, et élué avec un mélange Heptane / acétate d'éthyle. 23,21 g (rendement = 85%) de 2-((6-chloro-3-((3-(trifluorométhyl)phényl)éthynyl)imidazo[1,2-a]pyridin-2-yl) méthylthio)acétate d'éthyle ont été obtenus sous forme d'un solide jaune. LC-MS: m/z = 453 (MH⁺) pureté UV à 254 nm = 99%. RMN 1H (300 MHz, DMSO) δ 8.91 (dd, J = 1.9, 0.6 Hz, 1H), 8.14 (s, 1H), 8.01 (d, J = 7.6 Hz, 1H), 7.71 (ddd, J = 14.3, 9.4, 4.3 Hz, 3H), 7.47 (dd, J = 9.5, 2.0 Hz, 1H), 4.18 - 3.93 (m, 4H), 3.46 (s, 2H), 1.11 (t, J = 7.1 Hz, 3H).

Les dérivés **108** à **110** et **112** à **114** ont été préparés selon le même protocole

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **108** | 418.43 | Solide marron | 99 | 419 | | 8.72 (d, J = 6.7 Hz, 1H), 8.11 (s, 1H), 7.97 (d, J = 7.5 Hz, 1H), 7.86 - 7.60 (m, 3H), 7.52 - 7.37 (m, 1H), 7.13 (td, J = 6.8, 1.0 Hz, 1H), 4.09 (s, 2H), 4.03 (q, J = 7.1 Hz, 2H), 3.47 (s, 2H), 1.11 |
| **109** | 434.43 | Huile marron | 99 | 435 | | 8.68 (d, J = 6.7 Hz, 1H), 7.77 - 7.56 (m, 4H), 7.45 (d, J = 1.0 Hz, 2H), 7.13 (t, J = 6.6 Hz, 1H), 4.09 - 3.98 (m, 4H), 3.46 (s, 2H), 1.11 (t, J = |
| **110** | 402,87 | solide orange | 92 | 403 | | 8.82 (d, J = 1.2 Hz, 1H), 7.80 (dd, J = 8.6, 5.6 Hz, 2H), 7.69 (d, J = 9.5 Hz, 1H), 7.47 (dd, J = 9.5, 1.9 Hz, 1H), 7.34 (t, J = 8.9 Hz, 2H), 4.02 (dd, J = 12.9, 5.8 Hz, 4H), 3.45 (s, 2H), 1.11 |
| **112** | 468,88 | Solide jaune | 99 | 469,1 | | 8.93 (d, J = 1.3 Hz, 1H), 7.84 (s, 1H), 7.78 (d, J = 7.8 Hz, 1H), 7.72 (d, J = 9.5 Hz, 1H), 7.62 (t, J = 8.0 Hz, 1H), 7.49 (td, J = 9.5, 1.6 Hz, 2H), 4.09 (s, 2H), 4.04 (q, J = 7.1 Hz, 2H), 3.47 (s, 2H), |
| **113** | 447,47 | Huile marron | 96,2 | 448,1 | | 8.74 (d, *J* = 6.7 Hz, 1H), 8.13 (s, 1H), 7.99 (d, *J* = 7.7 Hz, 1H), 7.79 (d, *J* = 8.0 Hz, 1H), 7.69 (dd, *J* = 15.5, 8.1 Hz, 2H), 7.49 - 7.41 (m, 1H), 7.14 (t, *J* = 6.8 Hz, 1H), 4.01 (p, *J* = 7.3 Hz, 4H), 3.61 (t, *J* = 6.3 Hz, 1H), 2.84 (t, *J* = 6.8 Hz, 2H), 1.19 - 0.98 (m, 5H). |
| **114** | 448,46 | Solide beige | 99 | 449,1 | | 8.32 (d, 1H), 8.11 (s, 1H), 7.98 (d, 1H), 7.77 (m, J = 21.6 Hz, 2H), 7.03 (t, 1H), 6.88 (d, 1H), 4.07 (s,3H), 4.01 (q, *J* = 31.6 Hz, 2H), 3.96 (s,2H), 3.46 (s, 2H), |

### Exemple 25 : Préparation du dérivé N°118 : acide 2-((6-chloro-3-((3-(trifluorométhyl)phényl)éthynyl)imidazo[1,2-a]pyridin-2-yl) méthylthio)acétique

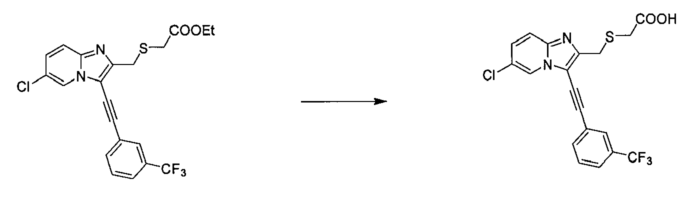

16 g (35,3 mmol) de 2-((6-chloro-3-((3-(trifluorométhyl)phényl)éthynyl)imidazo[1,2-a]pyridin-2-yl)méthylthio)acétate d'éthyle ont été ajoutés à 75 ml d'éthanol et 75 ml de tétrahydrofurane avant l'addition de 4,24 ml (42,4 mmol) d'une solution aqueuse d'hydroxyde de sodium à 32%. Le mélange a été agité à t.a. pendant 2 heures, mais 50 ml d'eau ont été additionnés après 30 mn pour solubiliser le solide précipité. Le solvant a été évaporé sous vide, le résidu obtenu a été dilué dans 250 ml d'eau et 50 ml d'éther diisopropylique, puis on a amené la solution à un pH acide par addition de 2,53 ml (44,2 mmol) d'acide acétique. Le solide précipité a été filtré puis lavé avec de l'eau et de l'éther diisopropylique. 12,45 g (rendement = 83% d'acide 2-((6-chloro-3-((3-(trifluorométhyl) phényl)éthynyl)imidazo [1,2-a]pyridin-2-yl)méthylthio)acétique a été obtenu sous forme d'un solide jaune. LC-MS: m/z = 425 (MH⁺) pureté UV à 254 nm = 99%. RMN 1H (300 MHz, DMSO) δ 12.65 (s, 1H), 8.96 (s, 1H), 8.19 (s, 1H), 8.03 (d, J = 7.6 Hz, 1H), 7.84 - 7.63 (m, 3H), 7.50 (dd, J = 9.5, 1.8 Hz, 1H), 4.07 (s, 2H), 3.40 (s, 2H).

Les dérivés **115** à **117, 119** et **120** ont été préparés selon le même protocole

| **No** | **Poids moléculaire** | **Apparence** | **Pureté LCMS UV à 254 nm** | **Spectrométrie de masse m/z** | | **RMN ¹H (300 MHz, DMSO) δ** |
|---|---|---|---|---|---|---|
| | **g/mol** | | | **MH⁺** | **M-H⁺** | |
| **115** | 390.38 | Solide jaune | 99 | 391 | 389 | 8.73 (d, J = 6.7 Hz, 1H), 8.12 (s, 1H), 7.98 (d, J = 7.6 Hz, 1H), 7.85 - 7.59 (m, 3H), 7.52 - 7.40 (m, 1H), 7.13 (td, J = 6.8, 1.0 Hz, 1H), 4.20 - 3.98 (m, 2H), 3.40 (s, 2H). |
| **116** | 406.38 | Solide | 97 | 407 | 405 | 12.62 (s, 1H), 8.69 (d, J = 6.3 Hz, 1H), 7.86 - 7.52 (m, 4H), 7.45 (dd, J = 8.0, 7.0 Hz, 2H), 7.12 (dd, J = 14.8, 8.1 Hz, 1H), 4.07 (s, 2H), 3.40 |
| **117** | 374,82 | Solide orange | 99 | 375 | | 12.66 (s, 1H), 8.83 (s, 1H), 7.81 (dd, J = 7.6, 5.7 Hz, 2H), 7.70 (d, J = 9.5 Hz, 1H), 7.47 (d, J = 9.5 Hz, 1H), 7.33 (t, J = 8.6 Hz, 2H), 4.04 (s, |
| **119** | 440,82 | solide jaune pâle | 97 | 441,1 | 439 | 12.64 (s, 1H), 8.92 (d, J = 1.2 Hz, 1H), 7.84 (s, 1H), 7.78 (d, J = 7.8 Hz, 1H), 7.71 (d, J = 9.5 Hz, 1H), 7.60 (t, J = 8.0 Hz, 1H), 7.54 - 7.37 (m, 2H), 4.07 (s, 2H), 3.39 (s, 2H) |
| **120** | 420,41 | Solide | 99 | 421,6 | 419,6 | 8.32 (d, 1H), 8.12 (s, 1H), 8.01 (d, 1H), 7.72 (m, 2H), 7.02 (t, 1H), 6.87 (d, 1H), 3.97 (s, 2H), 3.96 (s, 3H), 3.24 |

### Exemple 26 : Préparation du dérivé N°121 : 2-((3-((3-(trifluorométhyl)phényl)éthynyl)imidazo[1,2-a]pyridin-2-yl) méthylsulfonyl)acétate d'éthyle

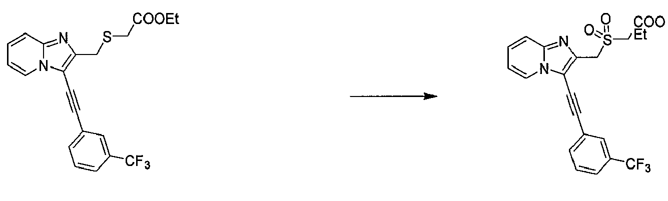

40 mg (0,096 mmol) de 2-((3-((3-(trifluorométhyl)phényl)éthynyl)imidazo [1,2-a]pyridin-2-yl)méthylthio)acétate d'éthyle ont été solubilisés dans 1 ml d'acétone suivi par l'addition de 8,03 mg (0,096 mmol) d'hydrogénocarbonate de sodium. La solution a été refroidit à 0°C avant l'addition d'une solution de 118 mg (0,096 mmol) d'oxone dans 1 ml d'eau. Le mélange a été agité à t.a. pendant 4 heures. La solution a été lavée avec 5 ml d'eau, et le solide obtenu a été filtré et lavé avec 5 ml d'eau et 2 ml d'éther diisopropylique. 29 mg (rendement = 64%) de 2-((3-((3-(trifluorométhyl)phényl)éthynyl) imidazo[1,2-a]pyridin-2-yl)méthylsulfonyl) acétate d'éthyle ont été obtenus sous forme d'un solide jaune pâle. LC-MS: m/z = 451 (MH⁺) pureté UV à 254 nm = 94.6%. RMN 1H (300 MHz, DMSO) δ 8.80 (d, J = 6.8 Hz, 1H), 8.17 (s, 1H), 8.03 (d, J = 7.6 Hz, 1H), 7.76 (dt, J = 13.6, 7.8 Hz, 3H), 7.62 - 7.45 (m, 1H), 7.21 (t, J = 6.6 Hz, 1H), 4.98 (s, 2H), 4.62 (s, 2H), 4.19 (dd, J = 14.1, 7.0 Hz, 2H), 1.21 (t, J = 7.1 Hz, 3H).

Le dérivé **122** a été préparé selon le même protocole

| **No** | **Poids moléculaire g/mol** | **Apparence** | **Pureté LCMS UV à 254 nm** | **Spectrométrie de masse m/z** | | **RMN ¹H (300 MHz, DMSO) δ** |
|---|---|---|---|---|---|---|
| | | | | **MH⁺** | **M-H⁺** | |
| **122** | 500,88 | Solide gris | 99 | 501 | 499 | 9.01 (s, 1H), 7.82 (dd, J = 17.1, 9.6 Hz, 3H), 7.65 - 7.46 (m, 3H), 4.97 (s, 2H), 4.60 (s, 2H), 4.19 (q, J = 7.1 Hz, 2H), 1.21 (t, J = 7.1 Hz, 3H). |

### Exemple 27 : Préparation du dérivé N°123 : acide 2-((6-chloro-3-((3-(trifluorométhoxy)phényl)éthynyl)imidazo[1,2-a]pyridin-2-yl)méthylsulfonyl)acétique

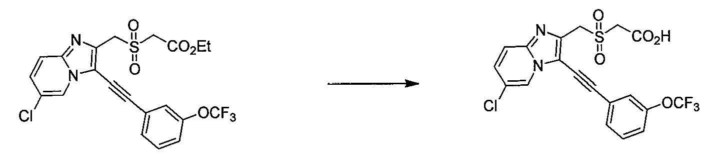

100 mg (0,200 mmol) de 2-((6-chloro-3-((3-(trifluorométhoxy) phényl)éthynyl) imidazo[1,2-a]pyridin-2-yl)méthylsulfonyl)acétate d'éthyle ont été dissous dans 2 ml d'éthanol avant addition de 0,060 ml (0,599 mmol) d'une solution aqueuse d'hydroxyde de sodium à 32%. Le mélange a été agité à t.a. pendant 16 heures. La solution a été versée dans 10 ml d'eau, acidifiée à pH acide avec de l'acide acétique, extraite avec 2 fois 5 ml de dichlorométhane, les phases organiques combinées ont été séchées sur Na₂SO₄ qui a ensuite été éliminé par filtration et le filtrat obtenu a été concentré sous vide. 76 mg (rendement = 80%) d'acide 2-((6-chloro-3-((3-(trifluorométhoxy)phényl)éthynyl)imidazo[1,2-a]pyridin-2-yl) méthylsulfonyl)acétique ont été obtenus sous forme d'un solide jaune. LC-MS: m/z = 473 (MH⁺) pureté UV à 254 nm = 99%. RMN 1H (300 MHz, DMSO) δ 14.72 - 12.20 (m, 1H), 9.00 (d, J = 1.2 Hz, 1H), 7.88 - 7.76 (m, 3H), 7.62 - 7.45 (m, 3H), 4.96 (s, 2H), 4.42 (s, 2H).

### Exemple 28: Préparation du dérivé N°124: N-(pyridin-3-yl)-2-((3-((3-(trifluorométhyl)phényl)éthynyl)imidazo[1,2-a]pyridin-2-yl)méthylthio)acétamide

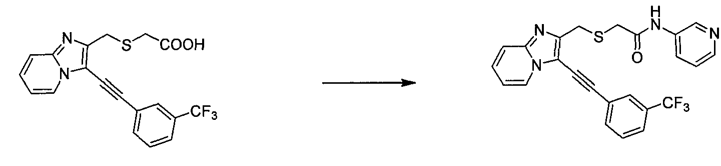

141 mg (0,358 mmol) d'acide 2-((3-((3-(trifluorométhyl)phényl)éthynyl) imidazo [1,2-a]pyridin-2-yl)méthylthio)acétique ont été dissous dans 5 ml de dichlorométhane suivi par l'addition de 0,094 ml (1,073 mmol) de chlorure d'oxalyle et d'une goutte de DMF. La solution a été agitée à t.a. pendant 2 heures puis le solvant a été évaporé sous vide. Le résidu obtenu a été repris dans 5 ml de tétrahydrofurane, 99 mg (0,715 mmol) de carbonate de sodium ont été ajoutés suivi par l'addition de 40,4 mg (0,429 mmol) de 3-aminopyridine. La solution a été agitée à t.a. pendant 16 heures. Le mélange été ensuite versé sur 15 ml d'eau, extrait 3 fois avec 15 ml d'acétate d'éthyle, les phases organiques combinées ont été lavées avec 20 ml de saumure puis ont été séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de Dichlorométhane / Méthanol, 100% à 90% de Dichlorométhane, v/v). 89 mg de N-(pyridin-3-yl)-2-((3-((3-(trifluorométhyl)phényl)éthynyl)imidazo[1,2-a]pyridin-2-yl)méthylthio) acétamide ont été obtenus sous forme de solide jaune pâle. LC-MS: m/z = 467 (MH⁺) pureté UV à 254 nm = 95%. RMN 1H (300 MHz, DMSO) δ 10.13 (s, 1H), 8.76 (dd, J = 16.4, 4.5 Hz, 2H), 8.24 (dd, J = 4.7, 1.5 Hz, 1H), 8.07 (ddd, J = 8.3, 2.5, 1.5 Hz, 1H), 7.79 - 7.60 (m, 3H), 7.60 - 7.35 (m, 3H), 7.30 (dd, J = 8.3, 4.7 Hz, 1H), 7.16 (td, J = 6.8, 1.1 Hz, 1H), 4.90 (s, 2H), 4.26 (s, 2H).

Les dérivés **125** à **128** ont été préparés selon le même protocole

| **No** | **Poids moléculaire** | **Apparence** | **Pureté LCMS** | **Spectrométrie de masse m/z** | | **RMN ¹H (300 MHz, DMSO) δ** |
|---|---|---|---|---|---|---|
| | **g/mol** | | **UV à 254 nm** | **MH⁺** | **M-H⁺** | |
| **125** | 390,31 | Solide jaune pâle | 99 | 391 | 389 | 10.13 (s, 1H), 8.76 (dd, J = 16.4, 4.5 Hz, 2H), 8.24 (dd, J = 4.7, 1.5 Hz, 1H), 8.07 (ddd, J = 8.3, 2.5, 1.5 Hz, 1H), 7.79 - 7.60 (m, 3H), 7.60 - 7.35 (m, 3H), 7.30 (dd, J = 8.3, 4.7 Hz, 1H), 7.16 (td, J = 6.8, 1.1 Hz, 1H), 4.90 (s, 2H), 4.26 (s, 2H). |
| **126** | 482,48 | Solide jaune | 99 | 483 | 481 | 10.30 (s, 1H), 8.73 - 8.59 (m, 2H), 8.22 (dd, J = 4.7, 1.4 Hz, 1H), 8.00 (ddd, J = 8.3, 2.5, 1.5 Hz, 1H), 7.72 - 7.59 (m, 3H), 7.56 - 7.37 (m, 3H), 7.26 (dd, J = 8.3, 4.7 Hz, 1H), 7.12 (td, J = 6.8, 1.1 Hz, 1H), 4.13 |
| **127** | 451,89 | Solide | 98 | 452 | | 8.96 (s, 1H), 8.20 (s, 1H), 8.05 (d, J = 7.6 Hz, 1H), 7.97 (s, 1H), 7.79 (d, J = 7.8 Hz, 1H), 7.71 (d, J = 9.7 Hz, 2H), 7.50 (d, J = 11.4 Hz, 1H), 4.10 (s, 2H), 3.20 (s, 2H), 3.05 (dt, J = 14.2, |
| **128** | 467,89 | Solide | 97 | 468 | | 8.92 (s, 1H), 7.97 (s, 1H), 7.85 (s, 1H), 7.79 (d, J = 7.8 Hz, 1H), 7.71 (d, J = 9.5 Hz, 1H), 7.60 (t, J = 8.0 Hz, 1H), 7.54 - 7.40 (m, 2H), 4.09 (s, 2H), 3.20 (s, 2H), 3.11 - 2.99 (m, 2H), 0.96 (t, J |

### Exemple 29 : Préparation du dérivé N°129 : acide 4-hydroxy-3,3-diméthyl-2-((3-((3-(trifluorométhyl)phényl)éthynyl)imidazo[1,2-a]pyridin-2-yl)méthylthio)butanoique

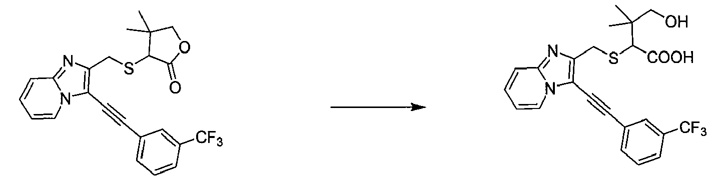

130 mg (0,303 mmol) de 4,4-diméthyl-3-((3-((3-(trifluorométhyl)phényl)éthynyl) imidazo[1,2-a]pyridin-2-yl)méthylthio)dihydrofuran-2(3H)-one ont été dissous dans 2 ml de méthanol et 2 ml de tétrahydrofurane suivi de l'addition de 0,334 ml (0,334 mmol) d'une solution aqueuse d'hydroxyde de sodium à 1N. La solution a été agitée pendant 16 heures. On a acidifiée jusqu'à pH acide avec de l'acide acétique, et on a dilué avec de l'eau jusqu'à précipitation d'un solide, qui a ensuite été filtré et lavé avec de l'eau. 115 mg d'acide 4-hydroxy-3,3-diméthyl-2-((3-((3-(trifluorométhyl)phényl)éthynyl)imidazo[1,2-a]pyridin-2-yl)méthylthio) butanoique ont été obtenus sous forme de solide marron pâle. LC-MS: m/z = 447 (MH⁺) pureté UV à 254 nm = 99%. RMN 1H (300 MHz, DMSO) δ 12.69 (s, 1H), 8.77 (d, J = 6.7 Hz, 1H), 8.11 (s, 1H), 7.97 (d, J = 7.5 Hz, 1H), 7.79 (d, J = 7.7 Hz, 1H), 7.70 (t, J = 8.2 Hz, 2H), 7.54 - 7.41 (m, 1H), 7.16 (t, J = 6.6 Hz, 1H), 4.81 (d, J = 12.3 Hz, 1H), 4.70 (sl, 1H), 4.58 (d, J = 12.3 Hz, 1H), 3.92 (s, 1H), 3.40 (s, 1H), 3.10 (d, J = 10.4 Hz, 1H), 0.86 (s, 3H), 0.82 (s, 3H)

### Exemple 30 : Préparation du dérivé N°130 : 2-((3-((3-(trifluorométhyl)phényl)éthynyl)imidazo[1,2-a]pyridin-2-yl) méthylthio)éthanol

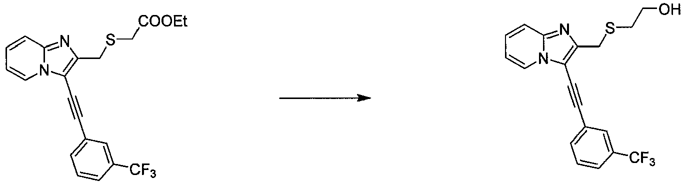

Sous atmosphère d'argon, 200 mg (0,446 mmol) de 2-((3-((3-(trifluorométhyl) phényl)éthynyl)imidazo[1,2-a]pyridin-2-yl)méthylthio) acétate d'éthyle ont été dissous dans 4 ml de tétrahydrofurane et 33,9 mg (0,892 mmol de LiAlH₄ ont été additionnés. La solution a été chauffée à 50°C pendant 1 heure. 0,030 ml d'eau a été lentement additionnée, suivi de l'addition de 0,030 ml de d'hydroxyde de sodium 6N puis de 0,1 ml d'eau. Après 30 mn d'agitation, le précipité a été filtré, et lavée avec de l'acétate d'éthyle. Les phases organiques réunies ont été lavées avec de la saumure puis ont été séchées sur Na₂SO₄ qui a ensuite été éliminé par filtration et le filtrat obtenu a été concentré sous vide. Le solide obtenu a été trituré dans de l'acétate d'éthyle, filtré puis lavé avec de l'acétate d'éthyle. 50 mg (rendement = 28%) de 2-((3-((3-(trifluorométhyl) phényl) éthynyl)imidazo [1,2-a]pyridin-2-yl)méthylthio) éthanol ont été obtenus sous forme d'un solide blanc. LC-MS: m/z = 407 (MH⁺) pureté UV à 254 nm = 89%. RMN 1H (300 MHz, DMSO) δ 8.30(d,1H), 8.12(s,1H), 7.99(m,1H), 7.75(m,2H), 7.03(m,1H), 6.88(d,1H), 4.84(t,1H), 3.96(s,5H), 3.60(m,2H), 2.67(t,2H).

Le dérivé **131** a été préparé selon le même protocole :

| **No** | **Poids moléculaire** | **Apparence** | **Pureté LCMS UV à 254 nm** | **Spectrométrie de masse m/z** | | **RMN ¹H (300 MHz, DMSO) δ** |
|---|---|---|---|---|---|---|
| | **g/mol** | | | **MH⁺** | **M-H⁺** | |
| **131** | 410,84 | Solide beige | 99 | 410,9 | | 8.95 (s, 1H), 8.18 (s, 1H), 8.04 (d, 1H), 7.76 (m, 3H), 7.50 (d, 1H), 4.84 (t, 1H), 3.98 (s, 1H), 3.59 (q, 2H), 2.67 |

### Exemple 31 : Préparation du dérivé N°132: N-(N-(tert-butoxycarbonyl)carbamimidoyl)-2-((3-((4-fluorophényl)éthynyl) imidazo[1,2-a]pyridin-2-yl)méthoxy)acétamide

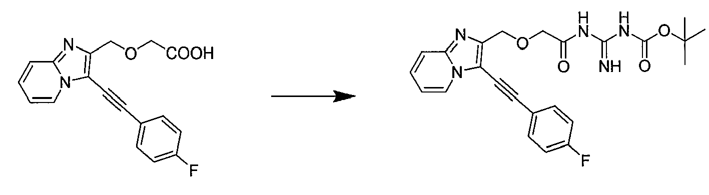

200 mg d'acide 2-((3-((4-fluorophényl)éthynyl)imidazo[1,2-a]pyridin-2-yl)méthoxy) acétique, 200 mg (0,617 mmol) de PyBOP et 118 mg (0,740 mmol) de guanidine-BOC ont été charges dans 2 ml de DMF. 0,258 ml (1,850 mmol) de triéthylamine a ensuite été coulée et la réaction a été agitée à t.a. pendant 4 heures. Le mélange a été versé dans 10 ml d'eau et 5 ml d'heptane sous agitation. Le solide obtenu a été filtré puis lavé avec 2 ml d'eau et 1 ml d'heptane. 220 mg (rendement = 77%) de N-(N-(tert-butoxycarbonyl)carbamimidoyl)-2-((3-((4-fluorophényl)éthynyl) imidazo [1,2-a]pyridin-2-yl)méthoxy)acétamide ont été obtenus sous forme de solide gris. LC-MS: m/z = 466 (MH⁺) pureté UV à 254 nm = 99%. RMN 1H (300 MHz, DMSO) δ 11.10 (s, 1H), 8.71 (t, J = 37.0 Hz, 3H), 7.86 - 7.62 (m, 3H), 7.51 - 7.37 (m, 1H), 7.30 (t, J = 8.9 Hz, 2H), 7.13 (dd, J = 6.7, 6.0 Hz, 1H), 4.81 (s, 2H), 4.18 (s, 2H), 1.38 (s, 9H).

Le dérivé **133** a été préparé selon le même protocole

| **No** | **Poids moléculaire** | **Apparence** | **Pureté LCMS UV à 254 nm** | **Spectrométrie de masse m/z** | | **RMN ¹H (300 MHz, DMSO) δ** |
|---|---|---|---|---|---|---|
| | **g/mol** | | | **MH⁺** | **M-H⁺** | |
| **133** | 531,55 | Solide jaune | 99 | 532,2 | 530,2 | 10.98 (s, 1H), 8.78 (t, J = 25.6 Hz, 3H), 8.10 (s, 1H), 7.93 (d, J = 7.6 Hz, 1H), 7.71 (dt, J = 17.7, 8.3 Hz, 3H), 7.52 - 7.38 (m, 1H), 7.13 (td, J = 6.8, 1.0 Hz, 1H), 4.10 (s, 2H), 3.43 (s, 2H), 1.38 |

### Exemple 32 : Préparation du dérivé N°134 : Chlorhydrate de N-carbamimidoyl-2-((3-((4-fluorophényl)éthynyl)imidazo[1,2-a] pyridin-2-yl)méthoxy)acétamide

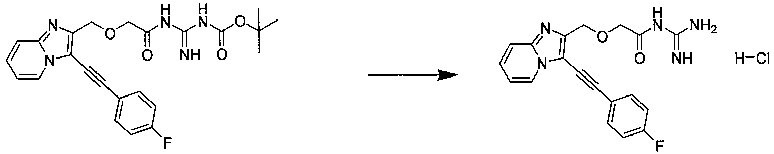

190 mg (0,408 mmol) de N-(N-(tert-butoxycarbonyl)carbamimidoyl)-2-((3-((4-fluorophényl)éthynyl)imidazo[1,2-a]pyridin-2-yl)méthoxy)acétamide ont été dissous dans 2 ml d'éthanol avant l'addition de 1,020 ml (4,08 mmol) de solution d'acide chlorhydrique dans du dioxanne à 4N. La solution a été chauffée à 60°C pendant 2 heures. Le solvant a été évaporé sous vide. Par refroidissement, le solide cristallisé a été filtré et lavé avec de l'éthanol. 85 mg (rendement = 52%) de chlorhydrate de N-carbamimidoyl-2-((3-((4-fluorophényl)éthynyl)imidazo[1,2-a]pyridin-2-yl) méthoxy)acétamide ont été obtenus sous forme d'un solide jaune. LC-MS: m/z = 366 (MH⁺) pureté UV à 254 nm = 99%. RMN 1H (300 MHz, DMSO) δ 11.36 (s, 1H), 8.86 (d, J = 6.7 Hz, 1H), 8.55 (d, J = 28.4 Hz, 4H), 7.92 - 7.71 (m, 4H), 7.44 - 7.31 (m, 3H), 4.99 (s, 2H), 4.40 (s, 2H).

Le dérivé **135** a été préparé selon le même protocole :

| **No** | **Poids moléculaire** | **Apparence** | **Pureté LCMS UV à 254 nm** | **Spectrométrie de masse m/z** | | **RMN ¹H (300 MHz, DMSO) δ** |
|---|---|---|---|---|---|---|
| | **g/mol** | | | **MH⁺** | **M-H⁺** | |
| **135** | 467,90 | Solide gris clair | 99 | 432,1 | | 11.80 (s, 1H), 8.81 (d, J = 6.7 Hz, 1H), 8.62 - 8.05 (m, 5H), 8.00 (d, J = 7.7 Hz, 1H), 7.81 (d, J = 7.9 Hz, 1H), 7.72 (dd, = 7.9 Hz, 7.72 (dd, J = 12.8, 4.9 Hz, 2H), 7.63 - 7.52 (m, 1H), 7.24 (t, J = 6.5 Hz, 1H), 4.16 |

### Exemple 33: Préparation du dérivé N°138: 2-((pyridin-2-ylthio)méthyl)-3-((3-(trifluorométhyl)phényl)éthynyl)imidazo [1,2-a]pyridine

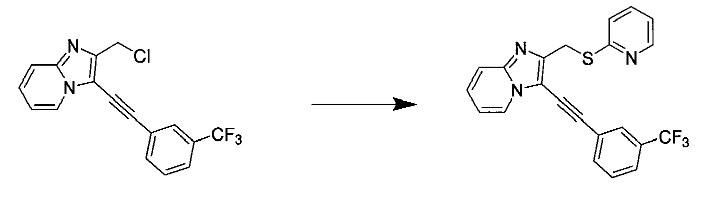

150 mg (0,421 mmol) de 2-(chlorométhyl)-3-((3-(trifluorométhyl) phényl)éthynyl)imidazo[1,2-a]pyridine ont été dissous dans 5 ml d'acétonitrile suivi de l'addition de 96 mg (0,842 mmol) de pyridine-2-thiol et 274 mg (0,842 mmol) de carbonate de césium. La solution a été agitée à t.a. pendant 2h30. Le mélange est ensuite versé dans 40 ml d'eau, puis extrait 3 fois avec 15 ml d'acétate d'éthyle. Les phases organiques réunies ont été lavées avec 30 ml d'une solution saturée d'hydrogénocarbonate de sodium saturée, avec 30 ml de saumure puis ont été séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient Heptane / Acétate d'éthyle, 80% à 70% d'Heptane, v/v). 129 mg (rendement = 74%) de 2-((pyridin-2-ylthio)méthyl)-3-((3-(trifluorométhyl)phényl)éthynyl)imidazo[1,2-a] pyridine ont été obtenus sous forme d'un solide beige. LC-MS: m/z = 410 (MH⁺) pureté UV à 254 nm = 99%. RMN 1H (300 MHz, DMSO) δ 8.70 (d, J = 6.7 Hz, 1H), 8.52 - 8.37 (m, 1H), 8.02 (s, 1H), 7.91 (d, J = 7.6 Hz, 1H), 7.71 (dd, J = 41.7, 4.5 Hz, 4H), 7.43 (s, 2H), 7.13 (d, J = 1.1 Hz, 2H), 4.73 (s, 2H).

Les dérivés **136** et **137** ont été préparés selon le même protocole :

| **No** | **Poids moléculaire** | **Apparence** | **Pureté LCMS UV à 254 nm** | **Spectrométrie de masse m/z** | | **RMN ¹H (300 MHz, DMSO) δ** |
|---|---|---|---|---|---|---|
| | **g/mol** | | | **MH⁺** | **M-H⁺** | |
| **136** | 417,47 | Solide marron clair | 94 | 417,9 | | 8.72 (d, J = 6.8 Hz, 1H), 8.10 (s, 1H), 7.97 (d, J = 7.6 Hz, 1H), 7.80 (d, J = 7.9 Hz, 1H), 7.76 - 7.64 (m, 2H), 7.51 - 7.42 (m, 1H), 7.15 (td, J = 6.8, 1.1 Hz, 1H), 4.69 (s, 2H), 4.16 (t, J = 8.0 Hz, |
| **137** | 412,43 | solide beige | 99 | 413,1 | | 8.77 (d, J = 6.7 Hz, 1H), 8.04 (s, 1H), 7.90 (d, J = 7.6 Hz, 1H), 7.73 (dt, J = 7.4, 6.3 Hz, 3H), 7.48 (dd, J = 13.5, 5.9 Hz, 2H), 7.21 - 7.07 (m, 2H), 6.99 (dd, J = 4.9, 3.5 Hz, 1H), 4.78 (d, J = |

### Test de stimulation de la sécrétion d'insuline par des cellules INS1

Les différents composés ont été testés sur la lignée beta pancréatique INS-1 pour évaluer leur capacité à potentialiser la sécrétion d'insuline en réponse au glucose. Très brièvement, les cellules sont cultivées dans du milieu de culture, RPMI 1640 10 mM glucose contenant 1 mM de pyruvate de sodium, 50 µM de 2-mercaptoéthanol, 2 mM de glutamine, 10 mM d'HEPES, 100 IU/mL de pénicilline, 100 µg/mL de streptomycine et 10% de sérum de veau foetal inactivé, comme décrit par Asfari et al. [9]. Pour le test de sécrétion d'insuline, les cellules INS-1 sont ensemencées et cultivées en plaque 96 puits. Après 3 jours de culture à 37°C dans une atmosphère humide (95% air/ 5% CO₂), le milieu est retiré et les cellules incubées pendant 16h dans du milieu contenant 5mM de glucose et 1% de sérum de veau foetal inactivé. Le jour du test, les cellules sont lavées avec un tampon Krebs (pH 7,4) contenant 0,1% d'albumine bovine puis pré-incubées pendant 30 minutes à 37°C dans ce même tampon contenant 2,8 mM de glucose. Enfin, les cellules sont lavées à nouveau avec du tampon Krebs puis incubées pendant 1 h dans le tampon du test de sécrétion (Krebs pH 7.4 contenant 0.1% d'albumine bovine et 3,5 mM de glucose et les molécules à évaluer). A la fin du test, le surnageant cellulaire est récupéré pour y mesurer l'insuline sécrétée à l'aide d'un kit ELISA utilisant un anticorps anti-insuline de rat (ELISA Alpco Cat n° 80-INSRTH-E10). Chaque condition est testée en triplicate. Le glucose à 3,5 mM, le GLP-1 à 10⁻⁷M et le mélange Forskoline 10⁻⁷/ IBMX 10⁻⁵M sont utilisés comme contrôles positifs du test. Un composé stimule la sécrétion d'insuline si ce facteur est supérieur ou égal à 130% du contrôle pour une dose de glucose donnée.

| **N° du dérivé** | **INS-1 % du ctrl @ 50 µM** | **INS-1 % du ctrl @ 10 µM** | **N° du dérivé** | **INS-1 % du ctrl @ 50 µM** | **INS-1 % du ctrl @ 10 µM** |
|---|---|---|---|---|---|
| **1** | 211 | 121 | **79** | 152 | 128 |
| **2** | 333 | 127 | **81** | 134 | 142 |
| **3** | 472 | 130 | **87** | 167 | 182 |
| **5** | 195 | 180 | **88** | 263 | 138 |
| **9** | 175 | 116 | **92** | 211 | 135 |
| **12** | 204 | 123 | **101** | 111 | 163 |
| **14** | 242 | 100 | **102** | 107 | 176 |
| **25** | 226 | 134 | **110** | 140 | 162 |
| **26** | 246 | 138 | **111** | 173 | 136 |
| **39** | 137 | 121 | **112** | 256 | 150 |
| **54** | | 203 | **113** | 112 | 136 |
| **55** | 135 | 130 | **117** | 137 | 130 |
| **58** | 233 | 127 | **129** | 146 | 109 |
| **59** | 220 | 134 | **130** | 114 | 150 |
| **72** | 151 | 172 | **131** | | 131 |
| **76** | 202 | 156 | **135** | | 131 |
| **78** | 152 | 197 | | | |

Les dérivés de formule I testés ont donc un effet significatif sur la potentialisation de la sécrétion d'insuline en réponse au glucose par les cellules INS-1 · pancréatiques. Les valeurs sont comprises entre 131% et plus de 450% d'activation indépendamment de la dose considérée.

### Étude de l'effet sur la production hépatique de glucose

### Matériel et méthode :

Les hépatocytes sont isolés à partir de foie de rat Wistar à jeun depuis 24 h après perfusion de collagénase dans la veine porte. 1) Les hépatocytes fraichement isolés sont ensemencés en plaques 6 puits coatées au collagène et contenant du milieu d'attachement (Milieu Williams). Après attachement, le milieu est remplacé par du milieu RPMI 1640 sans glucose contenant de l'hydrocortisone (7.10⁻⁵M) pour une durée de 16 à 18 h. Le jour suivant, le test de production hépatique de glucose est réalisé en milieu krebs pendant 3 h. Les conditions basales sont les cellules incubées avec du krebs seul, les conditions stimulées sont les cellules placées en Krebs+ lactate+ pyruvate, les conditions produits sont les cellules exposées aux composés chimiques dans un milieu krebs/ lactate/ pyruvate. Dans le cas où les composés sont dissous dans le DMSO, toutes les conditions du test sont réalisées en présence d'une concentration finale de DMSO de 0,1%. Le control positif du test est le mercaptopicolinate connu pour son action inhibitrice sur la production hépatique de glucose via la phosphoénolpyruvate carboxykinase. Pour les traitements à court terme, les composés sont incubés 3 h. Pour les traitements à long terme, les composés sont incubés 20 h au moment de la mise en culture des hépatocytes en RPMI puis de nouveau ajoutés lors du test de production hépatique pour 3 h. A la fin des 3 h d'incubation, le surnageant est récupéré pour la mesure de glucose par une méthode colorimétrique utilisant la glucose oxydase. Les cellules sont lysées par une solution aqueuse de NaOH à 0,1% afin de mesurer la quantité de protéine par la méthode Lowry. Les résultats sont exprimés en glucose par mg de protéine. Un composé inhibe la production hépatique de glucose si ce facteur est inférieur ou égal à 75% du contrôle.

### Incubation 3 h

| **N° du dérivé** | **HGP % du ctrl @ 100 µM** | **HGP % du ctrl @ 50 µM** | **N° du dérivé** | **HGP % du ctrl @ 100 µM** | **HGP % du ctrl @ 50 µM** |
|---|---|---|---|---|---|
| **1** | 56 | 70 | **87** | 25 | 43 |
| **3** | 60 | 73 | **88** | 50 | 67 |
| **4** | 74 | 81 | **89** | 33 | 78 |
| **7** | 45 | 55 | **90** | 27 | 77 |
| **10** | 71 | 86 | **92** | 39 | 58 |
| **14** | 73 | 86 | **93** | 19 | 26 |
| **23** | 73 | 95 | **95** | 62 | 65 |
| **25** | 73 | 83 | **96** | 66 | 81 |
| **26** | 67 | 78 | **99** | 68 | 85 |
| **29** | 40 | 63 | **108** | 64 | 89 |
| **30** | 60 | 74 | **109** | 61 | 80 |
| **35** | 64 | 73 | **110** | 37 | 50 |
| **46** | 69 | 85 | **111** | 27 | 73 |
| **51** | 45 | 61 | **112** | 26 | 65 |
| **59** | 45 | 97 | **115** | 54 | 94 |
| **76** | 59 | 67 | **116** | 64 | 88 |
| **77** | 50 | 87 | **117** | 43 | 55 |
| **78** | 44 | 51 | **119** | | 73 |
| **79** | 74 | 86 | **126** | 68 | 72 |
| **81** | 47 | 76 | | | |

Les dérivés de formule I testés ont donc un effet significatif sur l'inhibition de la production hépatique de glucose. Les plus fortes inhibitions sont obtenues avec les dérivés **7, 78, 87, 92, 93, 110** et **117** aux deux doses et avec les dérivés **29, 51, 59, 76, 77, 81, 88, 89, 90, 111, 112** et **115** à la dose de 100 µM.

### Incubation 20 h

| **N°du dérivé** | **HGP % du ctrl @ 100 µM** | **HGP % du ctrl @ 50 µM** | **N°du dérivé** | **HGP % du ctrl @ 100 µM** | **HGP % du ctrl @ 50 µM** |
|---|---|---|---|---|---|
| **12** | 54 | 89 | **58** | 17 | 32 |
| **46** | 52 | 93 | **118** | | 57 |
| **54** | 44 | 64 | | | |

Les dérivés de formule I testés ont donc un effet significatif sur l'inhibition de la production hépatique de glucose. Les plus fortes inhibitions sont obtenues aux deux doses avec le dérivé **58.**

### Etude de l'effet des composés sur la sécrétion en réponse au glucose au niveau de pancréas perfusés isolés de rats diabétiques NOSTZ

### Matériel et méthode :

Le pancréas est prélevé sur des rats rendus diabétiques par injection de Streptozotocine le jour de la naissance [10] et anesthésiés au Pentobarbital (Nembutal^{®} : 45 mg/kg ; voie intrapéritonéale). Ces rats présentent un défaut spécifique de la réponse insulinique au glucose [11], comme observé chez l'homme diabétique de type 2. L'isolement et la perfusion du pancréas ont été réalisés selon une modification [12] du protocole décrit par Sussman et al. [13]. L'effet des composés ou des substances de référence est testé pendant 35 minutes (de t = 20 min. à t = 55 min.) dans du tampon Krebs, puis 20 minutes (de t = 55 min. à t = 75 min.) en présence de glucose 16,5 mM. La concentration d'insuline sécrétée dans le milieu est mesurée par un dosage Elisa (ELISA Alpco Cat n° 80-INSRTH-E10). Les résultats sont exprimés comme la moyenne +/-ESM (écart standard moyen) de plusieurs expériences.

**Tableau de résultats :**

| **Numéro du dérivé** | **Concentration testée (µM)** | **Pic de Sécrétion d'insuline à G16.5mM** | |
|---|---|---|---|
| | | **Produit testé (µU/min.)** | **Groupe Contrôle (µU/min.)** |
| **44** | 10 | 1724 | 560 ± 71 |
| **54** | 1 | 961 ± 130 | 560 ± 71 |
| **54** | 10 | 1637 | 560 ± 71 |

Les dérivés de formule I testés ont un effet significatif sur la restauration de la sécrétion d'insuline en réponse au glucose au niveau de pancréas perfusés isolés de rats diabétiques N0STZ.

### Etude de l'activité antidiabétique chez le rat GK (Goto-Kakisaki)

L'activité antidiabétique du composé 118 a été évaluée chez le rat GK, un modèle de diabète de type 2 non obèse. Ce modèle a été obtenu par croisement de rats Wistar sélectionnés sur la base d'une légère intolérance au glucose [14]. Ces rats présentent la majorité des dysfonctions observées dans le diabète de type 2 chez l'homme [15] : hyperglycémie, intolérance au glucose, insulinorésistance, et réponse insulinique au glucose détériorée. Ces animaux sont élevés chez Metabrain et sont hébergés dans une animalerie à température régulée (22 ± 2°C) sous humidité constante (50 ± 20%) avec un cycle jour/nuit de 12h (lumière 7h-19h) et ont accès à volonté à la nourriture et la boisson. Les conditions d'hébergement et d'expérimentation répondent aux règles européennes concernant la santé et le traitement éthique des animaux de laboratoire (ETS123). Dans cette étude, les rats utilisés sont des rats GK mâles âgés de 16 semaines mis à jeun 2 heures avant le début de l'étude (état postabsorptif). Un suivi de la glycémie est réalisé sur 2 groupes de rats : Un groupe traité avec le composé **118** par voie orale à la dose unique de 50 mg/kg et un groupe contrôle traité par voie orale avec le véhicule. Une prise de sang est effectuée à T0 juste avant l'administration du composé **118** ou du véhicule et aux temps T1h, T2h, T4h et T6h après administration. La détermination de la glycémie est réalisée à partir d'une goutte de sang prélevée à la queue et mesurée par un glucomètre Accu-Check Performa. Les résultats présentés ci-dessous sont exprimés en pourcentage de diminution de la glycémie à T1h, T2h, T4h et T6h pour le groupe traité avec le composé **118** comparé au groupe contrôle

| **Composé 118** | **Temps après administration** | | | |
|---|---|---|---|---|
| | **T1h** | **T2h** | **T4h** | **T6h** |
| **% d'activité sur la glycémie** | -14% | -15% | -10% | -8% |
| **Signification statistique (test de t de Student)** | *p*=0,0242 * | *p*=0,0171 * | *p*=0,1468 *NS* | *p*=0,2450 *NS* |

Ces résultats montrent que le composé **118** administré en dose unique à 50 mg/kg est capable de réduire l'hyperglycémie d'un rat diabétique de type 2.

### BIBLIOGRAPHIE

[1] WO 2010089292 ;
[2] WO 2010070008 ;
[3] WO 2010034500 ;
[4] EP 2168965 ;
[5] WO 2009080291 ;
[6] WO 2009023179 ;
[7] Helvetica Chimica Acta (2007), 90(12), 2349-2367 ;
[8] Letters in Organic Chemistry (2005), 2(2), 184-187 ;
[9] Asfari et al., Endocrinology 130: 167-178, 1992 ;
[10] Portha et al., Diabetes, 23, (1974), 889-895 ;
[11] Giroix et al., Diabetes, 32, (1983), 445-451
[12] Assan et al., Nature, 239, (1972), 125-126 ;
[13] Sussman et al., Diabetes, 15, (1966), 466-472 ;
[14] Goto et al., Proc. Jpn. Acad. 51, 80-85, 1975 ;
[15] Portha et al., Mol. Cell. Endocrinol., 297 : 73-85, 2009 ;

## Revendications

1. Dérivé d'imidazopyridine de formule générale I suivante : 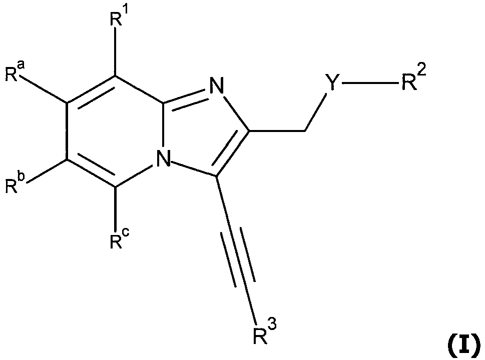 dans laquelle :
**Y** représente un atome d'oxygène ou de soufre, le groupe SO, SO₂ ou - NR¹⁹, dans lequel **R¹⁹** représente un atome d'hydrogène ou un groupe alkyle en C₁-C₆;
**R¹** R^{a}, R^{b} et R^{c} représentent indépendamment l'un de l'autre un atome d'hydrogène ; un atome d'halogène ; un groupe alkyle en C₁-C₆ éventuellement substitué par un groupe -OH ; un groupe -OH ; un groupe -O(alkyle en C₁-C₆); un groupe -O(alkyle en C₁-C₆)O(alkyle en C₁-C₆) ; un groupe -CN ; un groupe -NR⁴R⁵ dans lequel **R⁴** et **R⁵** représentent indépendamment l'un de l'autre un atome d'hydrogène ou un groupe alkyle en C₁-C₆ ; ou un groupe -(alkyle en C₁-C₆)NR⁶R⁷ dans lequel **R⁶** et **R⁷** représentent indépendamment l'un de l'autre un atome d'hydrogène ou un groupe alkyle en C₁-C₆ ;
**R²** représente
- un atome d'hydrogène ;
- un groupe alkyle en C₁-C₆ substitué par un groupe -OH ;
- un groupe (alkyle en C₁-C₆)COOR⁸ dans lequel **R⁸** représente un atome d'hydrogène ou un groupe alkyle en C₁-C₆, le groupe alkyle pouvant être substitué par un groupe -NH₂ ou -OH ;
- un groupe (alkyle en C₁-C₆)CONHR⁹ dans lequel **R⁹** représente un groupe -OH ; un groupe alkyle en C₁-C₆; un groupe -O(alkyle en C₁-C₆) ; un groupe aryle ; un groupe hétéroaryle ; un groupe -(C=NH)NHCOO(alkyle en C₁-C₆); un groupe -(C=NH)NH₂ ou un groupe -(alkyle en C₁-C₆)NR¹⁰R¹¹ dans lequel **R¹⁰** et **R¹¹** représentent indépendamment l'un de l'autre un groupe alkyle en C₁-C₆ ;
- un groupe(alkyle en C₁-C₆)COmorpholine ;
- un groupe C(=O)R¹² dans lequel **R¹²** représente un groupe -O(alkyle en C₁-C₆) ; un groupe alkyle en C₁-C₆ éventuellement substitué par un groupe -OH ; un groupe morpholine ; un groupe NH-aryle dans lequel le groupe aryle est éventuellement substitué par un groupe -COOH ou -COO(alkyle en C₁-C₆) ; ou un groupe -NR¹³R¹⁴ dans lequel **R¹³** et **R¹⁴** représentent indépendamment l'un de l'autre un groupe alkyle en C₁-C₆ ;
- un groupe (alkyle en C₁-C₆)aryle, dans lequel le groupe aryle est éventuellement substitué par un groupe -CN, COOH ou -COO(alkyle en C₁-C₆) ;
- un groupe (alkyle en C₁-C₆)hétéroaryle ;
- un groupe aryle, avantageusement phényle, dans lequel le groupe aryle est éventuellement substitué par un ou plusieurs groupes choisi parmi - COOH, -COO(alkyle en C₁-C₆), alkyle en C₁-C₆ substitué par un groupe - OH, -CN, -CONHOH, -NHSO₂(alkyle en C₁-C₆) ou -CONH-(alkyle en C₁-C₆)NR¹⁵R¹⁶ dans lequel **R¹⁵** et **R¹⁶** représentent indépendamment l'un de l'autre un groupe alkyle en C₁-C₆ ;
- un groupe hétéroaryle;
- un groupe hétérocyclique pouvant éventuellement comporter une insaturation;
- un groupe lactone de 3 à 6 membres, éventuellement substitué par un ou plusieurs groupes alkyles en C₁-C₆ ;
- ou un groupe -SO₂(alkyle en C₁-C₆) ;
**R³** représente un groupe aryle, avantageusement phényle, ou hétéroaryle, dans lequel le groupe aryle est éventuellement substitué par un ou plusieurs groupes choisi parmi -alkyle en C₁-C₆ dans lequel le groupe alkyle est éventuellement substitué par un ou plusieurs atomes d'halogène avantageusement F ou par un groupe -CN; un atome d'halogène avantageusement F, - O(alkyle en C₁-C₆) dans lequel le groupe alkyle est éventuellement substitué par un ou plusieurs atomes d'halogène avantageusement F ; -CN ; -OH ; -NO₂ ;-COOH ; NR¹⁷R¹⁸ dans lequel **R¹⁷** et **R¹⁸** représentent indépendamment l'un de l'autre un groupe alkyle en C₁-C₆; ou -NHCO-(alkyle en C₁-C₆) ;
ou un énantiomère, diastéréoisomère, hydrate, solvate, tautomère, mélange racémique ou sel pharmaceutiquement acceptable de celui-ci.

2. Dérivé d'imidazopyridine selon la revendication 1 **caractérisé en ce que Y** représente un atome d'oxygène ou de soufre ou le groupe -NH ou - NMe, avantageusement un atome d'oxygène ;

3. Dérivé d'imidazopyridine selon l'une quelconque des revendications 1 ou 2 **caractérisé en ce que R¹ R^{a}, R^{b}** et **R^{c}** représentent un atome d'hydrogène.

4. Dérivé d'imidazopyridine selon l'une quelconque des revendications 1 à 3 **caractérisé en ce que R²** représente
- un groupe (alkyle en C₁-C₆)COOR⁸ dans lequel **R⁸** représente un atome d'hydrogène ou un groupe alkyle en C₁-C₆ ;
- un groupe (alkyle en C₁-C₆)CONHR⁹ dans lequel **R⁹** représente un groupe -OH ; un groupe -O(alkyle en C₁-C₆) ; ou un groupe -(alkyle en C₁-C₆)NR¹⁰R¹¹ dans lequel **R¹⁰** et **R¹¹** représentent indépendamment l'un de l'autre un groupe alkyle en C₁-C₆ ;
- un groupe aryle, avantageusement phényle, dans lequel le groupe aryle est éventuellement substitué par un ou plusieurs groupes choisi parmi - COOH, -COO(alkyle en C₁-C₆), -CONHOH, ou -CONH-(alkyle en C₁-C₆)NR¹⁵R¹⁶ dans lequel **R¹⁵** et **R¹⁶** représentent indépendamment l'un de l'autre un groupe alkyle en C₁-C₆ ; ou
- un groupe -CONH aryle éventuellement substitué par un groupe -COOH ou -COO(alkyle en C₁-C₆).

5. Dérivé d'imidazopyridine selon la revendication 4 **caractérisé en ce que R²** représente un groupe -(alkyle en C₁-C₆)COOH, avantageusement - CH₂COOH.

6. Dérivé d'imidazopyridine selon l'une quelconque des revendications 1 à 5 **caractérisé en ce que R³** représente un groupe aryle, avantageusement phényle, éventuellement substitué par un ou plusieurs groupes choisi parmi -alkyle en C₁-C₆ dans lequel le groupe alkyle est éventuellement substitué par un ou plusieurs atomes d'halogène avantageusement F; un atome d'halogène avantageusement F, - O(alkyle en C₁-C₆) dans lequel le groupe alkyle est éventuellement substitué par un ou plusieurs atomes d'halogène avantageusement F ; -CN ; ou -NO₂.

7. Dérivé d'imidazopyridine selon la revendication 6 **caractérisé en ce que R³** représente un groupe phényle substitué par un ou plusieurs groupes choisi parmi -alkyle en C₁-C₆ substitué par un ou plusieurs atomes d'halogène, avantageusement F; un atome d'halogène avantageusement F, - O(alkyle en C₁-C₆) dans lequel le groupe alkyle est substitué par un ou plusieurs atomes d'halogène, avantageusement F ; ou -CN.

8. Dérivé d'imidazopyridine selon l'une quelconque des revendications 1 à 7 **caractérisé en ce qu'**il est choisi parmi les composés suivants :
Acide 2-(((3-((3-(trifluorométhyl)phényl)éthynyl)imidazo[1,2-a]pyridin-2-yl)méthyl)amino) acétique **(68)** ;
Acide 2-((3-((2-fluorophényl)éthynyl)imidazo[1,2-a]pyridin-2-yl)méthoxy) acétique **(42)** ;
Acide 2-((3-((3-(difluorométhoxy)phényl)éthynyl)imidazo[1,2-a]pyridin-2-yl)méthoxy) acétique **(35)** ;
Acide 2-((3-((3-(trifluorométhoxy)phényl)éthynyl)imidazo[1,2-a]pyridin-2-yl)méthoxy) acétique **(29)** ;
Acide 2-((3-((3-(trifluorométhyl)phényl)éthynyl)imidazo[1,2-a]pyridin-2-yl)méthoxy) benzoïque **(89)** ;
2-((3-((3-(trifluorométhyl)phényl)éthynyl)imidazo[1,2-a]pyridin-2-yl)méthoxy) acétate de sodium **(51)** ;
Acide 2-((3-((3,4-difluorophényl)éthynyl)imidazo[1,2-a]pyridin-2-yl)méthoxy) acétique **(38)** ;
Acide 2-((3-((3-cyanophényl)éthynyl)imidazo[1,2-a]pyridin-2-yl)méthoxy) acétique (**27**) ;
Acide 2-((3-((3-fluoro-4-(trifluorométhoxy)phényl)éthynyl)imidazo[1,2-a]pyridin-2-yl)méthoxy) acétique (**39**) ;
Acide 2-((3-((3-fluorophényl)éthynyl)imidazo[1,2-a]pyridin-2-yl)méthoxy) acétique (**40**) ;
Acide 2-((3-((3-méthoxyphényl)éthynyl)imidazo[1,2-a]pyridin-2-yl)méthoxy) acétique (**30**) ;
Acide 2-((3-((4-(trifluorométhoxy)phényl)éthynyl)imidazo[1,2-a]pyridin-2-yl)méthoxy)acétique (**32**) ;
Acide 2-((3-((4-fluorophényl)éthynyl)imidazo[1,2-a]pyridin-2-yl)méthoxy) acétique (**44**) ;
Acide 2-((3-((4-fluorophényl)éthynyl)imidazo[1,2-a]pyridin-2-yl)méthoxy) benzoïque (**88**) ;
2-((3-((4-fluorophényl)éthynyl)imidazo[1,2-a]pyridin-2-yl)méthoxy)-N-hydroxyacétamide (**59**₎ ;
2-((3-((4-fluorophényl)éthynyl)imidazo[1,2-a]pyridin-2-yl)méthoxy)-N-hydroxybenzamide (**96**) ;
2-((3-((4-nitrophényl)éthynyl)imidazo[1,2-a]pyridin-2-yl)méthoxy) acétate de sodium (**48**) ;
2-((3-(phényléthynyl)imidazo[1,2-a]pyridin-2-yl)méthoxy) acétate de sodium (**45**) ;
2-((3-(phényléthynyl)imidazo[1,2-a]pyridin-2-yl)méthoxy) benzoate de sodium (**92**) ;
2-((3-(p-tolyléthynyl)imidazo[1,2-a]pyridin-2-yl)méthoxy) acétate de sodium(**47**); Acide 2-(méthyl((3-((3-(trifluorométhyl)phényl)éthynyl)imidazo[1,2-a]pyridin-2-yl)méthyl)amino) acétique (**73**) ;
Acide 3-((3-(phényléthynyl)imidazo[1,2-a]pyridin-2-yl)méthoxy) benzoïque **(93)** ; Acide 3-(3-((3-((3-(trifluorométhyl)phényl)éthynyl)imidazo[1,2-a]pyridin-2-yl)méthyl)ureido) benzoïque (**75**) ;
Acide 4-((3-((3-(trifluorométhyl)phényl)éthynyl)imidazo[1,2-a]pyridin-2-yl)méthoxy) benzoïque (**90**) ;
2-((3-((2-(trifluorométhyl)phényl)éthynyl)imidazo[1,2-a]pyridin-2-yl)méthoxy) acétate d'éthyle (**26**) ;
2-((3-((2-fluorophényl)éthynyl)imidazo[1,2-a]pyridin-2-yl)méthoxy) acétate d'éthyle (**25**) ;
2-((3-((3-(difluorométhoxy)phényl)éthynyl)imidazo[1,2-a]pyridin-2-yl)méthoxy) acétate d'éthyle (**20**) ;
2-((3-((3-(trifluorométhoxy)phényl)éthynyl)imidazo[1,2-a]pyridin-2-yl)méthoxy) acétate d'éthyle (**18**) ;
2-((3-((3-(trifluorométhyl)phényl)éthynyl)imidazo[1,2-a]pyridin-2-yl)méthoxy) acétate d'éthyle (**7**) ;
2-((3-((3,4-difluorophényl)éthynyl)imidazo[1,2-a]pyridin-2-yl)méthoxy) acétate d'éthyle (**10**) ;
2-((3-((3-cyanophényl)éthynyl)imidazo[1,2-a]pyridin-2-yl)méthoxy) acétate d'éthyle (**14**) ;
2-((3-((3-fluoro-4-(trifluorométhoxy)phényl)éthynyl)imidazo[1,2-a]pyridin-2-yl)méthoxy) acétate d'éthyle (**11**) ;
2-((3-((3-fluorophényl)éthynyl)imidazo[1,2-a]pyridin-2-yl)méthoxy) acétate d'éthyle (**23**) ;
2-((3-((3-méthoxyphényl)éthynyl)imidazo[1,2-a]pyridin-2-yl)méthooy) acétate d'éthyle (**12**) ;
2-((3-((4-(trifluorométhoxy)phényl)éthynyl)imidazo[1,2-a]pyridin-2-yl)méthoxy) acétate d'éthyle (**17**) ;
2-((3-((4-fluorophényl)éthynyl)imidazo[1,2-a]pyridin-2-yl)méthoxy) acétate d'éthyle (**3**) ;
2-((3-((4-nitrophényl)éthynyl)imidazo[1,2-a]pyridin-2-yl)méthoxy) acétate d'éthyle (**5**) ;
2-((3-(phényléthynyl)imidazo[1,2-a]pyridin-2-yl)méthoxy) acétate d'éthyle (**1**) ;
2-((3-(p-tolyléthynyl)imidazo[1,2-a]pyridin-2-yl)méthoxy) acétate d'éthyle **(2)** ; 2-((3-((4-fluorophényl)éthynyl)imidazo[1,2-a]pyridin-2-yl)méthoxy) acétate d'isopropyle (**54**) ;
2-((3-((3-(trifluorométhyl)phényl)éthynyl)imidazo[1,2-a]pyridin-2-yl)méthoxy) benzoate de méthyle (**77**) ;
2-((3-((3-cyanophényl)éthynyl)imidazo[1,2-a]pyridin-2-yl)méthoxy) benzoate de méthyle (**79**) ;
2-((3-((4-fluorophényl)éthynyl)imidazo[1,2-a]pyridin-2-yl)méthoxy) benzoate de méthyle (**76**) ;
2-((3-(phényléthynyl)imidazo[1,2-a]pyridin-2-yl)méthoxy) benzoate de méthyle **(78)** ;
2-(méthyl((3-((3-(trifluorométhyl)phényl)éthynyl)imidazo[1,2-a]pyridin-2-yl)méthyl)amino) acétate d'éthyle (**72**) ;
3-((3-((3-(trifluorométhyl)phényl)éthynyl)imidazo[1,2-a]pyridin-2-yl)méthoxy) benzoate de méthyle (**81**) ;
3-((3-(phényléthynyl)imidazo[1,2-a]pyridin-2-yl)méthoxy) benzoate de méthyle (**87**) ;
3-(3-((3-((3-(trifluorométhyl)phényl)éthynyl)imidazo[1,2-a]pyridin-2-yl)méthyl)ureido) benzoate de méthyle (**74**) ;
4-((3-((3-(trifluorométhyl)phényl)éthynyl)imidazo[1,2-a]pyridin-2-yl)méthoxy) benzoate de méthyle (**83**) ;
N-(2-(diméthylamino)éthyl)-2-((3-((3-(trifluorométhyl)phényl)éthynyl)imidazo [1,2-a]pyridin-2-yl)méthoxy) acétamide (**55**) ;
N-(2-(diméthylamino)éthyl)-2-((3-((4-fluorophényl)éthynyl)imidazo[1,2-a]pyridin-2-yl)méthoxy) acétamide (**57**) ;
N-(2-(diméthylamino)éthyl)-2-((3-((4-fluorophényl)éthynyl)imidazo[1,2-a]pyridin-2-yl)méthoxy)benzamide (**95**) ;
N-éthoxy-2-((3-((4-fluorophényl)éthynyl)imidazo[1,2-a]pyridin-2-yl)méthoxy) acétamide (**58**) ;
2-((3-((4-fluorophényl)éthynyl)imidazo[1,2-a]pyridin-2-yl)méthoxy) acétate de sodium (**46**) ;
chlorhydrate de 2-[méthyl-[[3-[2-[3-(trifluorométhyl)phényl]éthynyl]imidazo[1,2-a]pyridin-2-yl]méthyl]amino]acétate d'éthyle (**101**) ;
2-[[6-chloro-3-[2-[3-(trifluorométhyl)phényl]éthynyl]imidazo[1,2-a]pyridin-2-yl]méthyl-méthyl-amino]acétate d'éthyle (**102**) ;
2-(méthyl((3-((3-(trifluorométhoxy)phényl)éthynyl)imidazo[1,2-a]pyridin-2-yl)méthyl)amino)acétate d'éthyle (**103**) ;
Chlorhydrate de 2-(((6-chloro-3-((3-(trifluorométhoxy)phényl)éthynyl)imidazo [1,2-a]pyridin-2-yl)méthyl)(méthyl)amino)acétate d'éthyle (**104**) ;
Acide 2-(((6-chloro-3-((3-(trifluorométhoxy)phényl)éthynyl)imidazo[1,2-a]pyridin-2-yl)méthyl)(méthyl)amino)acétique (**105**) ;
Acide 2-(méthyl((3-((3-(trifluorométhoxy)phényl)éthynyl)imidazo[1,2-a]pyridin-2-yl)méthyl)amino)acétique (**106**) ;
Acide 2-(((6-chloro-3-((3-(trifluorométhyl)phényl)éthynyl)imidazo[1,2-a]pyridin-2-yl)méthyl)(méthyl)amino)acétique (**107**) ;
2-[[3-[2-[3-(trifluorométhyl)phényl]éthynyl]imidazo[1,2-a]pyridin-2-yl] méthylsulfanyl]acétate d'éthyle (**108**) ;
2-[[3-[2-[3-(trifluorométhoxy)phényl]éthynyl]imidazo[1,2-a]pyridin-2-yl] méthylsulfanyl]acétate d'éthyle (**109**) ;
2-[[6-chloro-3-[2-[4-(fluoro)phényl]éthynyl]imidazo[1,2-a]pyridin-2-yl] méthylsulfanyl]acétate d'éthyle (**110**) ;
2-[[6-chloro-3-[2-[3-(trifluorométhyl)phényl]éthynyl]imidazo[1,2-a]pyridin-2-yl] méthylsulfanyl]acétate d'éthyle (**111**) ;
2-[[6-chloro-3-[2-[3-(trifluorométhoxy)phényl]éthynyl]imidazo[1,2-a]pyridin-2-yl] méthylsulfanyl]acétate d'éthyle (**112**) ;
(2R)-2-amino-3-[[3-[2-[3-(trifluorométhyl)phényl]éthynyl]imidazo[1,2-a]pyridin-2-yl]méthylsulfanyl]propanoate d'éthyle (**113**) ;
2-[[8-méthoxy-3-[2-[3-(trifluorométhyl)phényl]éthynyl]imidazo[1,2-a]pyridin-2-yl]méthylsulfanyl]acétate d'éthyle (**114**) ;
Acide 2-[[3-[2-[3-(trifluorométhyl)phényl]éthynyl]imidazo[1,2-a]pyridin-2-yl]méthylsulfanyl]acétique (**115**) ;
Acide 2-[[3-[2-[3-(trifluorométhoxy)phényl]éthynyl]imidazo[1,2-a]pyridin-2-yl]méthylsulfanyl]acétique (**116**) ;
Acide 2-[[6-chloro-3-[2-(4-fluorophényl)éthynyl]imidazo[1,2-a]pyridin-2-yl] méthylsulfanyl]acétique (**117**) ;
Acide 2-[[6-chloro-3-[2-[3-(trifluorométhyl)phényl]éthynyl]imidazo[1,2-a]pyridin-2-yl]méthylsulfanyl]acétique (**118**) ;
Acide 2-[[6-chloro-3-[2-[3-(trifluorométhoxy)phényl]éthynyl]imidazo[1,2-a] pyridin-2-yl]méthylsulfanyl]acétique (**119**) ;
Acide 2-[[8-méthoxy-3-[2-[3-(trifluorométhyl)phényl]éthynyl]imidazo[1,2-a] pyridin-2-yl]méthylsulfanyl]acétique (**120**) ;
2-[[3-[2-[3-(trifluorométhyl)phényl]éthynyl]imidazo[1,2-a]pyridin-2-yl] méthylsulfonyl]acétate d'éthyle (**121**) ;
2-[[6-chloro-3-[2-[3-(trifluorométhoxy)phényl]éthynyl]imidazo[1,2-a]pyridin-2-yl] méthylsulfonyl]acétate d'éthyle (**122**) ;
Acide 2-[[6-chloro-3-[2-[3-(trifluorométhoxy)phényl]éthynyl]imidazo[1,2-a] pyridin-2-yl]méthylsulfonyl]acétique (**123**) ;
N-(3-pyridyl)-2-[[3-[2-[3-(trifluorométhyl)phényl]éthynyl]imidazo[1,2-a]pyridin-2-yl]méthylsulfanyl]acétamide (**124**) ;
N-(3-pyridyl)-2-[[3-[2-[3-(trifluorométhoxy)phényl]éthynyl]imidazo[1,2-a]pyridin-2-yl]méthoxy]acétamide (**125**) ;
N-(3-pyridyl)-2-[[3-[2-[3-(trifluorométhoxy)phényl]éthynyl]imidazo[1,2-a]pyridin-2-yl]méthylsulfanyl]acétamide (**126**) ;
Acide 4-hydroxy-3,3-diméthyl-2-[[3-[2-[3-(trifluorométhyl)phényl]éthynyl]imidazo [1,2-a]pyridin-2-yl]méthoxy]butanoïque (**129**) ;
2-[[8-méthoxy-3-[2-[3-(trifluorométhyl)phényl]éthynyl]imidazo[1,2-a]pyridin-2-yl]méthylsulfanyl]éthanol (**130**) ;
2-[[6-chloro-3-[2-[3-(trifluorométhyl)phényl]éthynyl]imidazo[1,2-a]pyridin-2-yl] méthylsulfanyl]éthanol (**131**) ;
Chlorhydrate de N-carbamimidoyl-2-[[3-[2-[3-(trifluorométhyl)phényl]éthynyl] imidazo[1,2-a]pyridin-2-yl]méthylsulfanyl]acétamide (**135**)

9. Composition pharmaceutique comprenant un dérivé selon l'une quelconque des revendications 1 à 8 et un excipient pharmaceutiquement acceptable.

10. Composition pharmaceutique selon la revendication 9 comprenant en outre un autre agent antidiabétique, en particulier la metformine.

11. Dérivé selon l'une quelconque des revendications 1 à 8 pour utilisation à titre de médicament.

12. Dérivé selon l'une quelconque des revendications 1 à 8 pour utilisation à titre de médicament destiné au traitement et/ou à la prévention du diabète, de ses complications et/ou des pathologies associées, avantageusement du diabète de type 2 ou de l'hyperglycémie.

## Patentansprüche

1. Imidazopyridin-Derivat der folgenden allgemeinen Formel I: 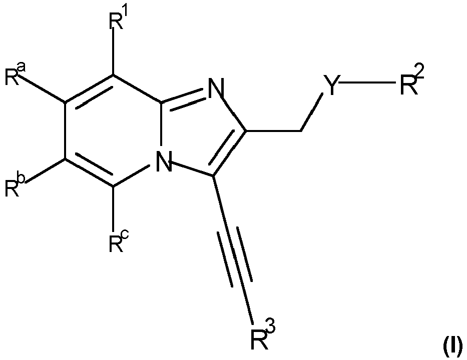 worin:
Y ein Sauerstoff- oder Schwefelatom, die Gruppe SO, SO₂ oder -NR¹⁹ darstellt, bei der **R¹⁹** ein Wasserstoffatom oder eine C₁-C₆-Alkylgruppe darstellt;
**R¹** R^{a}, R^{b} und R^{c} unabhängig voneinander ein Wasserstoffatom; ein Halogenatom; eine C₁-C₆-Alkylgruppe, eventuell substituiert durch eine -OH-Gruppe; eine -OH-Gruppe; eine -O(C₁-C₆-Alkyl)-Gruppe; eine -O(C₁-C₆-Alkyl)O(C₁-C₆-Alkyl)-Gruppe; eine -CN-Gruppe; eine -NR⁴R⁵-Gruppe, bei der **R⁴** und **R⁵** unabhängig voneinander ein Wasserstoffatom oder eine C₁-C₆-Alkylgruppe darstellen; oder eine -(C₁-C₆-Alkyl)NR⁶R⁷-Gruppe darstellen, bei der **R⁶** und **R⁷** unabhängig voneinander ein Wasserstoffatom oder eine C₁-C₆-Alkylgruppe darstellen;
**R²** folgendes darstellt
- ein Wasserstoffatom;
- eine C₁-C₆-Alkylgruppe, substituiert durch eine -OH-Gruppe;
- eine (C₁-C₆-Alkyl)COOR⁸-Gruppe, bei der **R⁸** ein Wasserstoffatom oder eine C₁-C₆-Alkylgruppe darstellt, wobei die Alkylgruppe durch eine -NH₂- oder -OH-Gruppe substituiert sein kann;
- eine (C₁-C₆-Alkyl)CONHR⁹-Gruppe, bei der **R⁹** eine -OH-Gruppe darstellt; eine C₁-C₆-Alkylgruppe; eine -O(C₁-C₆-Alkyl)-Gruppe; eine Arylgruppe; eine Heteroarylgruppe; eine -(C=NH)NHCOO(C₁-C₆-Alkyl)-Gruppe; eine -(C=NH)NH₂-Gruppe oder eine -(C₁-C₆-Alkyl)NR¹⁰R¹¹-Gruppe, bei der **R¹⁰** und **R¹¹** unabhängig voneinander eine C₁-C₆-Alkylgruppe darstellen;
- eine (C₁-C₆-Alkyl)COMorpholin-Gruppe;
- eine C(=O)R¹²-Gruppe, bei der **R¹²** eine -O(C₁-C₆-Alkyl)-Gruppe darstellt; eine C₁-C₆-Alkylgruppe, eventuell substituiert durch eine -OH-Gruppe; eine Morpholin-Gruppe; eine NH-Arylgruppe, bei der die Arylgruppe eventuell durch eine -COOH-oder -COO(C₁-C₆-Alkyl)-Gruppe substituiert ist; oder eine -NR¹³R¹⁴ -Gruppe, bei der **R¹³** und **R¹⁴** unabhängig voneinander eine C₁-C₆-Alkylgruppe darstellen;
- eine (C₁-C₆-Alkyl)Aryl-Gruppe, bei der die Arylgruppe eventuell durch eine -CN-, COOH- oder -COO(C₁-C₆-Alkyl)-Gruppe substituiert ist;
- eine (C₁-C₆-Alkyl)Heteroaryl-Gruppe;
- eine Aryl-, vorteilhafterweise Phenylgruppe, wobei die Arylgruppe eventuell durch eine oder mehrere Gruppe substituiert ist, die ausgewählt ist aus -COOH, -COO(C₁-C₆-Alkyl), C₁-C₆-Alkyl, substituiert durch eine Gruppe -OH, -CN, -CONHOH, -NHSO₂(C₁-C₆-Alkyl) oder -CONH-(C₁-C₆-Alkyl)NR¹⁵R¹⁶, bei der **R¹⁵** und **R¹⁶** unabhängig voneinander eine C₁-C₆-Alkylgruppe darstellen;
- eine Heteroarylgruppe;
- eine heterozyklische Gruppe, die eventuell eine Unsättigung enthalten kann;
- eine 3- bis 6-gliedrige Lactongruppe, die eventuell durch eine oder mehrere C₁-C₆-Alkylgruppen substituiert ist;
- oder eine -SO₂(C₁-C₆-Alkyl)-Gruppe;
**R³** eine Aryl-, vorteilhafterweise Phenyl-, oder Heteroarylgruppe, wobei die Arylgruppe eventuell durch eine oder mehrere Gruppen substituiert ist, die ausgewählt ist aus -C₁-C₆-Alkyl, wobei die Alkylgruppe eventuell durch ein oder mehrere Halogenatome, vorteilhafterweise F oder durch eine -CN-Gruppe substituiert ist; ein Halogenatom, vorteilhafterweise F, -O(C₁-C₆-Alkyl), wobei die Alkylgruppe eventuell durch ein oder mehrere Halogenatome, vorteilhafterweise F substituiert ist; -CN; -OH; -NO₂;-COOH; NR¹⁷R¹⁸, wobei **R¹⁷** und **R¹⁸** unabhängig voneinander eine C₁-C₆-Alkylgruppe darstellen; oder-NHCO-(C₁-C₆-Alkyl) darstellt;
oder ein Enantiomer, Diastereoisomer, Hydrat, Solvat, Tautomer, eine racemische Mischung oder ein pharmazeutisch akzeptables Salz davon.

2. Imidazopyridin-Derivat nach Anspruch 1, **dadurch gekennzeichnet, dass Y** ein Sauerstoff- oder Schwefelatom oder die Gruppe -NH oder -NMe, vorteilhafterweise ein Sauerstoffatom darstellt.

3. Imidazopyridin-Derivat nach einem der Ansprüche 1 oder 2, **dadurch gekennzeichnet, dass R¹ R^{a}, R^{b}** und **R^{c}** ein Wasserstoffatom darstellen.

4. Imidazopyridin-Derivat nach einem der Ansprüche 1 bis 3, **dadurch gekennzeichnet, dass R²** folgendes darstellt
- eine (C₁-C₆-Alkyl)COOR⁸-Gruppe, bei der **R⁸** ein Wasserstoffatom oder eine C₁-C₆-Alkylgruppe darstellt;
- eine (C₁-C₆-Alkyl)CONHR⁹-Gruppe, bei der **R⁹** eine -OH-Gruppe darstellt; eine -O(C₁-C₆-Alkyl)-Gruppe; oder eine -(C₁-C₆-Alkyl)NR¹⁰R¹¹-Gruppe, bei der **R¹⁰** und **R¹¹** unabhängig voneinander eine C₁-C₆-Alkylgruppe darstellen;
- eine Aryl-, vorteilhafterweise Phenylgruppe, wobei die Arylgruppe eventuell durch eine oder mehrere Gruppe substituiert ist, die ausgewählt ist aus -COOH, -COO(C₁-C₆-Alkyl),-CONHOH,oder-CONH-(C₁-C₆-Alkyl)NR¹⁵R¹⁶ bei der **R¹⁵** und **R¹⁶** unabhängig voneinander eine C₁-C₆-Alkylgruppe darstellen; oder
- eine -CONH Aryl-Gruppe, eventuell substituiert durch eine -COOH- oder -COO(C₁-C₆-Alkyl)-Gruppe.

5. Imidazopyridin-Derivat nach Anspruch 4, **dadurch gekennzeichnet, dass R²** eine -(C₁-C₆-Alkyl)COOH Gruppe, vorteilhafterweise -CH₂COOH darstellt.

6. Imidazopyridin-Derivat nach einem der Ansprüche 1 bis 5, **dadurch gekennzeichnet, dass R³** eine Aryl-, vorteilhafterweise Phenylgruppe, eventuell substituiert durch eine oder mehrere Gruppen ausgewählt aus -C₁-C₆-Alkyl, wobei die Alkylgruppe eventuell durch ein oder mehrere Halogenatome, vorteilhafterweise F substituiert ist; ein Halogenatom, vorteilhafterweise F, -O(C₁-C₆-Alkyl), wobei die Alkylgruppe eventuell durch ein oder mehrere Halogenatome, vorteilhafterweise F substituiert ist; -CN; oder-NO₂ darstellt.

7. Imidazopyridin-Derivat nach Anspruch 6, **dadurch gekennzeichnet, dass R³** eine Phenylgruppe, substituiert durch eine oder mehrere Gruppen ausgewählt aus - C₁-C₆-Alkyl, substituiert durch ein oder mehrere Halogenatome, vorteilhafterweise F; ein Halogenatom, vorteilhafterweise F, -O(C₁-C₆-Alkyl), wobei die Alkylgruppe durch ein oder mehrere Halogenatome, vorteilhafterweise F substituiert ist; oder-CN darstellt.

8. Imidazopyridin-Derivat nach einem der Ansprüche 1 bis 7, **dadurch gekennzeichnet, dass** es aus den folgenden Verbindungen ausgewählt ist:
2-(((3-((3-(Trifluormethyl)phenyl)ethynyl)imidazo[1,2-a]pyridin-2-yl)methyl)amino) essigsäure **(68);**
2-((3-((2-Fluorphenyl)ethynyl)imidazo[1,2-a]pyridin-2-yl)methoxy) essigsäure **(42);** 2-((3-((3-(Difluormethoxy)phenyl)ethynyl)imidazo[1,2-a]pyridin-2-yl)methoxy) essigsäure **(35);**
2-((3-((3-(Trifluormethoxy)phenyl)ethynyl)imidazo[1,2-a]pyridin-2-yl)methoxy) essigsäure **(29);**
2-((3-((3-(Trifluormethyl)phenyl)ethynyl)imidazo[1,2-a]pyridin-2-yl)methoxy) benzoesäure **(89);**
2-((3-((3-(Trifluormethyl)phenyl)ethynyl)imidazo[1,2-a]pyridin-2-yl)methoxy) natriumacetat **(51);**
2-((3-((3,4-Difluorphenyl)ethynyl)imidazo[1,2-a]pyridin-2-yl)methoxy) essigsäure **(38);**
2-((3-((3-Cyanophenyl)ethynyl)imidazo[1,2-a]pyridin-2-yl)methoxy) essigsäure **(27);**
2-((3-((3-Fluor-4-(trifluormethoxy)phenyl)ethynyl)imidazo[1,2-a]pyridin-2-yl)methoxy) essigsäure **(39);**
2-((3-((3-Fluorphenyl)ethynyl)imidazo[1,2-a]pyridin-2-yl)methoxy) essigsäure **(40);**
2-((3-((3-Methoxyphenyl)ethynyl)imidazo[1,2-a]pyridin-2-yl)methoxy) essigsäure **(30);**
2-((3-((4-(Trifluormethoxy)phenyl)ethynyl)imidazo[1,2-a]pyridin-2-yl)methoxy) essigsäure **(32);**
2-((3-((4-Fluorphenyl)ethynyl)imidazo[1,2-a]pyridin-2-yl)methoxy) essigsäure **(44);**
2-((3-((4-Fluorphenyl)ethynyl)imidazo[1,2-a]pyridin-2-yl)methoxy) benzoesäure **(88);**
2-((3-((4-Fluorphenyl)ethynyl)imidazo[1,2-a]pyridin-2-yl)methoxy)-N-hydroxyacetamid **(59);**
2-((3-((4-Fluorphenyl)ethynyl)imidazo[1,2-a]pyridin-2-yl)methoxy)-N-hydroxybenzamid **(96);**
2-((3-((4-Nitrophenyl)ethynyl)imidazo[1,2-a]pyridin-2-yl)methoxy) natriumacetat **(48);**
2-((3-(Phenylethynyl)imidazo[1,2-a]pyridin-2-yl)methoxy) natriumacetat **(45);**
2-((3-(Phenylethynyl)imidazo[1,2-a]pyridin-2-yl)methoxy) natriumbenzoat **(92);**
2-((3-(p-Tolylethynyl)imidazo[1,2-a]pyridin-2-yl)methoxy) natriumacetat(**47**);
2-(Methyl((3-((3-(trifluormethyl)phenyl)ethynyl)imidazo[1,2-a]pyridin-2-yl)methyl)amino) essigsäure **(73);**
3-((3-(Phenylethynyl)imidazo[1,2-a]pyridin-2-yl)methoxy) benzoesäure **(93);**
3-(3-((3-((3-(Trifluormethyl)phenyl)ethynyl)imidazo[1,2-a]pyridin-2-yl)methyl)ureido) benzoesäure **(75);**
4-((3-((3-(Trifluormethyl)phenyl)ethynyl)imidazo[1,2-a]pyridin-2-yl)methoxy) benzoesäure **(90);**
2-((3-((2-(Trifluormethyl)phenyl)ethynyl)imidazo[1,2-a]pyridin-2-yl)methoxy) ethylacetat **(26);**
2-((3-((2-Fluorphenyl)ethynyl)imidazo[1,2-a]pyridin-2-yl)methoxy) ethylacetat **(25);**
2-((3-((3-(Difluormethoxy)phenyl)ethynyl)imidazo[1,2-a]pyridin-2-yl)methoxy) ethylacetat **(20);**
2-((3-((3-(Trifluormethoxy)phenyl)ethynyl)imidazo[1,2-a]pyridin-2-yl)methoxy) ethylacetat **(18);**
2-((3-((3-(Trifluormethyl)phenyl)ethynyl)imidazo[1,2-a]pyridin-2-yl)methoxy) ethylacetat **(7);**
2-((3-((3,4-Difluorphenyl)ethynyl)imidazo[1,2-a]pyridin-2-yl)methoxy) ethylacetat **(10);**
2-((3-((3-Cyanophenyl)ethynyl)imidazo[1,2-a]pyridin-2-yl)methoxy) ethylacetat **(14);**
2-((3-((3-Fluor-4-(trifluormethoxy)phenyl)ethynyl)imidazo[1,2-a]pyridin-2-yl)methoxy) ethylacetat **(11**);
2-((3-((3-Fluorphenyl)ethynyl)imidazo[1,2-a]pyridin-2-yl)methoxy) ethylacetat **(23);**
2-((3-((3-Methoxyphenyl)ethynyl)imidazo[1,2-a]pyridin-2-yl)methoxy) ethylacetat **(12);**
2-((3-((4-(Trifluormethoxy)phenyl)ethynyl)imidazo[1,2-a]pyridin-2-yl)methoxy) ethylacetat **(17);**
2-((3-((4-Fluorphenyl)ethynyl)imidazo[1,2-a]pyridin-2-yl)methoxy) ethylacetat **(3);**
2-((3-((4-Nitrophenyl)ethynyl)imidazo[1,2-a]pyridin-2-yl)methoxy) ethylacetat **(5);**
2-((3-(Phenylethynyl)imidazo[1,2-a]pyridin-2-yl)methoxy) ethylacetat **(1);**
2-((3-(p-Tolylethynyl)imidazo[1,2-a]pyridin-2-yl)methoxy) ethylacetat **(2);**
2-((3-((4-Fluorphenyl)ethynyl)imidazo[1,2-a]pyridin-2-yl)methoxy) isopropylacetat **(54);**
2-((3-((3-(Trifluormethyl)phenyl)ethynyl)imidazo[1,2-a]pyridin-2-yl)methoxy) methylbenzoat **(77);**
2-((3-((3-Cyanophenyl)ethynyl)imidazo[1,2-a]pyridin-2-yl)methoxy) methylbenzoat **(79);**
2-((3-((4-Fluorphenyl)ethynyl)imidazo[1,2-a]pyridin-2-yl)methoxy) methylbenzoat **(76);**
2-((3-(Phenylethynyl)imidazo[1,2-a]pyridin-2-yl)methoxy) methylbenzoat **(78);**
2-(Methyl((3-((3-(trifluormethyl)phenyl)ethynyl)imidazo[1,2-a]pyridin-2-yl)methyl)amino) ethylacetat **(72);**
3-((3-((3-(Trifluormethyl)phenyl)ethynyl)imidazo[1,2-a]pyridin-2-yl)methoxy) methylbenzoat **(81);**
3-((3-(Phenylethynyl)imidazo[1,2-a]pyridin-2-yl)methoxy) methylbenzoat **(87);**
3-(3-((3-((3-(Trifluormethyl)phenyl)ethynyl)imidazo[1,2-a]pyridin-2-yl)methyl)ureido) methylbenzoat **(74);**
4-((3-((3-(Trifluormethyl)phenyl)ethynyl)imidazo[1,2-a]pyridin-2-yl)methoxy) methylbenzoat **(83);**
N-(2-(Dimethylamino)ethyl)-2-((3-((3-(trifluormethyl)phenyl)ethynyl)imidazo [1,2-a]pyridin-2-yl)methoxy) acetamid **(55);**
N-(2-(Dimethylamino)ethyl)-2-((3-((4-fluorphenyl)ethynyl)imidazo[1,2-a]pyridin-2-yl)methoxy) acetamid **(57);**
N-(2-(Dimethylamino)ethyl)-2-((3-((4-fluorphenyl)ethynyl)imidazo[1,2-a]pyridin-2-yl)methoxy) benzamid **(95);**
N-Ethoxy-2-((3-((4-fluorphenyl)ethynyl)imidazo[1,2-a]pyridin-2-yl)methoxy) acetamid **(58);**
2-((3-((4-Fluorphenyl)ethynyl)imidazo[1,2-a]pyridin-2-yl)methoxy) natriumacetat **(46);**
2-[Methyl-[[3-[2-[3-(trifluormethyl)phenyl]ethynyl]imidazo[1,2-a]pyridin-2-yl]methyl]amino] ethylacetat-Hydrochlorid **(101);**
2-[[6-Chlor-3-[2-[3-(trifluormethyl)phenyl]ethynyl]imidazo[1,2-a]pyridin-2-yl]methyl-methyl-amino] ethylacetat **(102);**
2-(Methyl((3-((3-(trifluormethoxy)phenyl)ethynyl)imidazo[1,2-a]pyridin-2-yl)methyl)amino) ethylacetat **(103);**
2-(((6-Chlor-3-((3-(trifluormethoxy)phenyl)ethynyl)imidazo [1,2-a]pyridin-2-yl)methyl)(methyl)amino) ethylacetat-Hydrochlorid **(104);**
2-(((6-Chlor-3-((3-(trifluormethoxy)phenyl)ethynyl)imidazo[1,2-a]pyridin-2-yl)methyl)(methyl)amino) essigsäure **(105);**
2-(Methyl((3-((3-(trifluormethoxy)phenyl)ethynyl)imidazo[1,2-a]pyridin-2-yl)methyl)amino) essigsäure **(106);**
2-(((6-Chlor-3-((3-(trifluormethyl)phenyl)ethynyl)imidazo[1,2-a]pyridin-2-yl)methyl)(methyl)amino) essigsäure **(107);**
2-[[3-[2-[3-(Trifluormethyl)phenyl]ethynyl]imidazo[1,2-a]pyridin-2-yl] methylsulfanyl] ethylacetat **(108);**
2-[[3-[2-[3-(Trifluormethoxy)phenyl]ethynyl]imidazo[1,2-a]pyridin-2-yl] methylsulfanyl] ethylacetat **(109);**
2-[[6-Chlor-3-[2-[4-(fluor)phenyl]ethynyl]imidazo[1,2-a]pyridin-2-yl] methylsulfanyl] ethylacetat **(110);**
2-[[6-Chlor-3-[2-[3-(trifluormethyl)phenyl]ethynyl]imidazo[1,2-a]pyridin-2-yl] methylsulfanyl] ethylacetat **(111);**
2-[[6-Chlor-3-[2-[3-(trifluormethoxy)phenyl]ethynyl]imidazo[1,2-a]pyridin-2-yl] methylsulfanyl] ethylacetat **(112);**
(2R)-2-Amino-3-[[3-[2-[3-(trifluormethyl)phenyl]ethynyl]imidazo[1,2-a]pyridin-2-yl]methylsulfanyl] ethylpropanoat **(113);**
2-[[8-Methoxy-3-[2-[3-(trifluormethyl)phenyl]ethynyl]imidazo[1,2-a]pyridin-2-yl]methylsulfanyl] ethylacetat **(114);**
2-[[3-[2-[3-(Trifluormethyl)phenyl]ethynyl]imidazo[1,2-a]pyridin-2-yl]methylsulfanyl] essigsäure **(115);**
2-[[3-[2-[3-(Trifluormethoxy)phenyl]ethynyl]imidazo[1,2-a]pyridin-2-yl]methylsulfanyl] essigsäure **(116);**
2-[[6-Chlor-3-[2-(4-fluorphenyl)ethynyl]imidazo[1,2-a]pyridin-2-yl] methylsulfanyl] essigsäure **(117);**
2-[[6-Chlor-3-[2-[3-(trifluormethyl)phenyl]ethynyl]imidazo[1,2-a]pyridin-2-yl]methylsulfanyl] essigsäure **(118);**
2-[[6-Chlor-3-[2-[3-(trifluormethoxy)phenyl]ethynyl]imidazo[1,2-a] pyridin-2-yl]methylsulfanyl] essigsäure **(119);**
2-[[8-Methoxy-3-[2-[3-(trifluormethyl)phenyl]ethynyl]imidazo[1,2-a] pyridin-2-yl]methylsulfanyl] essigsäure **(120);**
2-[[3-[2-[3-(Trifluormethyl)phenyl]ethynyl]imidazo[1,2-a]pyridin-2-yl] methylsulfonyl] ethylacetat **(121**);
2-[[6-Chlor-3-[2-[3-(trifluormethoxy)phenyl]ethynyl]imidazo[1,2-a]pyridin-2-yl] methylsulfonyl] ethylacetat **(122);**
2-[[6-Chlor-3-[2-[3-(trifluormethoxy)phenyl]ethynyl]imidazo[1,2-a] pyridin-2-yl]methylsulfonyl] essigsäure **(123);**
N-(3-Pyridyl)-2-[[3-[2-[3-(trifluormethyl)phenyl]ethynyl]imidazo[1,2-a]pyridin-2-yl]methylsulfanyl] acetamid **(124);**
N-(3-Pyridyl)-2-[[3-[2-[3-(trifluormethoxy)phenyl]ethynyl]imidazo[1,2-a]pyridin-2-yl]methoxy] acetamid **(125);**
N-(3-Pyridyl)-2-[[3-[2-[3-(trifluormethoxy)phenyl]ethynyl]imidazo[1,2-a]pyridin-2-yl]methylsulfanyl] acetamid **(126);**
4-Hydroxy-3,3-dimethyl-2-[[3-[2-[3-(trifluormethyl)phenyl]ethynyl]imidazo [1,2-a]pyridin-2-yl]methoxy] butansäure **(129);**
2-[[8-Methoxy-3-[2-[3-(trifluormethyl)phenyl]ethynyl]imidazo[1,2-a]pyridin-2-yl]methylsulfanyl] ethanol **(130);**
2-[[6-Chlor-3-[2-[3-(trifluormethyl)phenyl]ethynyl]imidazo[1,2-a]pyridin-2-yl] methylsulfanyl] ethanol **(131);**
N-Carbamimidoyl-2-[[3-[2-[3-(trifluormethyl)phenyl]ethynyl] imidazo [1,2-a]pyridin-2-yl]methylsulfanyl] acetamid-Hydrochlorid **(135).**

9. Pharmazeutische Zusammensetzung, die ein Derivat nach einem der Ansprüche 1 bis 8 und einen pharmazeutisch akzeptablen Hilfsstoff umfasst.

10. Pharmazeutische Zusammensetzung nach Anspruch 9, die ferner ein weiteres Antidiabetikum, insbesondere Metformin, umfasst.

11. Derivat nach einem der Ansprüche 1 bis 8, für die Verwendung als Medikament.

12. Derivat nach einem der Ansprüche 1 bis 8 für die Verwendung als Medikament, das für die Behandlung und/oder die Prävention von Diabetes, ihren Komplikationen und/oder der assoziierten Pathologien, vorteilhafterweise von Diabetes Typ 2 oder von Hyperglykämie bestimmt ist.

## Claims

1. An imidazopyridine derivative of the following general formula I: 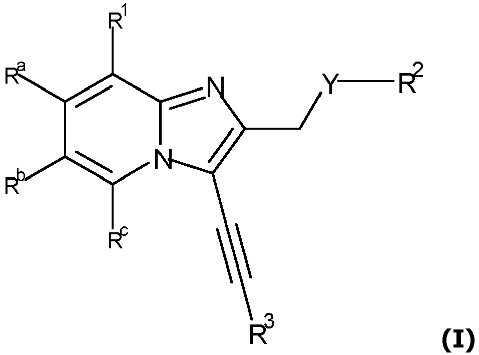 wherein:
**Y** represents an oxygen or sulfur atom, the group SO, SO₂ or -NR¹⁹, wherein **R¹⁹** represents a hydrogen atom ou a C₁-C₆ alkyl group;
**R¹**, **R^{a}, R^{b}** and **R^{c}** represent independently of each other a hydrogen atom; a halogen atom; a C₁-C₆ alkyl group optionally substituted with an - OH group; an -OH group; a -O(C₁-C₆ alkyl) group; a -O(C₁-C₆ alkyl)O(C₁-C₆ alkyl) group; a -CN group; a -NR⁴R⁵ group wherein **R⁴** and **R⁵** represent independently of each other a hydrogen atom or a C₁-C₆ alkyl group; or a - (C₁-C₆ alkyl)NR⁶R⁷ group wherein **R⁶** and **R⁷** represent independently of each other a hydrogen atom or a C₁-C₆ alkyl group;
**R²** represents
- a hydrogen atom;
- a C₁-C₆ alkyl group substituted with an -OH group;
- a (C₁-C₆ alkyl)COOR⁸ group wherein **R⁸** represents a hydrogen atom or a C₁-C₆ alkyl group, the alkyl group may be substituted with an -NH₂ or -OH group;
- a (C₁-C₆ alkyl)CONHR⁹ group wherein **R⁹** represents an -OH group; a C₁-C₆ alkyl group; an -O(C₁-C₆ alkyl) group; an aryl group; a heteroaryl group; a -(C=NH)NHCOO(C₁-C₆ alkyl) group; a -(C=NH)NH₂ group or a -(C₁-C₆ alkyl)NR¹⁰R¹¹ group wherein **R¹⁰** and **R¹¹** represent independently of each other a C₁-C₆ alkyl group;
- a (C₁-C₆ alkyl)COmorpholine group;
- a C(=O)R¹² group wherein **R¹²** represents an -O(C₁-C₆ alkyl) group; a C₁-C₆ alkyl group optionally substituted with an -OH group; a morpholine group; an NH-aryl group wherein the aryl group is optionally substituted with a-COOH or -COO(C₁-C₆ alkyl) group; or a-NR¹³R¹⁴ group wherein **R¹³** and **R¹⁴** represent independently of each other a C₁-C₆ alkyl group;
- a (C₁-C₆ alkyl)aryl group, wherein the aryl group is optionally substituted with a-CN, COOH or -COO(C₁-C₆ alkyl) group;
- a (C₁-C₆ alkyl)heteroaryl group;
- an aryl group, advantageously a phenyl, wherein the aryl group is optionally substituted with one or more groups selected from -COOH, - COO(C₁-C₆ alkyl), C₁-C₆ alkyl substituted with a -OH, -CN, -CONHOH, - NHSO₂(C₁-C₆ alkyl) or -CONH-( C₁-C₆ alkyl)NR¹⁵R¹⁶ group wherein **R¹⁵** and **R¹⁶** represent independently of each other a C₁-C₆ alkyl group;
- a heteroaryl group;
- a heterocyclic group which may optionally include an unsaturation;
- a lactone group with 3 to 6 members, optionally substituted with one or more C₁-C₆ alkyl groups;
- or a -SO₂(C₁-C₆ alkyl) group;
**R³** represents an aryl group, advantageously a phenyl, or heteroaryl group, wherein the aryl group is optionally substituted with one or more groups selected from -C₁-C₆ alkyl wherein the alkyl group is optionally substituted with one or more halogen atoms, advantageously F or with a - CN group; a halogen atom, advantageously F, -O(C₁-C₆ alkyl) wherein the alkyl group is optionally substituted with one or more halogen atoms, advantageously F; -CN; -OH; -NO₂;-COOH; NR¹⁷R¹⁸ wherein **R¹⁷** and **R¹⁸** represent independently of each other a C₁-C₆ alkyl group; or -NHCO-(C₁-C₆ alkyl);
or an enantiomer, diastereoisomer, hydrate, solvate, tautomer, racemic mixture or a pharmaceutically acceptable salt thereof.

2. The imidazopyridine derivative according to claim 1, **characterized in that Y** represents an oxygen or sulfur atom or the -NH or -Nme group, advantageously an oxygen atom;

3. The imidazopyridine derivative according to any of claims 1 or 2 **characterized in that R¹ R^{a}, R^{b}** and **R^{c}** represent a hydrogen atom.

4. The imidazopyridine derivative according to any of claims 1 to 3, **characterized in that R²** represents
- a (C₁-C₆ alkyl)COOR⁸ group wherein **R⁸** represents a hydrogen atom or a C₁-C₆ alkyl group;
- a (C₁-C₆ alkyl)CONHR⁹ group wherein **R⁹** represents an -OH group; an -O(C₁-C₆ alkyl) group; or a -(C₁-C₆ alkyl)NR¹⁰R¹¹ group wherein **R¹⁰** and **R¹¹** represent independently of each other a C₁-C₆ alkyl group;
- an aryl group, advantageously a phenyl, wherein the aryl group is optionally substituted with one or more groups selected from -COOH, -COO(C₁-C₆ alkyl), -CONHOH, or -CONH-(C₁-C₆ alkyl)NR¹⁵R¹⁶ wherein **R¹⁵** and **R¹⁶** represent independently of each other a C₁-C₆ alkyl group; or
- a -CONHaryl group optionally substituted with a-COOH or -COO(C₁-C₆ alkyl) group.

5. The imidazopyridine derivative according to claim 4, **characterized in that R²** represents a -(C₁-C₆ alkyl)COOH group, advantageously -CH₂COOH.

6. The imidazopyridine derivative according to any of claims 1 to 5 **characterized in that R³** represents an aryl group, advantageously phenyl, optionally substituted with one or more groups selected from -(C₁-C₆ alkyl) wherein the alkyl group is optionally substituted with one or more halogen atoms, advantageously F; a halogen atom, advantageously F, -O(C₁-C₆ alkyl) wherein the alkyl group is optionally substituted with one or more halogen atoms, advantageously F; -CN; or -NO₂.

7. The imidazopyridine derivative according to claim 6, **characterized in that R³** represents a phenyl group substituted with one or more groups selected from -(C₁-C₆ alkyl) substituted with one or more halogen atoms, advantageously F; a halogen atom, advantageously F, -O(C₁-C₆ alkyl) wherein the alkyl group is substituted with one or more halogen atoms, advantageously F; or -CN.

8. The imidazopyridine derivative according to any of claims 1 to 7, **characterized in that** it is selected from the following compounds:
2-(((3-((3-(trifluoromethyl)phenyl)ethynyl)imidazo[1,2-a]pyridin-2-yl)methyl)amino) acetic acid **(68);**
2-((3-((2-fluorophenyl)ethynyl)imidazo[1,2-a]pyridin-2-yl)methoxy) acetic acid **(42);**
2-((3-((3-(difluoromethoxy)phenyl)ethynyl)imidazo[1,2-a]pyridin-2-yl)methoxy) acetic acid **(35);**
2-((3-((3-(trifluoromethoxy)phenyl)ethynyl)imidazo[1,2-a]pyridin-2-yl)methoxy) acetic acid **(29);**
2-((3-((3-(trifluoromethyl)phenyl)ethynyl)imidazo[1,2-a]pyridin-2-yl)methoxy) benzoic acid **(89);**
sodium 2-((3-((3-(trifluoromethyl)phenyl)ethynyl)imidazo[1,2-a]pyridin-2-yl)methoxy) acetate **(51);**
2-((3-((3,4-difluorophenyl)ethynyl)imidazo[1,2-a]pyridin-2-yl)methoxy) acetic acid **(38);**
2-((3-((3-cyanophenyl)ethynyl)imidazo[1,2-a]pyridin-2-yl)methoxy) acetic acid **(27);**
2-((3-((3-fluoro-4-(trifluoromethoxy)phenyl)ethynyl)imidazo[1,2-a]pyridin-2-yl)methoxy) acetic acid **(39);**
2-((3-((3-fluorophenyl)ethynyl)imidazo[1,2-a]pyridin-2-yl)methoxy) acetic acid **(40);**
2-((3-((3-methoxyphenyl)ethynyl)imidazo[1,2-a]pyridin-2-yl)methoxy) acetic acid **(30);**
2-((3-((4-(trifluoromethoxy)phenyl)ethynyl)imidazo[1,2-a]pyridin-2-yl)methoxy) acetic acid **(32);**
2-((3-((4-fluorophenyl)ethynyl)imidazo[1,2-a]pyridin-2-yl)methoxy) acetic acid **(44);**
2-((3-((4-fluorophenyl)ethynyl)imidazo[1,2-a]pyridin-2-yl)methoxy) benzoic acid **(88);**
2-((3-((4-fluorophenyl)ethynyl)imidazo[1,2-a]pyridin-2-yl)methoxy)-N-hydroxyacetamide **(59);**
2-((3-((4-fluorophenyl)ethynyl)imidazo[1,2-a]pyridin-2-yl)methoxy)-N-hydroxybenzamide **(96);**
sodium 2-((3-((4-nitrophenyl)ethynyl)imidazo[1,2-a]pyridin-2-yl)methoxy) acetate **(48);**
sodium 2-((3-(phenylethynyl)imidazo[1,2-a]pyridin-2-yl)methoxy) acetate **(45);**
sodium 2-((3-(phenylethynyl)imidazo[1,2-a]pyridin-2-yl)methoxy) benzoate **(92);**
sodium 2-((3-(p-tolylethynyl)imidazo[1,2-a]pyridin-2-yl)methoxy) acetate **(47);**
2-(methyl((3-((3-(trifluoromethyl)phenyl)ethynyl)imidazo[1,2-a]pyridin-2-yl)methyl)amino) acetic acid **(73);**
3-((3-(phenylethynyl)imidazo[1,2-a]pyridin-2-yl)methoxy) benzoic acid **(93);**
3-(3-((3-((3-(trifluoromethyl)phenyl)ethynyl)imidazo[1,2-a]pyridin-2-yl)methyl)ureido) benzoic acid **(75);**
4-((3-((3-(trifluoromethyl)phenyl)ethynyl)imidazo[1,2-a]pyridin-2-yl)methoxy) benzoic acid **(90);**
ethyl 2-((3-((2-(trifluoromethyl)phenyl)ethynyl)imidazo[1,2-a]pyridin-2-yl)methoxy) acetate **(26);**
ethyl 2-((3-((2-fluorophenyl)ethynyl)imidazo[1,2-a]pyridin-2-yl)methoxy) acetate **(25);**
ethyl 2-((3-((3-(difluoromethoxy)phenyl)ethynyl)imidazo[1,2-a]pyridin-2-yl)methoxy) acetate **(20);**
ethyl 2-((3-((3-(trifluoromethoxy)phenyl)ethynyl)imidazo[1,2-a]pyridin-2-yl)methoxy) acetate **(18);**
ethyl 2-((3-((3-(trifluoromethyl)phenyl)ethynyl)imidazo[1,2-a]pyridin-2-yl)methoxy) acetate **(7);**
ethyl 2-((3-((3,4-difluorophenyl)ethynyl)imidazo[1,2-a]pyridin-2-yl)methoxy) acetate **(10);**
ethyl 2-((3-((3-cyanophenyl)ethynyl)imidazo[1,2-a]pyridin-2-yl)methoxy) acetate **(14);**
ethyl 2-((3-((3-fluoro-4-(trifluoromethoxy)phenyl)ethynyl)imidazo[1,2-a]pyridin-2-yl)methoxy) acetate **(11);**
ethyl 2-((3-((3-fluorophenyl)ethynyl)imidazo[1,2-a]pyridin-2-yl)methoxy) acetate **(23);**
ethyl 2-((3-((3-methoxyphenyl)ethynyl)imidazo[1,2-a]pyridin-2-yl)methoxy) acetate **(12);**
ethyl 2-((3-((4-(trifluoromethoxy)phenyl)ethynyl)imidazo[1,2-a]pyridin-2-yl)methoxy) acetate **(17);**
ethyl 2-((3-((4-fluorophenyl)ethynyl)imidazo[1,2-a]pyridin-2-yl)methoxy) acetate **(3);**
ethyl 2-((3-((4-nitrophenyl)ethynyl)imidazo[1,2-a]pyridin-2-yl)methoxy) acetate **(5);**
ethyl 2-((3-(phenylethynyl)imidazo[1,2-a]pyridin-2-yl)methoxy) acetate **(1);**
ethyl 2-((3-(p-tolylethynyl)imidazo[1,2-a]pyridin-2-yl)methoxy) acetate **(2);**
isopropyl 2-((3-((4-fluorophenyl)ethynyl)imidazo[1,2-a]pyridin-2-yl)methoxy) acetate **(54);**
methyl 2-((3-((3-(trifluoromethyl)phenyl)ethynyl)imidazo[1,2-a]pyridin-2-yl)methoxy) benzoate **(77);**
methyl 2-((3-((3-cyanophenyl)ethynyl)imidazo[1,2-a]pyridin-2-yl)methoxy) benzoate **(79);**
methyl 2-((3-((4-fluorophenyl)ethynyl)imidazo[1,2-a]pyridin-2-yl)methoxy) benzoate **(76);**
methyl 2-((3-(phenylethynyl)imidazo[1,2-a]pyridin-2-yl)methoxy) benzoate **(78);**
ethyl 2-(methyl((3-((3-(trifluoromethyl)phenyl)ethynyl)imidazo[1,2-a]pyridin-2-yl)methyl)amino) acetate **(72);**
methyl 3-((3-((3-(trifluoromethyl)phenyl)ethynyl)imidazo[1,2-a]pyridin-2-yl)methoxy) benzoate **(81);**
methyl 3-((3-(phenylethynyl)imidazo[1,2-a]pyridin-2-yl)methoxy) benzoate **(87);**
methyl 3-(3-((3-((3-(trifluoromethyl)phenyl)ethynyl)imidazo[1,2-a]pyridin-2-yl)methyl)ureido) benzoate **(74);**
methyl 4-((3-((3-(trifluoromethyl)phenyl)ethynyl)imidazo[1,2-a]pyridin-2-yl)methoxy) benzoate **(83);**
N-(2-(dimethylamino)ethyl)-2-((3-((3-(trifluoromethyl)phenyl)ethynyl)imidazo [1,2-a]pyridin-2-yl)methoxy) acetamide **(55);**
N-(2-(dimethylamino)ethyl)-2-((3-((4-fluorophenyl)ethynyl)imidazo[1,2-a]pyridin-2-yl)methoxy) acetamide **(57);**
N-(2-(dimethylamino)ethyl)-2-((3-((4-fluorophenyl)ethynyl)imidazo[1,2-a]pyridin-2-yl)methoxy)benzamide **(95);**
N-ethoxy-2-((3-((4-fluorophenyl)ethynyl)imidazo[1,2-a]pyridin-2-yl)methoxy) acetamide **(58);**
sodium 2-((3-((4-fluorophenyl)ethynyl)imidazo[1,2-a]pyridin-2-yl)methoxy) acetate **(46);**
ethyl 2-[methyl-[[3-[2-[3-(trifluoromethyl)phenyl]ethynyl]imidazo[1,2-a]pyridin-2-yl]methyl]amino]acetate hydrochloride **(101);**
ethyl 2-[[6-chloro-3-[2-[3-(trifluoromethyl)phenyl]ethynyl]imidazo[1,2-a]pyridin-2-yl]methyl-methyl-amino]acetate **(102);**
ethyl 2-(methyl((3-((3-(trifluoromethoxy)phenyl)ethynyl)imidazo[1,2-a]pyridin-2-yl)methyl)amino)acetate **(103);**
ethyl 2-(((6-chloro-3-((3-(trifluoromethoxy)phenyl)ethynyl)imidazo [1,2-a]pyridin-2-yl)methyl)(methyl)amino)acetate hydrochloride **(104);**
2-(((6-chloro-3-((3-(trifluoromethoxy)phenyl)ethynyl)imidazo[1,2-a]pyridin-2-yl)methyl)(methyl)amino)acetic acid **(105);**
2-(methyl((3-((3-(trifluoromethoxy)phenyl)ethynyl)imidazo[1,2-a]pyridin-2-yl)methyl)amino) acetic acid **(106);**
2-(((6-chloro-3-((3-(trifluoromethyl)phenyl)ethynyl)imidazo[1,2-a]pyridin-2-yl)methyl)(methyl)amino)acetic acid **(107);**
ethyl 2-[[3-[2-[3-(trifluoromethyl)phenyl]ethynyl]imidazo[1,2-a]pyridin-2-yl] methylsulfanyl]acetate **(108);**
ethyl 2-[[3-[2-[3-(trifluoromethoxy)phenyl]ethynyl]imidazo[1,2-a]pyridin-2-yl] methylsulfanyl]acetate **(109);**
ethyl 2-[[6-chloro-3-[2-[4-(fluoro)phenyl]ethynyl]imidazo[1,2-a]pyridin-2-yl] methylsulfanyl]acetate **(110);**
ethyl 2-[[6-chloro-3-[2-[3-(trifluoromethyl)phenyl]ethynyl]imidazo[1,2-a]pyridin-2-yl] methylsulfanyl]acetate **(111);**
ethyl 2-[[6-chloro-3-[2-[3-(trifluoromethoxy)phenyl]ethynyl]imidazo[1,2-a]pyridin-2-yl] methylsulfanyl]acetate **(112);**
ethyl (2R)-2-amino-3-[[3-[2-[3-(trifluoromethyl)phenyl]ethynyl]imidazo[1,2-a]pyridin-2-yl]methylsulfanyl]propanoate **(113);**
ethyl 2-[[8-methoxy-3-[2-[3-(trifluoromethyl)phenyl]ethynyl]imidazo[1,2-a]pyridin-2-yl]methylsulfanyl]acetate **(114);**
2-[[3-[2-[3-(trifluoromethyl)phenyl]ethynyl]imidazo[1,2-a]pyridin-2-yl]methylsulfanyl]acetic acid **(115);**
2-[[3-[2-[3-(trifluoromethoxy)phenyl]ethynyl]imidazo[1,2-a]pyridin-2-yl]methylsulfanyl]acetic acid **(116);**
2-[[6-chloro-3-[2-(4-fluorophenyl)ethynyl]imidazo[1,2-a]pyridin-2-yl] methylsulfanyl]acetic acid **(117);**
2-[[6-chloro-3-[2-[3-(trifluoromethyl)phenyl]ethynyl]imidazo[1,2-a]pyridin-2-yl]methylsulfanyl]acetic acid **(118);**
2-[[6-chloro-3-[2-[3-(trifluoromethoxy)phenyl]ethynyl]imidazo[1,2-a] pyridin-2-yl]methylsulfanyl]acetic acid **(119);**
2-[[8-methoxy-3-[2-[3-(trifluoromethyl)phenyl]ethynyl]imidazo[1,2-a] pyridin-2-yl]methylsulfanyl]acetic acid **(120);**
ethyl 2-[[3-[2-[3-(trifluoromethyl)phenyl]ethynyl]imidazo[1,2-a]pyridin-2-yl] methylsulfonyl]acetate **(121);**
ethyl 2-[[6-chloro-3-[2-[3-(trifluoromethoxy)phenyl]ethynyl]imidazo[1,2-a]pyridin-2-yl] methylsulfonyl]acetate **(122);**
2-[[6-chloro-3-[2-[3-(trifluoromethoxy)phenyl]ethynyl]imidazo[1,2-a] pyridin-2-yl]methylsulfonyl]acetic acid **(123);**
N-(3-pyridyl)-2-[[3-[2-[3-(trifluoromethyl)phenyl]ethynyl]imidazo[1,2-a]pyridin-2-yl]methylsulfanyl]acetamide **(124);**
N-(3-pyridyl)-2-[[3-[2-[3-(trifluoromethoxy)phenyl]ethynyl]imidazo[1,2-a]pyridin-2-yl]methoxy]acetamide **(125);**
N-(3-pyridyl)-2-[[3-[2-[3-(trifluoromethoxy)phenyl]ethynyl]imidazo[1,2-a]pyridin-2-yl]methylsulfanyl]acetamide **(126);**
4-hydroxy-3,3-dimethyl-2-[[3-[2-[3-(trifluoromethyl)phenyl]ethynyl]imidazo [1,2-a]pyridin-2-yl]methoxy]butanoic acid**(129);**
2-[[8-methoxy-3-[2-[3-(trifluoromethyl)phenyl]ethynyl]imidazo[1,2-a]pyridin-2-yl]methylsulfanyl]ethanol **(130)**;
2-[[6-chloro-3-[2-[3-(trifluoromethyl)phenyl]ethynyl]imidazo[1,2-a]pyridin-2-yl] methylsulfanyl]ethanol **(131)**;
N-carbamimidoyl-2-[[3-[2-[3-(trifluoromethyl)phenyl]ethynyl] imidazo[1,2-a]pyridin-2-yl]methylsulfanyl]acetamide hydrochloride(**135**)

9. A pharmaceutical composition comprising a derivative according to any of claims 1 to 8 and a pharmaceutically acceptable excipient.

10. The pharmaceutical composition according to claim 9 further comprising another antidiabetic agent, in particular metformin.

11. The derivative according to any of claims 1 to 8 for use as a drug.

12. The derivative according to any of claims 1 to 8 for use as a drug intended for treating and/or preventing diabetes, its complications and/or associated pathologies, advantageously diabetes of type II or hyperglycaemia.
